# EUROPEAN PATENT APPLICATION

(11) **EP 4 600 242 A1**
(43) Date of publication of application: **13.08.2025**
(21) Application number: 23874950.1
(22) Date of filing: 06.10.2023
(51) Int. Cl.: C07C 311/07, A61K 31/165, A61K 31/18, A61K 31/277, A61K 31/341, A61K 31/40, A61K 31/4192, A61K 31/42, A61K 31/421, A61K 31/426, A61K 31/44, A61K 31/505, A61P 25/26, A61P 43/00, C07D 249/04, C07D 261/02, C07D 263/32, C07D 277/30, C07D 307/54

(54) **CYCLOPENTANE COMPOUND**

(30) Priority: 07.10.2022 JP 2022162168
(71) Applicant: Kissei Pharmaceutical Co., Ltd., Matsumoto-Shi, Nagano 399-8710 (JP)
(72) Inventor: KONDO, Atsushi, Azumino-shi, Nagano 399-8304 (JP); YOSHIDA, Masako, Azumino-shi, Nagano 399-8304 (JP); MATSUMOTO, Tsutomu, Azumino-shi, Nagano 399-8304 (JP); SHIOGAI, Akihiro, Azumino-shi, Nagano 399-8304 (JP); OKI, Tomoya, Azumino-shi, Nagano 399-8304 (JP)
(74) Representative: Boult Wade Tennant LLP
(86) International application number: PCT/JP2023/036458
(87) International publication number: WO 2024/075825

(57) **Abstract**

The present invention aims to provide a novel compound which has an OX2R agonist activity.

The present invention relates to a cyclopentane compound represented by the following formula (I) or a pharmaceutically acceptable salt thereof. The compound of the present invention or a pharmaceutically acceptable salt thereof has an agonist activity on OX2R and is useful as an agent for the treatment of sleep disorders involving OX2R (for example, narcolepsy, idiopathic hypersomnia, Kleine-Levin syndrome, hypersomnia due to physical diseases, hypersomnia due to psychiatric disorders, hypersomnia due to drugs or substances, circadian rhythm sleep-wake disorder, insufficient sleep syndrome and long sleep) or the like.

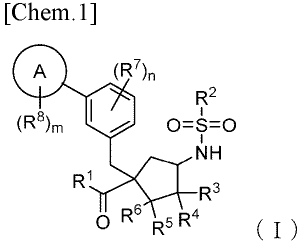

## Description

### Technical Field

The present invention relates to cyclopentane compounds useful as medicaments. More particularly, the present invention relates to cyclopentane compounds or pharmaceutically acceptable salts thereof which have an orexin 2 receptor (OX2R) agonist activity and are useful as agents for the treatment of sleep disorders involving OX2R.

### Background Art

Orexin is a neuropeptide produced and secreted by orexin neurons in the hypothalamus and contains two subtypes, orexin A and orexin B. Projections of orexin neurons are distributed throughout the central nervous system except for the cerebellum, with prominent projections to monoaminergic nerves and cholinergic nerves in the brainstem, the paraventricular nucleus in the thalamus, and the like, which are involved in sleep-wake mechanisms. Orexin receptors are expressed corresponding to the projection targets, and two kinds of subtypes, orexin 1 receptors (OX1R) and orexin 2 receptors (OX2R), are present in different topography.

Orexin nervous system plays an important role in maintenance of wakefulness, and its dysfunction is considered to be the cause of the onset of narcolepsy.

Narcolepsy is one of sleep disorders and is a chronic neurologic disease whose main symptoms are excessive daytime sleepiness and paroxysmal sleep. There are two types of narcolepsy according to symptoms: a case with cataplexy (a seizure in which the body suddenly loses strength triggered by strong emotions) is classified as Type 1 (NT1); and a case without cataplexy is classified as Type 2 (NT2).

Currently, in treatment of narcolepsy, Modafinil, Methylphenidate and Pemoline, which are central nerve stimulators, are used for hypersomnia, but all of them can cause paradoxical reaction, and Methylphenidate and Pemoline have strong side effects, including dependency and the like. On the other hand, on cataplexy, antidepressants are used because no therapeutic effect is observed by the above agents, but it is difficult to use them because of their strong side effect, and there are problems such as exacerbation due to a rebound phenomenon that is characteristic of antidepressants when the drugs are discontinued.

In this way, it is present situation of narcolepsy treatment that there are only symptomatic therapies for any symptom, and therapeutic agents based on the etiology of narcolepsy are desired.

In research using the postmortem brains of narcolepsy patients, it is reported that orexin neurons in the hypothalamus have decreased in comparison with healthy subjects (Non-patent literature 1). In addition, it is reported that the gene responsible for narcolepsy is the gene encoding OX2R in a canine model of hereditary narcolepsy (Non-patent literature 2) and that, while OX1R deficient mice don't develop narcolepsy symptoms, OX2R deficient mice develop the symptoms (Non-patent literature 3). In view of this background, it is strongly indicated that OX2R signaling is important in the maintenance of wakefulness and that its reduction is related to the onset of narcolepsy, and OX2R agonists are expected as therapeutic agents of narcolepsy or other sleep disorders.

As an OX2R agonist, for instance, TAK-925 is known, and prolonging of wake time by subcutaneous administration of TAK-925 was observed in wild-type mice (Non-patent literature 4). In addition, in a clinical study, it is reported that TAK-925 showed the effects of maintaining wakefulness and reducing daytime sleepiness in NT1 patients (Patent literature 1).

Compounds having an OX2R agonist activity are disclosed in Patent literatures 1 to 14 and Non-patent literature 4. In addition, compounds including cyclopentane substituted by sulfonamide as a substructure are disclosed in Patent literature 15 and Non-patent literature 5. However, the cyclopentane compounds of the invention of the present application are not described in any of Patent literatures 1 to 15, Non-patent literature 4 and Non-patent literature 5.

### Citation List

### [Patent Literature]

Patent literature 1: WO 2021/048822
Patent literature 2: WO 2021/166934
Patent literature 3: WO 2021/142083
Patent literature 4: WO 2021/048821
Patent literature 5: U.S. Published Application No. 2020/0247747
Patent literature 6: U.S. Published Application No. 2019/0040010
Patent literature 7: U.S. Published Application No. 2020/0385346
Patent literature 8: U.S. Published Application No. 2017/0226137
Patent literature 9: WO 2022/109117
Patent literature 10: U.S. Published Application No. 2022/0056017
Patent literature 11: WO 2022/040058
Patent literature 12: WO 2021/026047
Patent literature 13: U.S. Published Application No. 2020/0255403
Patent literature 14: U.S. Published Application No. 2022/0144771
Patent literature 15: U.S. Published Application No. 2007/0149532

### [Non-Patent Literature]

Non-patent literature 1: Thomas C. Thannickal et al., "Neuron" 2000, Vol. 27, No. 3, pp. 469-474
Non-patent literature 2: Ling Lin et al., "Cell" 1999, Vol. 98, No. 3, pp. 365-376
Non-patent literature 3: Michihiro Mieda et al., "Proceedings of the National Academy of Sciences of the United States of America" 2004, Vol. 101, No. 13, pp. 4649-4654
Non-patent literature 4: Hiroshi Yukitake et al, "Pharmacology, Biochemistry and Behavior" 2019, Vol. 187, p. 172794
Non-patent literature 5: Maris Vilums et al., "European Journal of Medicinal Chemistry" 2015, Vol. 93, pp. 121-134

### Summary of the Invention

### [Problems to be Solved by the Invention]

The present invention aims to provide a novel compound which has an OX2R agonist activity.

### [Means for Solving the Problems]

The present invention relates to a compound represented by the following formula (I) or a pharmaceutically acceptable salt thereof.

That is, the present invention relates to the following [1] to [12] and the like.
[1] A compound represented by the formula (I): 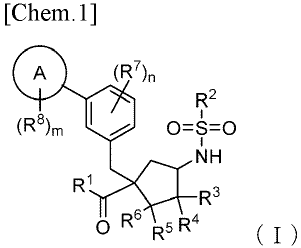 [in the formula,
   ring A is C₆₋₁₀ aryl, 5- or 6-membered heteroaryl, or 9- or 10-membered heteroaryl;
   R¹ is a group selected from the group consisting of the following (a) to (c):

      (a) -NR^{a}R^{a'},

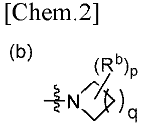

      and 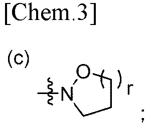
   R^{a} and R^{a}' are each independently a hydrogen atom, a hydroxy group, C₁₋₆ alkyl, C₁₋₆ alkoxy, hydroxy C₁₋₆ alkyl, C₁₋₆ alkoxy C₁₋₆ alkyl, or halo C₁₋₆ alkyl;
   R^{b} is a hydrogen atom, a halogen atom, C₁₋₆ alkyl, C₁₋₆ alkoxy, or halo C₁₋₆ alkyl;
   p is an integer number of 1 to 3;
   when p is 2 or 3, these R^{b} are the same or different from each other;
   q is an integer number of 1 to 3;
   r is 1 or 2;
   R² is C₁₋₆ alkyl, C₂₋₆ alkenyl, C₃₋₈ cycloalkyl which may be substituted by 1 to 3 C₁₋₆ alkyl, 3- to 8-membered heterocycloalkyl which may be substituted by 1 to 3 C₁₋₆ alkyl, halo C₁₋₆ alkyl, or C₁₋₆ alkylamino;
   R³ and R⁴ are each independently a hydrogen atom, a halogen atom, C₁₋₆ alkyl, or C₁₋₆ alkoxy;
   R⁵ and R⁶ are each independently a hydrogen atom or a halogen atom;
   R⁷ is a hydrogen atom, a halogen atom, a hydroxy group, an amino group, C₁₋₆ alkyl, halo C₁₋₆ alkyl, C₁₋₆ alkoxy, or C₁₋₆ alkylamino;
   R⁸ is a hydrogen atom, a halogen atom, a hydroxy group, a cyano group, C₁₋₆ alkyl, C₁₋₆ alkoxy, halo C₁₋₆ alkyl, or C₁₋₆ alkylamino;
   n is 1 or 2;
   when n is 2, these R⁷ are the same or different from each other; and
   m is 1 or 2;
   when m is 2, these R⁸ are the same or different from each other];
   or a pharmaceutically acceptable salt thereof.
[2] The compound according to the above [1]:
   wherein ring A is C₆₋₁₀ aryl or 5- or 6-membered heteroaryl;
   R⁵ and R⁶ are hydrogen atoms;
   R⁷ is a hydrogen atom, a halogen atom, a hydroxy group, C₁₋₆ alkyl, halo C₁₋₆ alkyl, or C₁₋₆ alkoxy; and
   n is 1;
   or a pharmaceutically acceptable salt thereof.
[3] The compound according to of the above [1] or [2]:
   wherein R^{a} and R^{a}' are each independently a hydrogen atom, a hydroxy group, C₁₋₆ alkyl, or C₁₋₆ alkoxy;
   R^{b} is C₁₋₆ alkyl;
   p is 1;
   q is 2;
   r is 1; and
   R³ and R⁴ are each independently a hydrogen atom, a halogen atom, or C₁₋₆ alkyl;
   or a pharmaceutically acceptable salt thereof.
[4] The compound according to any one of the above [1] to [3]:
   wherein R¹ is -NR^{a}R^{a'}; and
   R^{a} and R^{a}' are each independently C₁₋₆ alkyl or C₁₋₆ alkoxy;
   or a pharmaceutically acceptable salt thereof.
[5] The compound according to any one of the above [1] to [4]:
   wherein ring A is 5- or 6-membered heteroaryl;
   or a pharmaceutically acceptable salt thereof.
[6] The compound according to any one of the above [1] to [5]:
   wherein R² is C₁₋₆ alkyl, C₃₋₈ cycloalkyl which may be substituted by 1 to 3 C₁₋₆ alkyl, 3-to 8-membered heterocycloalkyl which may be substituted by 1 to 3 C₁₋₆ alkyl, or halo C₁₋₆ alkyl;
   or a pharmaceutically acceptable salt thereof.
[7] The compound according to any one of the above [1] to [6] which is represented by the formula (II): 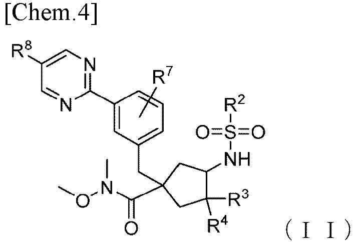 [in the formula,
   R² is C₁₋₆ alkyl, C₃₋₈ cycloalkyl which may be substituted by 1 to 3 C₁₋₆ alkyl, 3- to 8-membered heterocycloalkyl which may be substituted by 1 to 3 C₁₋₆ alkyl, or halo C₁₋₆ alkyl;
   R³ and R⁴ are each independently a hydrogen atom, a halogen atom, or C₁₋₆ alkyl;
   R⁷ is a hydrogen atom, a halogen atom, a hydroxy group, or C₁₋₆ alkyl; and
   R⁸ is a hydrogen atom, a halogen atom, or C₁₋₆ alkyl];
   or a pharmaceutically acceptable salt thereof.
[8] The compound according to any one of the above [1] to [7], selected from the group consisting of the following compounds: 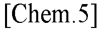 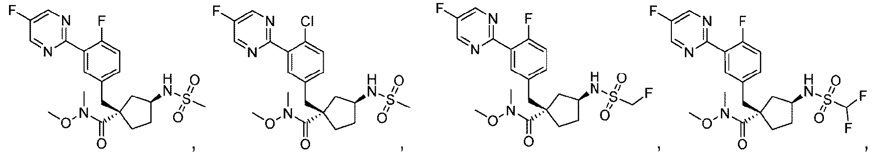 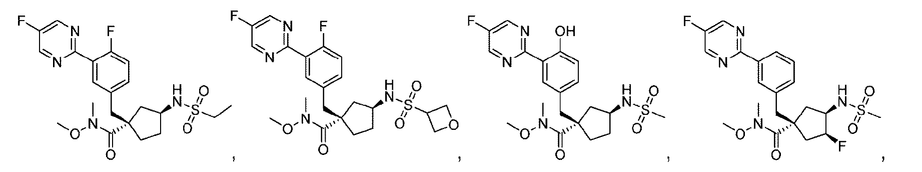 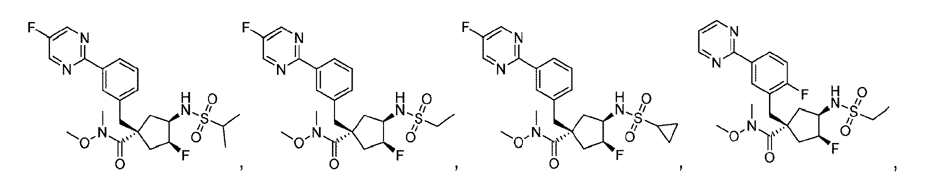 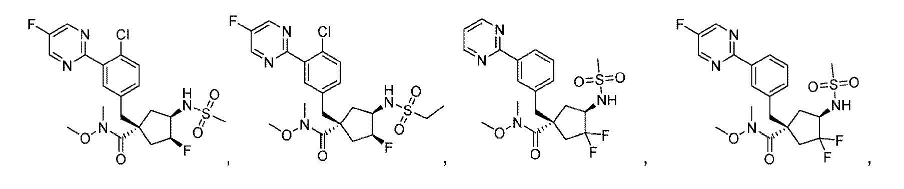 and 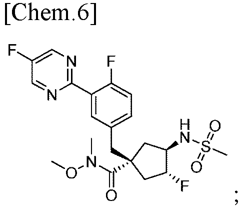 or a pharmaceutically acceptable salt thereof.
[9] The compound according to any one of the above [1] to [7], selected from the group consisting of the following compounds: 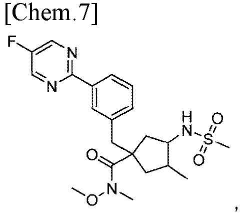 and 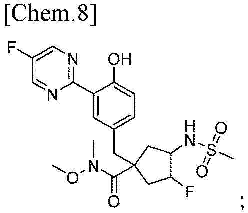 or a pharmaceutically acceptable salt thereof.
[10] A pharmaceutical composition comprising the compound according to any one of the above [1] to [9] or a pharmaceutically acceptable salt thereof, and a pharmaceutical additive.
[11] The pharmaceutical composition according to the above [10] which is a pharmaceutical composition for use in the treatment of a sleep disorder involving OX2R.
[12] The pharmaceutical composition according to the above [11], wherein the sleep disorder involving OX2R is narcolepsy.

In an embodiment of the compounds represented by the formula (I), for example, the compounds represented by the following formula (I-I) are preferred. 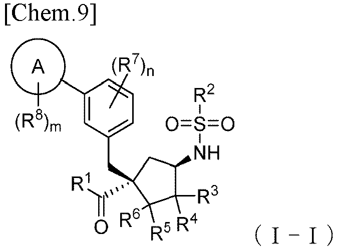 [The symbols in the formula have the same meanings as described in any one of the above [1] to [6]. ]

In an embodiment of the compounds represented by the formula (II), for example, the compounds represented by the following formula (II-I) are preferred. 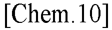 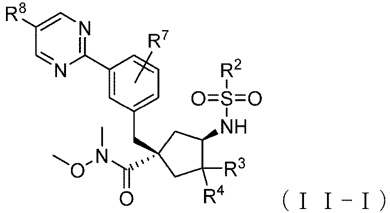 [The symbols in the formula have the same meanings as described in the above [7]. ]

In an embodiment, the present invention relates to a method for treating a sleep disorder involving OX2R, comprising administering a necessary amount of the pharmaceutical composition according to the above [10] to a patient.

In an embodiment, the present invention relates to a use of the compound according to any one of the above [1] to [9] or a pharmaceutically acceptable salt thereof for manufacturing a pharmaceutical composition for use in the treatment of a sleep disorder involving OX2R.

### [Effect of the Invention]

The compounds of the present invention have an excellent OX2R agonist activity. Therefore, the compounds of the present invention or pharmaceutically acceptable salts thereof are useful as agents for the treatment of a sleep disorder involving OX2R.

### [Mode for Carrying out the Invention]

Hereinafter, embodiments of the present invention are described in more detail.

In the present invention, each term has the following meaning unless otherwise specified.

The term "halogen atom" means a fluorine atom, a chlorine atom, a bromine atom or an iodine atom.

The term "C₁₋₆ alkyl" means a straight-chained or branched alkyl group having 1 to 6 carbon atoms. For example, methyl, ethyl, propyl, isopropyl, butyl, isobutyl, secbutyl, tert-butyl and the like can be illustrated.

The term "C₂₋₆ alkenyl" means a straight-chained or branched alkenyl group having 2 to 6 carbon atoms. For example, ethenyl, 1-propenyl, 2-propenyl and the like can be illustrated.

The term "C₁₋₆ alkoxy" means a straight-chained or branched alkoxy group having 1 to 6 carbon atoms. For example, methoxy, ethoxy, propoxy, isopropoxy and the like can be illustrated.

The term "halo C₁₋₆ alkyl" means C₁₋₆ alkyl substituted with 1 to 5 same or different halogen atoms. For example, monofluoromethyl, 2-fluoroethyl, difluoromethyl, trifluoromethyl, 1,1-difluoroethyl, 2,2,2-trifluoroethyl, 3,3,3-trifluoropropyl, 4,4,4-trifluorobutyl, pentafluoroethyl and the like can be illustrated.

The term "hydroxy C₁₋₆ alkyl" means C₁₋₆ alkyl substituted by 1 or 2 hydroxy groups. For example, hydroxymethyl, 2-hydroxyethyl, 3-hydroxypropyl, 2-hydroxypropan-2-yl and the like can be illustrated.

The term "C₁₋₆ alkoxy C₁₋₆ alkyl" means C₁₋₆ alkyl substituted by one C₁₋₆ alkoxy. For example, methoxymethyl, 2-methoxyethyl, ethoxymethyl, 2-ethoxyethyl and the like can be illustrated.

The term "C₁₋₆ alkylamino" means a group represented by (C₁₋₆ alkyl)-NH- or a group represented by (C₁₋₆ alkyl)₂N-, and the two C₁₋₆ alkyls in the group represented by (C₁₋₆ alkyl)₂N- may be same or different from each other. For example, methylamino, ethylamino, propylamino, isopropylamino, dimethylamino, methylethylamino, diethylamino and the like can be illustrated.

The term "C₆₋₁₀ aryl" means a phenyl group or a naphthyl group.

The term "5- or 6-membered heteroaryl" means a 5- or 6-membered aromatic heterocyclic group having any 1 to 4 hetero atoms selected from an oxygen atom, a nitrogen atom and a sulfur atom in the ring. For example, pyridyl, pyrimidyl, furyl, pyrrolyl, thienyl, imidazolyl, pyrazolyl, 1,2,4-triazolyl, isothiazolyl, isoxazolyl, oxazolyl, thiazolyl, 1,3,4-oxadiazolyl, 1,2,4-oxadiazolyl and the like can be illustrated.

The term "9- or 10-membered heteroaryl" means a bicyclic aromatic heterocyclic group having any 1 to 4 hetero atoms selected from an oxygen atom, a nitrogen atom and a sulfur atom in the ring. For example, indolyl, isoindolyl, benzofuryl, benzothiophenyl, benzoimidazolyl, purinyl, benzotriazolyl, quinolyl, isoquinolyl, quinazolyl, quinoxalyl, cinnolyl, pteridinyl, chromenyl, isochromenyl and the like can be illustrated.

The term "C₃₋₈ cycloalkyl" means a 3- to 8-membered ring saturated hydrocarbon group and includes those having a partially crosslinked structure. For example, cyclopropyl, cyclobutyl, cyclopentyl, cyclohexyl, bicyclo[1.1.1]pentyl, bicyclo[2.2.2]octyl and the like can be illustrated.
In addition, when the C₃₋₈ cycloalkyl is substituted by 1 to 3 C₁₋₆ alkyls, these substituents may be the same or different from each other. As C₃₋₈ cycloalkyl substituted by 1 to 3 C₁₋₆ alkyls, for example, 1-methylcyclopropyl, 2-methylcyclopropyl and the like can be illustrated.

The term "3- to 8-membered heterocycloalkyl" means a cycloalkyl group in which carbon atoms in the ring are substituted by 1 or 2 hetero atoms selected from an oxygen atom, a nitrogen atom and a sulfur atom and includes those having a partially crosslinked structure. For example, aziridinyl, azetidinyl, pyrrolidinyl, piperidinyl, piperazinyl, morpholinyl, thiomorpholinyl, azepanyl, azabicyclo[2.2.2]octyl, 2-oxa-5-azabicyclo[2.2.1]heptanyl, 6-oxa-3-azabicyclo[2.2.1]heptanyl, 8-oxa-3-azabicyclo[3.2.1]octanyl, 3-oxa-8-azabicyclo[3.2.1]octanyl, 2-azaspiro[3.3]heptanyl, 2-oxa-6-azaspiro[3.3]heptanyl, pyrrolidonyl, piperidonyl, oxiranyl, oxetyl, tetrahydrofuranyl, tetrahydropyranyl and the like can be illustrated.

The following abbreviations in the description, the figures and the tables have the following meanings, respectively.
AZADOL (registered trademark): 2-Hydroxy-2-azaadamantane
Boc₂O: di-tert-butyl dicarbonate
DIPEA: N,N-diisopropylethylamine
DCM: dichloromethane
DMAP: 4-dimethylaminopyridine
DMF: N,N-dimethylformamide
DMSO: dimethylsulfoxide
Deoxo-Fluor: bis(2-methoxyethyl)-aminosulfur trifluoride
HATU: O-(7-azabenzotriazol-1-yl)-N,N,N',N'-tetramethyluronium hexafluorophosphate
LDA: lithium diisopropylamide
MeCN: acetonitrile
Me4tBuXPhos: 2-di-tert-butylphosphino-3,4,5,6-tetramethyl-2',4',6'-triisopropyl-1,1'-biphenyl
MTBE: methyl tert-butyl ether
NMP: N-methylpyrrolidone
PdCl₂(PPh₃)₂: dichlorobis(triphenylphosphine)palladium(II)
Pd(dba)₂: bis(dibenzylideneacetone)palladium(0)
Pd₂(dba)₃: tris(dibenzylideneacetone)dipalladium(0)
Pd(dppf)Cl₂: [1,1'-bis(diphenylphosphino)ferrocene]dichloropalladium(II)
Pd(PPh₃)₄: tetrakis(triphenylphosphine)palladium(0)
TEA: triethylamine
TFA: trifluoroacetic acid
THF: tetrahydrofuran
Xphos: 2-dicyclohexylphosphino-2',4',6'-triisopropylbiphenyl
Xphos Pd G3: (2-dicyclohexylphosphino-2',4',6'-triisopropyl-1,1'-biphenyl)[2-(2'-amino-1,1'-biphenyl)]palladium(II) methanesulfonic acid salt
10% Pd/C: Palladium 10% on Carbon (wetted with ca. 55% water)
APS: aminopropylated silica gel
ODS: octadecyl-silylated silica gel
Method A: column chromatography with an aminopropylated silica gel column followed by a silica gel column
Chiral preparative column chromatography: chiral preparative column chromatography using YMC CHIRALART Cellulose-SB
Ref. No.: Reference Example Number
Str.: structural formula
Ex. No.: Example Number
Phys. data: physical data
¹H-NMR: hydrogen nuclear magnetic resonance spectrum
CDCl₃: chloroform-d1
MS: mass spectrometry (The measured values in the tables were measured by electrospray ionization or multiionization using electrospray ionization-atmospheric pressure chemical ionization.)
Act.: activation rate of test substance at a compound concentration of 10 µM calculated by setting the fluorescence intensity obtained when human orexin A peptide was added at a concentration of 1 µM as 100% and the fluorescence intensity obtained when the medium alone was added as 0%
CHO: Chinese hamster ovary
HEPES: 2-[4-(2-hydroxyethyl)-1-piperazinyl]ethanesulfonic acid

In the case where the compounds represented by the formula (I) or the formula (II) contain one or more asymmetric carbon atoms, stereoisomers in the R- or S-configuration at each of the asymmetric carbon atoms and compounds of any combinations thereof are included in the present invention. In such cases, racemic compounds, racemic mixtures, individual enantiomers and mixtures of diastereomers are included in the scope of the present invention.

In the case where the compounds represented by the formula (I) or the formula (II) have the cis-trans isomers, the present invention includes all the cis-trans isomers.

In the case where the compounds represented by the formula (I) or the formula (II) have tautomers, the present invention includes all the tautomers.

In the present invention, stereochemical determination can also be conducted according to well-known methods in the technical field.

The compounds represented by the formula (I) or the formula (II) can also be converted into pharmaceutically acceptable salts thereof according to a general method, if necessary. As such salts, acid addition salts and salts with a base can be illustrated.

As the acid addition salt, an acid addition salt with a mineral acid such as hydrochloric acid, hydrobromic acid, hydroiodic acid, sulfuric acid, nitric acid, phosphoric acid and the like, and an acid addition salt with an organic acid such as formic acid, acetic acid, trifluoroacetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, benzoic acid, glutamic acid, aspartic acid and the like can be illustrated.

As the salt with a base, a salt formed with an inorganic base such as lithium salt, sodium salt, potassium salt, calcium salt, magnesium salt and the like, and a salt formed with an organic base such as N-methyl-D-glucamine, N,N'-dibenzylethylenediamine, triethylamine, piperidine, morpholine, pyrrolidine, arginine, lysine, choline and the like can be illustrated.

Unless otherwise noted, suffixes to chemical names or structural formulae for salts such as "hydrochloride" or "HCl", for example, are not stoichiometric descriptions but solely mean salt forms.

In the case where the compounds represented by the formula (I) or the formula (II) or a pharmaceutically acceptable salt thereof exists, for example, as crystal, the present invention includes all the crystalline forms. For example, a pharmaceutically acceptable salt also includes a solvate thereof with a pharmaceutically acceptable solvent such as water or ethanol, a cocrystal thereof with an appropriate cocrystal former (coformer) and the like.

In the compounds represented by the formula (I) or the formula (II), part of the atoms may be replaced with corresponding isotopes. The present invention includes compounds in which atoms are replaced with these isotopes. Examples of the isotopes include isotopes of a hydrogen atom, a carbon atom, a chlorine atom, a fluorine atom, an iodine atom, a nitrogen atom, an oxygen atom and a sulfur atom represented by ²H, ³H, ¹¹C, ¹³C, ¹⁴C, ³⁶Cl , ¹⁸F, ¹²³I, ¹²⁵I, ¹³N, ¹⁵N, ¹⁵O, ¹⁷O, ¹⁸O and ³⁵S. In an embodiment, the compounds represented by the formula (I) in which part of the hydrogen atoms are replaced with ²H (D: deuterium atoms) can be illustrated.

The compounds represented by the formula (I) or the formula (II) in which part of the atoms are replaced with isotopes can be prepared by a similar method to the method for manufacturing described below using a commercial isotope-introduced reagent. In addition, they can also be prepared using a method described in a literature (for example, see, "Yukigosei-kagaku kyokaishi" 2007, Vol. 65, No. 12, pp. 1179-1190 or "RADIOISOTOPES" 2007, Vol. 56, No. 11, pp. 741-750).

The compounds represented by the formula (I) or the formula (II) of the present invention can also be prepared, for example, according to a method described in Schemes 1 to 12 or a similar method thereto, or a method described in a literature or a similar method thereto. In the Schemes, the compounds represented by the formula (I) or the formula (II) correspond to the compounds represented by the formulae (I-1) to (I-10).

The compounds represented by the formula (I) or the formula (II) of the present invention can be prepared by methods shown below, but the following production methods are exemplifications of general production methods and do not limit the methods of production.

For reaction in each process, commercial products can be used when the starting materials and the reagents are commercially available.

For reaction in each process, the reaction time is usually from 30 minutes to 3 days, varying with the used starting material, solvent, reaction temperature or the like, unless otherwise specified.

For reaction in each process, the reaction temperature is usually at -78°C to reflux temperature, varying with the used starting material, solvent or the like, unless otherwise specified.

For reaction in each process, the pressure is usually at 1 to 20 atm, varying with the used starting material, solvent, reaction temperature or the like, unless otherwise specified.

For reaction in each process, a microwave reactor such as Biotage's Initiator may be used. When the reaction is conducted using a microwave reactor, the reaction can be conducted under the conditions of pressure range: 1 to 30 bar, power range: 1 to 400 W, reaction temperature: room temperature to 300°C, and reaction time: a minute to 1 day, varying with the used starting material, solvent, model or the like.

Reaction in each process is conducted without any solvent or using an appropriate solvent unless otherwise specified. As an example of the appropriate solvent, a solvent which is inert to the reaction can be illustrated. As specific examples of the solvent to be used, the solvents which are described in the Reference Examples or the Examples corresponding to each process or the following solvents can be illustrated. The following solvents may be used as a mixture of two or more thereof at an appropriate ratio.
alcohols: methanol, ethanol, tert-butyl alcohol, 2-propanol and the like;
ethers: diethyl ether, THF, 1,2-dimethoxy ethane, 1,4-dioxane, cyclopentylmethylether, MTBE and the like;
aromatic hydrocarbons: benzene, chlorobenzene, 1,2-dichlorobenzene, toluene, xylene and the like;
saturated hydrocarbons: cyclohexane, n-hexane, n-pentane and the like;
amides: DMF, N,N-dimethylacetamide, NMP and the like;
halogenated hydrocarbons: DCM, 1,2-dichloroethane, chloroform, carbon tetrachloride and the like;
nitriles: MeCN and the like;
sulfoxides: DMSO and the like;
aromatic organic bases: pyridine and the like;
acid anhydrides: acetic anhydride and the like;
organic acids: formic acid, acetic acid, TFA, methanesulfonic acid and the like;
esters: ethyl acetate, methyl acetate, isopropyl acetate and the like;
ketones: acetone, methyl ethyl ketone and the like; and
water.

When a base is used for reaction in each process, the reaction is conducted using an appropriate base for the reaction. As specific examples of the base to be used, the bases which are described in the Reference Examples or the Examples corresponding to each process or the following bases can be illustrated:
inorganic bases: sodium hydroxide, lithium hydroxide, potassium hydroxide and the like; basic salts: sodium carbonate, sodium hydrogen carbonate, potassium carbonate, cesium carbonate and the like;
organic bases: TEA, DIPEA, diethylamine, pyridine, DMAP, 2,6-lutidine, 1,8-diazabicyclo[5.4.0]-7-undecene, imidazole, piperidine and the like;
metal alkoxides: sodium ethoxide, sodium methoxide, potassium tert-butoxide and the like;
alkali metal hydrides: sodium hydride and the like;
metal amides: sodium amide, LDA, lithium bis(trimethylsilyl)amide, sodium bis(trimethylsilyl)amide, potassium bis(trimethylsilyl)amide and the like;
organomagnesiums: isopropylmagnesium chloride and the like; and
organolithiums: methyllithium, n-butyllithium, sec-butyllithium, tert-butyllithium and the like.

When an acid is used for reaction in each process, the reaction is conducted using an appropriate acid for the reaction. As specific examples of the acid to be used, the acids which are described in the Reference Examples and the Examples corresponding to each process or the following acids can be illustrated:
inorganic acids: hydrochloric acid, sulfuric acid, nitric acid, hydrobromic acid, phosphoric acid and the like;
organic acids: acetic acid, TFA, citric acid, methanesulfonic acid, p-toluenesulfonic acid, 10-camphorsulfonic acid and the like;
Lewis acids: boron trifluoride-diethyl ether complex, zinc iodide, aluminum chloride, zinc chloride, titanium(IV) chloride and the like.

When a condensing reagent is used for reaction in each process, the reaction is conducted using an appropriate condensing reagent for the reaction. As specific examples of the condensing reagent to be used, the condensing reagents which are described in the Reference Examples or the Examples corresponding to each process or the following condensing reagents can be illustrated:
carbodiimides: 1-ethyl-3-(3-dimethylaminopropyl)carbodiimide hydrochloride, N,N'-dicyclohexylcarbodiimide and the like;
imidazoles: carbonyldiimidazole and the like;
uronium salts and phosphonium salts: HATU, 1H-benzotriazol-1-yloxytris(dimethylamino)phosphoniumhexafluorophosphate and the like;
triazins: 4-(4,6-dimethoxy-1,3,5-triazin-2-yl)-4-methylmorpholinium chloride and the like; and
others: propylphosphonic acid anhydride (cyclic trimer) and the like.

When a reducing reagent is used for reaction in each process, the reaction is conducted using an appropriate reducing reagent for the reaction. As specific examples of the reducing reagent to be used, the reducing reagents which are described in the Reference Examples or the Examples corresponding to each process or the following reducing reagents can be illustrated:
metal hydrides: lithium aluminum hydride, lithium borohydride, sodium borohydride, sodium triacetoxyborohydride, sodium cyanoborohydride, diisobutylaluminum hydride and the like; and
boranes: borane-THF complexes, picoline borane complex, decaborane and the like.

In each process, when a protective group is necessary based on the kind of a functional group, operations of introduction and removal can also be conducted optionally in combination according to a general method. Examples regarding the types of the protective groups, protection and deprotection include the methods described in Peter G.M. Wuts Eds., "Greene's Protective Groups in Organic Synthesis", fifth edition, Wiley-Interscience, 2014.

In each process, when hydrolysis is conducted, the reaction can be conducted in the presence of an acid or a base. As the acid and the base to be used, the above-mentioned examples can be illustrated.

In each process, when catalytic reduction is conducted, the reaction can be conducted in the presence of hydrogen and a catalyst. As the catalyst to be used, palladium on carbon powder, Pearlman's catalyst, platinum on carbon powder, Raney nickel and the like can be illustrated. An acid may be used for the reaction, if necessary.

In each process, when oxidation is conducted, the reaction can be conducted in the presence of an oxidizing reagent. As the oxidizing reagent to be used, m-chloroperbenzoic acid, iodobenzene diacetate and the like can be illustrated. An oxidation catalyst such as AZADOL may be used for the reaction, if necessary.

In each process, when reduction is conducted, the reaction can be conducted in the presence of a reducing reagent. As the reducing reagent to be used, the above-mentioned examples can be illustrated.

In each process, when amidation is conducted, the reaction can be conducted using a condensing reagent in the presence or absence of a base. As the condensing reagent and the base to be used, the above-mentioned examples can be illustrated. When a carbodiimide is used as the condensing reagent, the reaction may be conducted with the addition of an additive such as 1-hydroxybenzotriazole or DMAP, if necessary. The reaction may also be conducted using a halogenating agent in the presence or absence of a base. As the halogenating reagent to be used, thionyl chloride, oxalyl chloride, phosphoryl chloride, sulfuryl chloride, phosphorus trichloride and phosphorus pentachloride can be illustrated.

In each process, when reductive amination is conducted, the reaction can be conducted in the presence of a reducing reagent. As the reducing reagent to be used, the above-mentioned examples can be illustrated. The reaction can also be conducted in the presence of hydrogen and a catalyst. As the catalyst to be used, palladium on carbon powder, Pearlman's catalyst, platinum on carbon powder, Raney nickel and the like can be illustrated.

In each process, when aromatic nucleophilic substitution reaction is conducted, the reaction can be conducted in the presence of a base. As the base, the above-mentioned examples can be illustrated.

In each process, when Suzuki-Miyaura cross coupling reaction is conducted, the reaction can be conducted in the presence of a palladium catalyst and a base. As the palladium catalyst to be used, bis(di-tert-butyl(4-dimethylaminophenyl)phosphine)dichloropalladium(II), Pd(PPh₃)₄ and the like can be illustrated. As the base to be used, the above-mentioned examples can be illustrated.

In each process, when Mitsunobu reaction is conducted, the reaction can be conducted using an azodicarboxylic acid ester and triphenylphosphine. As the azodicarboxylic acid ester to be used, diethyl azodicarboxylate, diisopropyl azodicarboxylate and the like can be illustrated.

In each process, when sulfonylation is conducted, the reaction can be conducted using a sulfonylation reagent in the presence of a base. As the sulfonylation reagent to be used, methanesulfonyl chloride, ethanesulfonyl chloride and the like can be illustrated. As the base to be used, the above-mentioned examples can be illustrated.

In each process, when sulfamoylation is conducted, the reaction can be conducted using a sulfamoylation reagent in the presence of a base. As the sulfamoylation reagent to be used, N-methylsulfamoyl chloride, dimethylsulfamoyl chloride and the like can be illustrated. As the base to be used, the above-mentioned examples can be illustrated.

In each process, when fluorination is conducted, the reaction can be conducted using a fluorination reagent in the presence or absence of a base. As the fluorination reagent to be used, Deoxo-Fluor, N,N-diethylaminosulfur trifluoride and the like can be illustrated.

In each process, when Staudinger reaction is conducted, the reaction can be conducted using a phosphorous reagent. As the phosphorous reagent to be used, triphenylphosphine and the like can be illustrated.

A compound represented by the formula (I) can be prepared, for example, according to a method described in Scheme 1. 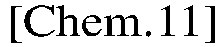 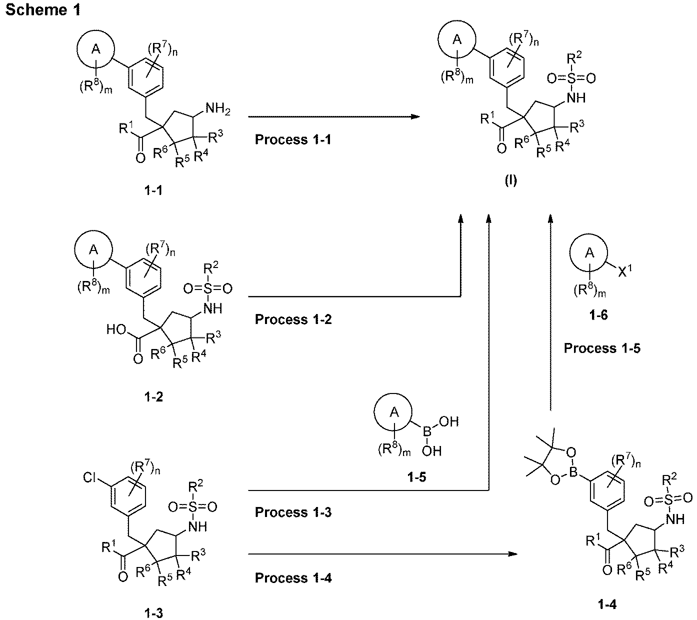 The symbols in the formulae have the same meanings as defined above. X¹ represents a chlorine atom, a bromine atom, or an iodine atom.

### Process 1-1

Compound (I) can also be prepared by sulfonylation or sulfamoylation of compound (1-1).

### Process 1-2

Compound (I) can also be prepared by amidation of compound (1-2).

### Process 1-3

Compound (I) can also be prepared by Suzuki-Miyaura cross coupling reaction of compound (1-3) with compound (1-5).

### Process 1-4

Compound (1-4) can also be prepared by reacting compound (1-3) with bis(pinacolato)diboron in the presence of a palladium catalyst.

### Process 1-5

Compound (I) can also be prepared by Suzuki-Miyaura cross coupling reaction of compound (1-4) with compound (1-6).

A compound represented by the formula (I-1) can be prepared, for example, according to a method described in Scheme 2.

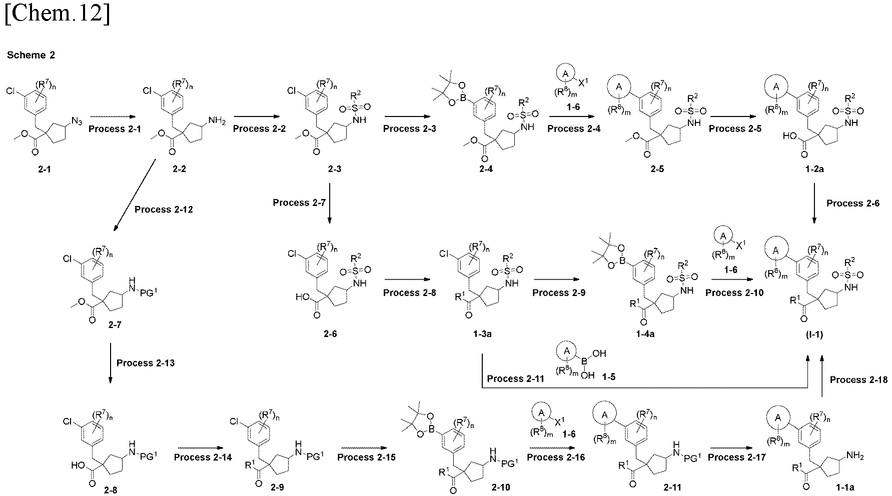

The symbols in the formulae have the same meanings as defined above. PG¹ represents a protective group.

### Process 2-1

Compound (2-2) can also be prepared by Staudinger reaction of compound (2-1).

### Process 2-2

Compound (2-3) can also be prepared by sulfonylation of compound (2-2).

### Process 2-3

Compound (2-4) can also be prepared by reacting compound (2-3) with bis(pinacolato)diboron in the presence of a palladium catalyst.

### Process 2-4

Compound (2-5) can also be prepared by Suzuki-Miyaura cross coupling reaction of compound (2-4) with compound (1-6).

### Process 2-5

Compound (1-2a) can also be prepared by hydrolysis of compound (2-5).

### Process 2-6

Compound (I-1) can also be prepared by amidation of compound (1-2a).

### Process 2-7

Compound (2-6) can also be prepared by hydrolysis of compound (2-3).

### Process 2-8

Compound (1-3a) can also be prepared by amidation of compound (2-6).

### Process 2-9

Compound (1-4a) can also be prepared by reacting compound (1-3a) with bis(pinacolato)diboron in the presence of a palladium catalyst.

### Process 2-10

Compound (I-1) can also be prepared by Suzuki-Miyaura cross coupling reaction of compound (1-4a) with compound (1-6).

### Process 2-11

Compound (I-1) can also be prepared by Suzuki-Miyaura cross coupling reaction of compound (1-3a) with compound (1-5).

### Process 2-12

Compound (2-7) can also be prepared by protecting the amino group of compound (2-2).

### Process 2-13

Compound (2-8) can also be prepared by hydrolysis of compound (2-7).

### Process 2-14

Compound (2-9) can also be prepared by amidation of compound (2-8).

### Process 2-15

Compound (2-10) can also be prepared by reacting compound (2-9) with bis(pinacolato)diboron in the presence of a palladium catalyst.

### Process 2-16

Compound (2-11) can also be prepared by Suzuki-Miyaura cross coupling reaction of compound (2-10) with compound (1-6).

### Process 2-17

Compound (1-1a) can also be prepared by removing the protective group of compound (2-11).

### Process 2-18

Compound (I-1) can also be prepared by sulfonylation or sulfamoylation of compound (1-1a).

A compound represented by compound (2-1) can be prepared, for example, according to the method described in Scheme 3.

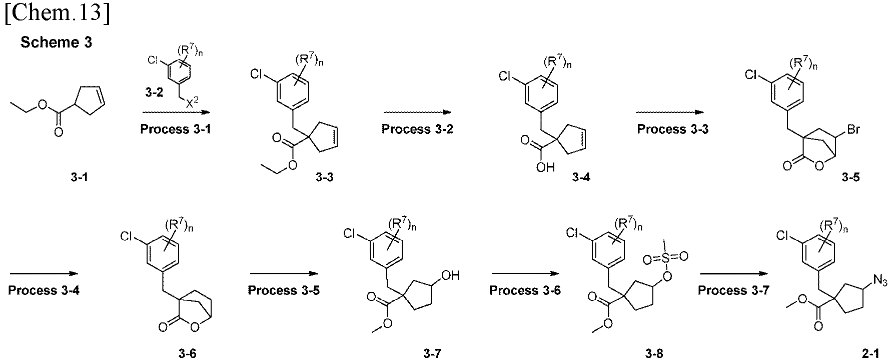

The symbols in the formulae have the same meanings as defined above. X² represents a chlorine atom, or a bromine atom.

### Process 3-1

Compound (3-3) can also be prepared by reacting compound (3-1) with compound (3-2) in the presence of a base.

### Process 3-2

Compound (3-4) can also be prepared by hydrolysis of compound (3-3).

### Process 3-3

Compound (3-5) can also be prepared by reacting compound (3-4) with DMSO and bromotrimethylsilane in the presence of a base.

### Process 3-4

Compound (3-6) can also be prepared by reacting compound (3-5) with 2,2'-azobis(isobutyronitrile) and tris(trimethylsilyl)silane.

### Process 3-5

Compound (3-7) can also be prepared by reacting compound (3-6) with methanol in the presence of an acid.

### Process 3-6

Compound (3-8) can also be prepared by reacting compound (3-7) with methanesulfonyl chloride in the presence of a base.

### Process 3-7

Compound (2-1) can also be prepared by reacting compound (3-8) with sodium azide.

A compound represented by the formula (I-2) can be prepared, for example, according to the method described in Scheme 4.

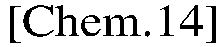

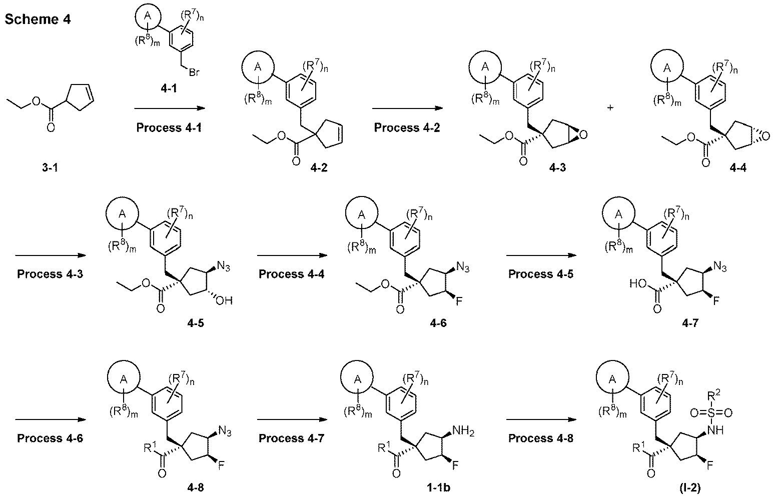

The symbols in the formulae have the same meanings as defined above. The stereochemical notations in the formulae represent relative configuration.

### Process 4-1

Compound (4-2) can also be prepared by reacting compound (3-1) with compound (4-1) in the presence of a base.

### Process 4-2

Compound (4-3) and compound (4-4) can also be prepared by oxidation of compound (4-2).

### Process 4-3

Compound (4-5) can also be prepared by reacting compound (4-4) with sodium azide.

### Process 4-4

Compound (4-6) can also be prepared by fluorination of compound (4-5).

### Process 4-5

Compound (4-7) can also be prepared by hydrolysis of compound (4-6).

### Process 4-6

Compound (4-8) can also be prepared by amidation of compound (4-7).

### Process 4-7

Compound (1-1b) can also be prepared by Staudinger reaction or catalytic reduction of compound (4-8).

### Process 4-8

Compound (I-2) can also be prepared by sulfonylation or sulfamoylation of compound (1-1b).

A compound represented by the formula (I-3) can be prepared, for example, according to the method described in Scheme 5.

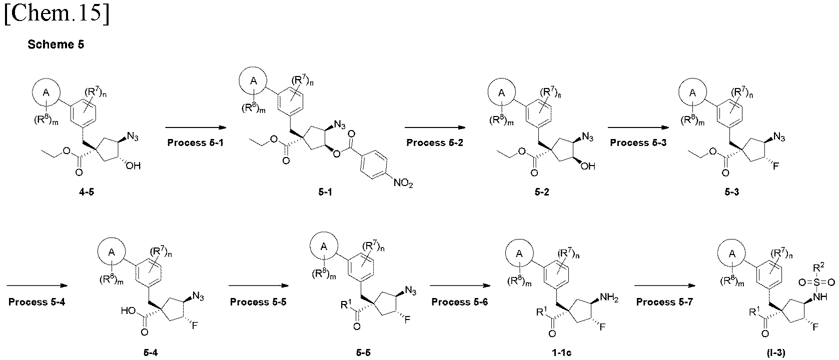

The symbols in the formulae have the same meanings as defined above. The stereochemical notations in the formulae represent relative configuration.

### Process 5-1

Compound (5-1) can also be prepared by Mitsunobu reaction of compound (4-5) and 4-nitrobenzoic acid.

### Process 5-2

Compound (5-2) can also be prepared by removing the p-nitrobenzoyl group of compound (5-1). The removal of the p-nitrobenzoyl group can be conducted, for example, by hydrolysis.

### Process 5-3

Compound (5-3) can also be prepared by fluorination of compound (5-2).

### Process 5-4

Compound (5-4) can also be prepared by hydrolysis of compound (5-3).

### Process 5-5

Compound (5-5) can also be prepared by amidation of compound (5-4).

### Process 5-6

Compound (1-1c) can also be prepared by Staudinger reaction or catalytic reduction of compound (5-5).

### Process 5-7

Compound (I-3) can also be prepared by sulfonylation or sulfamoylation of compound (1-1c).

A compound represented by the formula (I-4) can be prepared, for example, according to the method described in Scheme 6.

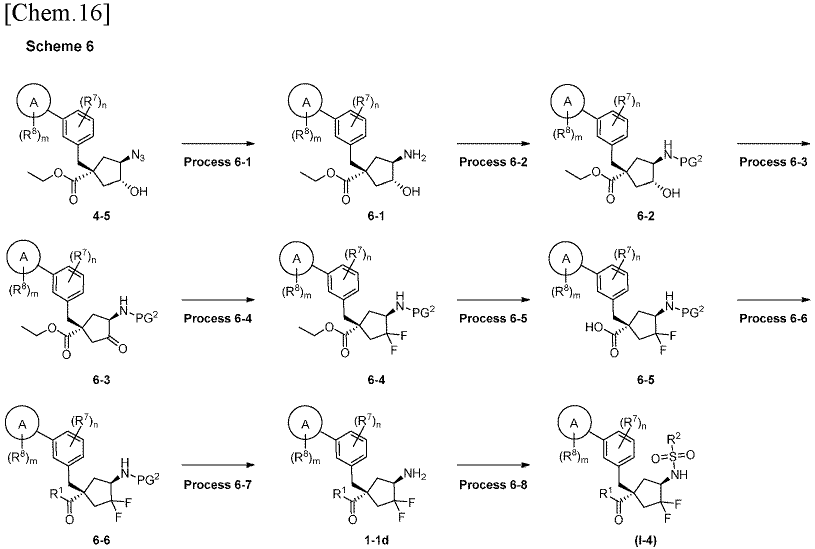

The symbols in the formulae have the same meanings as defined above. PG² represents a protective group. The stereochemical notations in the formulae represent relative configuration.

### Process 6-1

Compound (6-1) can also be prepared by Staudinger reaction or catalytic reduction of compound (4-5).

### Process 6-2

Compound (6-2) can also be prepared by protecting the amino group of compound (6-1).

### Process 6-3

Compound (6-3) can also be prepared by oxidation of compound (6-2).

### Process 6-4

Compound (6-4) can also be prepared by fluorination of compound (6-3).

### Process 6-5

Compound (6-5) can also be prepared by hydrolysis of compound (6-4).

### Process 6-6

Compound (6-6) can also be prepared by amidation of compound (6-5).

### Process 6-7

Compound (1-1d) can also be prepared by removing the protective group of compound (6-6).

### Process 6-8

Compound (I-4) can also be prepared by sulfonylation or sulfamoylation of compound (1-1d).

A compound represented by compound (4-1) can be prepared, for example, according to a method described in Scheme 7.

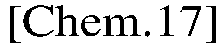

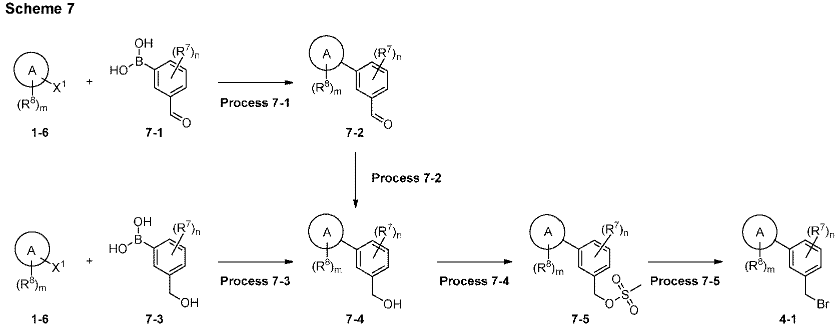

The symbols in the formulae have the same meanings as defined above.

### Process 7-1

Compound (7-2) can also be prepared by Suzuki-Miyaura cross coupling reaction of compound (1-6) and compound (7-1).

### Process 7-2

Compound (7-4) can also be prepared by reduction of compound (7-2).

### Process 7-3

Compound (7-4) can also be prepared by Suzuki-Miyaura cross coupling reaction of compound (1-6) and compound (7-3).

### Process 7-4

Compound (7-5) can also be prepared by reacting compound (7-4) with methanesulfonyl chloride in the presence of a base.

### Process 7-5

Compound (4-1) can also be prepared by reacting compound (7-5) with lithium bromide.

A compound represented by the formula (I-5) can be prepared, for example, according to a method described in Scheme 8.

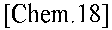

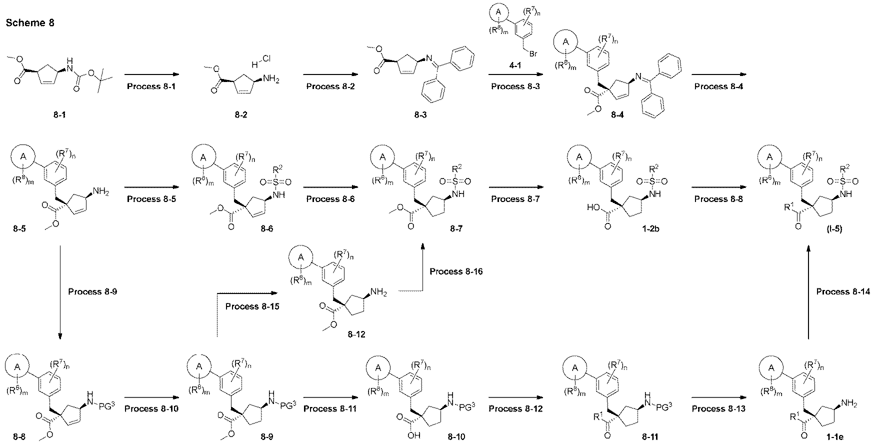

The symbols in the formulae have the same meanings as defined above. PG³ represents a carbamate protection group.

### Process 8-1

Compound (8-2) can also be prepared by removing the tert-butoxycarbonyl group of compound (8-1).

### Process 8-2

Compound (8-3) can also be prepared by reacting compound (8-2) with diphenylmethanimine.

### Process 8-3

Compound (8-4) can also be prepared by reacting compound (8-3) with compound (4-1) in the presence of a base.

### Process 8-4

Compound (8-5) can also be prepared by removing the diphenylmethylidene group of compound (8-4). The removal of the diphenylmethylidene group can be conducted, for example, by hydrolysis reaction.

### Process 8-5

Compound (8-6) can also be prepared by sulfonylation of compound (8-5).

### Process 8-6

Compound (8-7) can also be prepared by catalytic reduction of compound (8-6).

### Process 8-7

Compound (1-2b) can also be prepared by hydrolysis of compound (8-7).

### Process 8-8

Compound (I-5) can also be prepared by amidation of compound (1-2b).

### Process 8-9

Compound (8-8) can also be prepared by protecting the amino group of compound (8-5).

### Process 8-10

Compound (8-9) can also be prepared by catalytic reduction of compound (8-8).

### Process 8-11

Compound (8-10) can also be prepared by hydrolysis of compound (8-9).

### Process 8-12

Compound (8-11) can also be prepared by amidation of compound (8-10).

### Process 8-13

Compound (1-1e) can also be prepared by removing the protective group of compound (8-11).

### Process 8-14

Compound (I-5) can also be prepared by sulfonylation or sulfamoylation of compound (1-1e).

### Process 8-15

Compound (8-12) can also be prepared by removing the protective group of compound (8-9).

### Process 8-16

Compound (8-7) can also be prepared by sulfonylation or sulfamoylation of compound (8-12).

Compounds represented by the formula (I-6) and the formula (I-7) can be prepared, for example, according to a method described in Scheme 9.

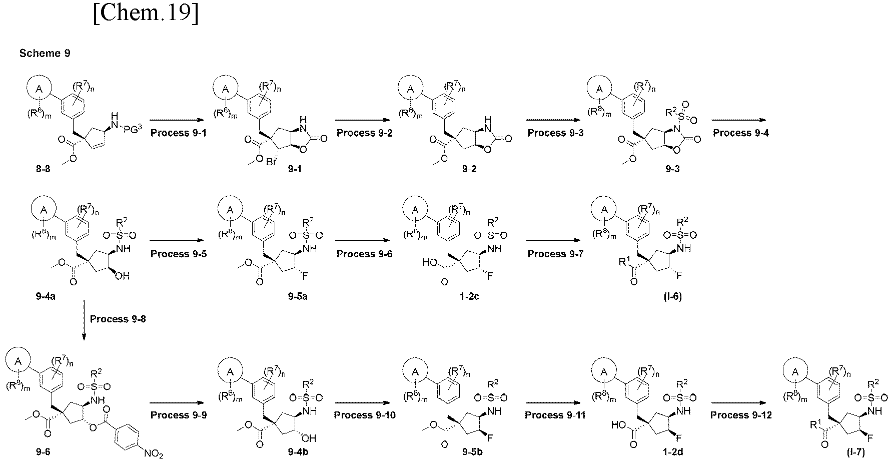

The symbols in the formulae have the same meanings as defined above.

### Process 9-1

Compound (9-1) can also be prepared by reacting compound (8-8) with DMSO and bromotrimethylsilane in the presence of a base.

### Process 9-2

Compound (9-2) can also be prepared by reacting compound (9-1) with 2,2'-azobis(isobutyronitrile) and tris(trimethylsilyl)silane.

### Process 9-3

Compound (9-3) can also be prepared by sulfonylation of compound (9-2).

### Process 9-4

Compound (9-4a) can also be prepared by reacting compound (9-3) with methanol in the presence of a base.

### Process 9-5

Compound (9-5a) can also be prepared by fluorination of compound (9-4a).

### Process 9-6

Compound (1-2c) can also be prepared by hydrolysis of compound (9-5a).

### Process 9-7

Compound (I -6) can also be prepared by amidation of compound (1-2c).

### Process 9-8

Compound (9-6) can also be prepared by Mitsunobu reaction of compound (9-4a) and 4-nitrobenzoic acid.

### Process 9-9

Compound (9-4b) can also be prepared by removing the p-nitrobenzoyl group of compound (9-6). The removal of the p-nitrobenzoyl group can be conducted, for example, by hydrolysis reaction.

### Process 9-10

Compound (9-5b) can also be prepared by fluorination of compound (9-4b).

### Process 9-11

Compound (1-2d) can also be prepared by hydrolysis of compound (9-5b).

### Process 9-12

Compound (I -7) can also be prepared by amidation of compound (1-2d).

A compound represented by the formula (I-8) can be prepared, for example, according to the method described in Scheme 10.

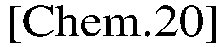

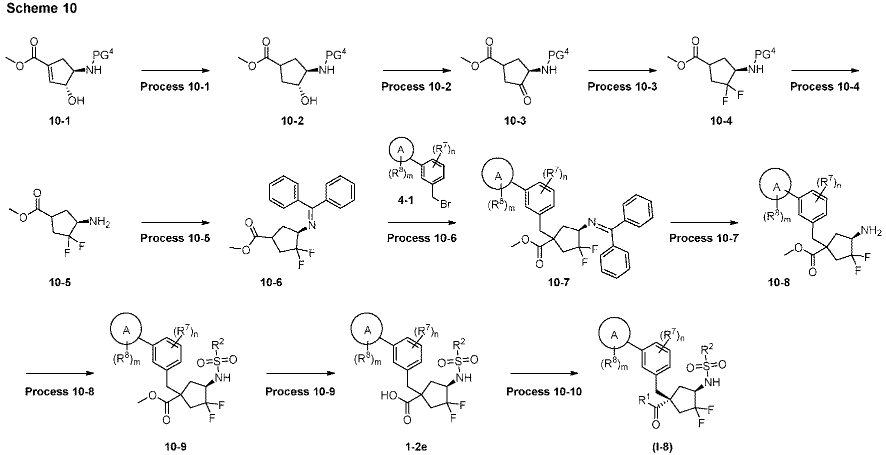

The symbols in the formulae have the same meanings as defined above. PG⁴ represents a protective group.

### Process 10-1

Compound (10-2) can also be prepared by catalytic reduction of compound (10-1).

### Process 10-2

Compound (10-3) can also be prepared by oxidation of compound (10-2).

### Process 10-3

Compound (10-4) can also be prepared by fluorination of compound (10-3).

### Process 10-4

Compound (10-5) can also be prepared by removing the protective group of compound (10-4).

### Process 10-5

Compound (10-6) can also be prepared by reacting compound (10-5) with diphenylmethanimine.

### Process 10-6

Compound (10-7) can also be prepared by reacting compound (10-6) with compound (4-1) in the presence of a base.

### Process 10-7

Compound (10-8) can also be prepared by removing the diphenylmethylidene group of compound (10-7). The removal of the diphenylmethylidene group can be conducted, for example, by hydrolysis reaction.

### Process 10-8

Compound (10-9) can also be prepared by sulfonylation of compound (10-8).

### Process 10-9

Compound (1-2e) can also be prepared by hydrolysis of compound (10-9).

### Process 10-10

Compound (I-8) can also be prepared by amidation of compound (1-2e).

A compound represented by the formula (I-9) can be prepared, for example, according to the method described in Scheme 11.

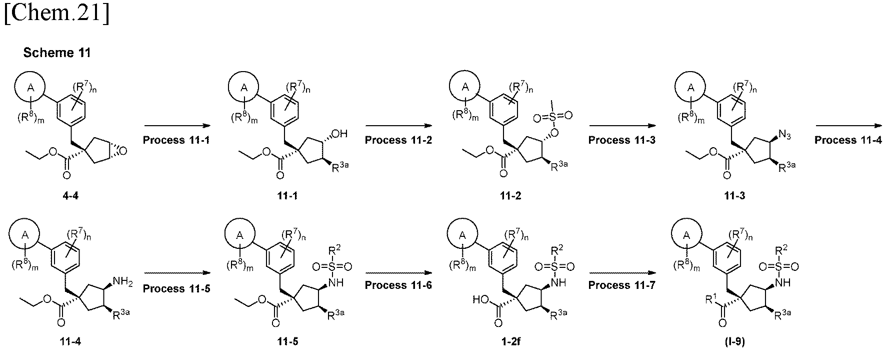

The symbols in the formulae have the same meanings as defined above. R^{3a} represents a C₁₋₆ alkyl group. The stereochemical notations in the formulae represent relative configuration.

### Process 11-1

Compound (11-1) can also be prepared by reacting compound (4-4) with a C₁₋₆ alkyllithium reagent or a C₁₋₆ alkylmagnesium reagent.

### Process 11-2

Compound (11-2) can also be prepared by reacting compound (11-1) with methanesulfonyl chloride in the presence of a base.

### Process 11-3

Compound (11-3) can also be prepared by reacting compound (11-2) with sodium azide.

### Process 11-4

Compound (11-4) can also be prepared by Staudinger reaction or catalytic reduction of compound (11-3).

### Process 11-5

Compound (11-5) can also be prepared by sulfonylation of compound (11-4).

### Process 11-6

Compound (1-2f) can also be prepared by hydrolysis of compound (11-5).

### Process 11-7

Compound (I-9) can also be prepared by amidation of compound (1-2f).

A compound represented by the formula (I-10) can be prepared, for example, according to the method described in Scheme 12.

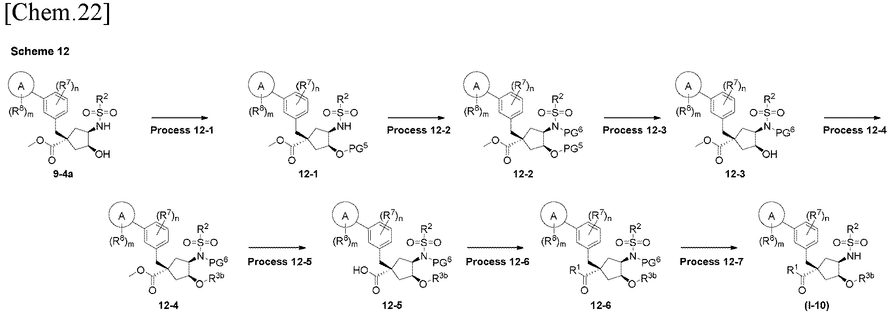

The symbols in the formulae have the same meanings as defined above. PG⁵ and PG⁶ represent protective groups. R^{3b} represents a C₁₋₆ alkyl group.

### Process 12-1

Compound (12-1) can also be prepared by protecting the hydroxy group of compound (9-4a).

### Process 12-2

Compound (12-2) can also be prepared by protecting NH of the sulfonamide of compound (12-1).

### Process 12-3

Compound (12-3) can also be prepared by removing the protective group of the hydroxy group of compound (12-2).

### Process 12-4

Compound (12-4) can also be prepared by reacting compound (12-3) with C₁₋₆ alkyl halide in the presence of a base. As the C₁₋₆ alkyl halide, methyl iodide, ethyl iodide and the like can be illustrated.

### Process 12-5

Compound (12-5) can also be prepared by hydrolysis of compound (12-4).

### Process 12-6

Compound (12-6) can also be prepared by amidation of compound (12-5).

### Process 12-7

Compound (I-10) can also be prepared by removing the protective group of compound (12-6).

The above-mentioned schemes are exemplifications of methods for preparing the compounds represented by the formula (I) or the formula (II) or the synthetic intermediates thereof. The above schemes can be modified in various ways to schemes that can be easily understood by a person ordinarily skilled in the art.

The compounds represented by the formula (I) or the formula (II) and synthetic intermediates thereof can also be isolated and purified, if required, according to isolation and purification means well known to a person ordinarily skilled in the art in the technical field, such as solvent extraction, crystallization, recrystallization, chromatography or preparative high performance liquid chromatography.

The compounds of the present invention have an excellent OX2R agonist activity and thus can be used as agents for the treatment of sleep disorders involving OX2R. In the present invention, sleep disorders involving OX2R include, for example, narcolepsy, idiopathic hypersomnia, Kleine-Levin syndrome, hypersomnia due to physical diseases (hypersomnia due to, for example, sleep apnea syndrome, periodic limb movement, Parkinson's disease, trauma, Niemann-Pick disease type C, Norrie's disease, Prader-Willi syndrome, myotonic dystrophy, Moebius syndrome, fragile X syndrome, cerebral tumor, cerebral infarction, cerebral contusion, infection disease, hypothyroidism, hepatic encephalopathy, renal failure, adrenal insufficiency, pancreatic insufficiency, exposure to poisons, infantile congenital metabolic disorder, progressive supranuclear palsy, hypoventilation syndrome, multiple sclerosis, acute disseminated encephalomyelitis, Guillain-Barre syndrome, Rasmussen's encephalitis, Wernicke's encephalopathy, limbic encephalitis, Hashimoto's encephalopathy, Lewy body dementia, Alzheimer's disease, migraine, headache, or neurogenic pain), hypersomnia due to psychiatric disorders (hypersomnia associated with, for example, depression, anxiety disorder, panic disorder, dependence, obsessive-compulsive disorder, eating disorders, bipolar disorder, epilepsy, schizophrenia, personality disorder, developmental disorder, or post-traumatic stress disorder), hypersomnia due to drugs or substances, circadian rhythm sleep-wake disorder, insufficient sleep syndrome and long sleep. Preferably, the compounds of the present invention can be used as agents for the treatment of narcolepsy, idiopathic hypersomnia, Kleine-Levin syndrome or sleep apnea syndrome. More preferably, the compounds of the present invention can be used as agents for the treatment of narcolepsy (see Patent literature 1)

In the present invention, the term "treatment" includes the meanings of "prevention".

In the present invention, the term "agonist" indicates a drug that activates the function of a target protein, regardless of the binding site thereof. The term "agonist" includes, for example, the meanings of "allosteric agonist" and "positive allosteric modulator (PAM)".

While OX2R is involved in maintaining wakefulness, signaling through OX1R is thought to be involved in the formation of drug dependence by the reward system (for example, see "PLoS One" 2022, Vol. 27, No. 7, e0271901).

In an embodiment, the compounds of the present invention have a selective agonist activity for OX2R among orexin receptors. The agonist activity on OX1R can be measured by well-known methods in the technical field or similar methods thereto (see, for example, Non-patent literature 4).

The therapeutic effects on sleep disorders involving OX2R of the compounds of the present invention can be determined according to well-known methods in the technical field. For example, as the confirmation method of effect in narcolepsy model animals, the methods described in "Neuron" 2001, Vol. 30, No. 2, pp. 345-354 and the like or similar methods thereto can be illustrated.

The pharmaceutical composition of the present invention is used in various dosage forms depending on the usage. As such dosage forms, for example, powders, granules, fine granules, dry syrups, tablets, capsules, injections, liquids, ointments, suppositories, poultices, ophthalmic solution and enema agents can be illustrated.

The pharmaceutical composition of the present invention comprises a compound represented by the formula (I) or the formula (II) or a pharmaceutically acceptable salt thereof as an active ingredient.

Pharmaceutical compositions of the present invention are prepared by using a compound represented by the formula (I) or the formula (II) or a pharmaceutically acceptable salt thereof and at least one pharmaceutical additive. These pharmaceutical compositions can also be formulated by appropriately admixing, diluting or dissolving with appropriate pharmaceutical additives such as excipients, disintegrants, binders, lubricants, diluents, buffers, tonicity agents, preservatives, wetting agents, emulsifying agents, dispersing agents, stabilizing agents, solubilizing agents and the like, according to a known formulation procedure depending upon their dosage forms.

When the pharmaceutical composition of the present invention is used in the treatment, the dosage of the compound represented by the formula (I) or the formula (II) or the pharmaceutically acceptable salt thereof is appropriately decided depending on the age, sex, body weight, degree of disorders and treatment of each patient and the like. The daily dose can be divided into one, two, three or four times and administered.

The dosage for an adult can be decided within the range of, for example, 0.01 to 1000 mg per day in the case of oral or parenteral administration. In an embodiment, the oral administration dosage can be decided within the range of 0.01 to 500 mg per day and is preferably within the range of 0.01 to 100 mg per day.

In an embodiment, the pharmaceutical composition of the present invention can also be used in combination with a medicament other than OX2R agonists. As such other medicaments which can be used in combination for the treatment of sleep disorders involving OX2R, for example, an agent for the treatment of narcolepsy (for example, modafinil, methylphenidate, pemoline, amphetamine, sodium oxybate, pitolisant, solriamfetol and the like), an antidepressant (for example, clomipramine, imipramine, paroxetine, fluvoxamine, milnacipran and the like), caffeine and the like can be illustrated.

When a compound represented by the formula (I) or the formula (II) or a pharmaceutically acceptable salt thereof is used in combination with the other medicament, they can be administered as a formulation comprising these active ingredients or as formulations which are each separately formulated from each active ingredient. When separately formulated, these formulations can be administered separately or concurrently. Furthermore, the dosage of the compound represented by the formula (I) or the formula (II) or a pharmaceutically acceptable salt thereof can be appropriately reduced depending on the dosage of the other medicament used in combination.

The compounds represented by the formula (I) or the formula (II) may be each converted to a prodrug appropriately and be used. For example, a prodrug of a compound represented by the formula (I) can also be prepared by introducing a group forming a prodrug using a corresponding halide compound or the like and purifying. As the group forming a prodrug, for example, a group described in "Development of medicine" (Hirokawa Shoten, 1990), Vol. 7, pp. 163-198 can be illustrated.

### [EXAMPLES]

The present invention is illustrated in more detail below based on Reference Examples, Examples and Test Example. However, the present invention is not limited to the contents thereof.

The compound names described in the following Examples were named using ChemDraw Professional (PerkinElmer), MarvinSketch (ChemAxon) or the like except for commercially available reagents.

The stereo configuration of the chiral center marked with "*" in a compound name means that it is a relative configuration. For example, methyl (1R*,3S*)-1-([1,1'-biphenyl]-3-ylmethyl)-3-azidocyclopentane-1-carboxylate (Reference Examples A-1) indicates the mixture of methyl (1R,3S)- and (1S,3R)-1-([1,1'-biphenyl]-3-ylmethyl)-3-azidocyclopentane-1-carboxylate.

### Reference Example A-1

### Methyl (1R*,3S*)-1-([1,1'-biphenyl]-3-ylmethyl)-3-azidocyclopentane-1-carboxylate

Under an argon atmosphere, to a mixture of ethyl 3-cyclopentene-1-carboxylate (1.00 g) and THF (15 mL) was added LDA (1.09 mol/L in THF/n-hexane, 7.85 mL) at - 78 °C, and the mixture was stirred at the same temperature for 30 minutes. To the reaction mixture was added a mixture of 3-(bromomethyl)-1,1'-biphenyl (1.94 g) and THF (9 mL) at -78 °C. The mixture was stirred at the same temperature for 10 minutes, and then stirred at room temperature for 30 minutes. To the reaction mixture were added a saturated aqueous solution of ammonium chloride and water. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=95/5) to give ethyl 1-([1,1'-biphenyl]-3-ylmethyl)cyclopent-3-ene-1-carboxylate (2.31 g).

A mixture of the obtained compound (2.19 g), 4 mol/L aqueous solution of lithium hydroxide (5 mL), methanol (14 mL) and THF (7 mL) was stirred at 150 °C under microwave irradiation for 15 minutes. To the reaction mixture were added 2 mol/L hydrochloric acid and water. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=80/20-70/30) to give 1-([1,1'-biphenyl]-3-ylmethyl)cyclopent-3-ene-1-carboxylic acid (2.30 g).

To a mixture of DMSO (0.911 g) and chloroform (18 mL) was added bromotrimethylsilane (1.79 g) under ice-cooling, and the mixture was stirred at the same temperature for 3 hours. To the mixture was added a mixture of 1-([1,1'-biphenyl]-3-ylmethyl)cyclopent-3-ene-1-carboxylic acid (1.99 g) and chloroform (9 mL) under ice-cooling, and the mixture was stirred at the same temperature for 10 minutes. To the reaction mixture was added DIPEA (1.47 g) under ice-cooling. The mixture was stirred at 70 °C for 2 hours, and allowed to cool to room temperature. To the reaction mixture was added water. The mixture was extracted with DCM. The extract was dried over anhydrous sodium sulfate, and concentrated under reduced pressure. The residue was purified by column chromatography on APS (eluent: n-hexane/ethyl acetate=95/5-50/50) to give (1S*,4R*,6S*)-4-([1,1'-biphenyl]-3-ylmethyl)-6-bromo-2-oxabicyclo[2.2.1]heptan-3-one (1.70 g).

To a mixture of the obtained compound (1.70 g), 2,2'-azobis(isobutyronitrile) (0.078 g) and toluene (10 mL) was added tris(trimethylsilyl)silane (1.78 g) at room temperature. The mixture was stirred at 90 °C for 30 minutes, and was allowed to cool to room temperature. To the reaction mixture were added water and a saturated aqueous solution of ammonium chloride. The mixture was extracted with ethyl acetate. The extract was dried over anhydrous sodium sulfate, and concentrated under reduced pressure. The residue was purified by column chromatography on silica gel (eluent: n-hexane/ethyl acetate=98/2-70/30) to give (1R*,4R*)-4-([1,1'-biphenyl]-3-ylmethyl)-2-oxabicyclo[2.2.1]heptan-3-one (1.30 g).

A mixture of the obtained compound (1.30 g), hydrogen chloride (4 mol/L in 1,4-dioxane , 5.84 mL) and methanol (10 mL) was stirred at 70 °C for 1 hour. The reaction mixture was allowed to cool to room temperature, and then concentrated under reduced pressure. The residue was purified by column chromatography on silica gel (eluent: n-hexane/ethyl acetate=80/20-30/70) to give methyl (1R*,3R*)-1-([1,1'-biphenyl]-3-ylmethyl)-3-hydroxycyclopentane-1-carboxylate (1.07 g).

To a mixture of the obtained compound (1.07 g), TEA (0.698 g) and DCM (15 mL) was added methanesulfonyl chloride (0.592 g) under ice-cooling, and the mixture was stirred at room temperature for 15 minutes. To the reaction mixture was added water. The mixture was extracted with DCM, and the extract was concentrated under reduced pressure.

To a mixture of the residue and DMSO (15 mL) was added sodium azide (0.672 g) at room temperature. The mixture was stirred at 60 °C for 30 minutes, and allowed to cool to room temperature. To the reaction mixture was added water. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=100/0-80/20) to give the title compound (1.06 g).

### Reference Example A-2

### (1R*,3S*)-1-([1,1'-Biphenyl]-3-ylmethyl)-3-(methylsulfonamido)cyclopentane-1-carboxylic acid

To a mixture of Reference Example A-1 (0.400 g), methanol (6 mL) and water (0.043 mL) was added triphenylphosphine (0.469 g) at room temperature. The mixture was stirred at the same temperature for 9 hours, and concentrated under reduced pressure.

To a mixture of the residue, TEA (0.362 g) and DCM (10 mL) was added methanesulfonyl chloride (0.205 g) under ice-cooling, and the mixture was stirred at the same temperature for 15 minutes. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=80/20-30/70) to give methyl (1R*,3S*)-1-([1,1'-biphenyl]-3-ylmethyl)-3-(methylsulfonamido)cyclopentane-1-carboxylate (0.391 g).

A mixture of the obtained compound (0.230 g), 4 mol/L aqueous solution of lithium hydroxide (0.742 mL), methanol (0.8 mL), THF (0.8 mL) and water (0.2 mL) was stirred at 100 °C under microwave irradiation for 15 minutes. To the reaction mixture was added 2 mol/L hydrochloric acid. The mixture was extracted with ethyl acetate. The extract was washed with brine, dried over anhydrous sodium sulfate, and concentrated under reduced pressure to give the title compound (0.222 g).

### Reference Example A-3

### (1R*,3S*)-1-([1,1'-Biphenyl]-3-ylmethyl)-3-(tert-butoxycarbonylamino)cyclopentane-1-carboxylic acid

To a mixture of Reference Example A-1 (0.250 g), methanol (2 mL) and water (0.027 mL) was added triphenylphosphine (0.293 g) at room temperature. The mixture was stirred at the same temperature for 15 hours, and concentrated under reduced pressure.

To a mixture of the residue, TEA (0.226 g) and DCM (4mL) was added Boc₂O (0.179 g) at room temperature, and the mixture was stirred at the same temperature for 30 minutes. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=100/0-75/25) to give methyl (1R*,3S*)-1-([1,1'-biphenyl]-3-ylmethyl)-3-(tert-butoxycarbonylamino)cyclopentane-1-carboxylate (0.300 g).

A mixture of the obtained compound (0.300 g), 4 mol/L aqueous solution of lithium hydroxide (0.916 mL), methanol (0.8 mL), THF (0.8 mL) and water (0.2 mL) was stirred at 100 °C under microwave irradiation for 15 minutes. To the reaction mixture was added 2 mol/L hydrochloric acid. The mixture was extracted with ethyl acetate. The extract was washed with brine, dried over anhydrous sodium sulfate, and concentrated under reduced pressure to give the title compound (0.289 g).

### Reference Example A-4

### (1R*,3S*)-1-([1,1'-Biphenyl]-3-ylmethyl)-3-amino-N-methoxy-N-methylcyclopentane-1-carboxamide hydrochloride

To a mixture of Reference Example A-3 (0.289 g), N,O-dimethylhydroxylamine hydrochloride (0.143 g), DIPEA (0.472 g) and DMF (4 mL) was added HATU (0.306 g) at room temperature. The mixture was stirred at 70 °C for 2 hours, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=80/20-50/50) to give tert-butyl ((1S*,3R*)-3-([1,1'-biphenyl]-3-ylmethyl)-3-(methoxy(methyl)carbamoyl)cyclopentyl)carbamate (0.270 g).

A mixture of the obtained compound (0.270 g) and hydrogen chloride (4 mol/L in 1,4-dioxane, 2 mL) was stirred at room temperature for 1 hour. The reaction mixture was concentrated under reduced pressure to give the title compound (0.230 g).

### Reference Example B-1

### Methyl (1R*,3S*)-3-azido-1-(3-chlorobenzyl)cyclopentane-1-carboxylate

Under an argon atmosphere, to a mixture of ethyl 3-cyclopentene-1-carboxylate (2.00 g) and THF (32 mL) was added LDA (1.09 mol/L in THF/n-hexane, 15.7 mL) at - 78 °C, and the mixture was stirred at the same temperature for 30 minutes. To the reaction mixture was added a mixture of 3-chlorobenzyl bromide (3.22 g) and THF (16 mL) at - 78 °C, and the mixture was stirred at the same temperature for 1 hour. To the reaction mixture were added a saturated aqueous solution of ammonium chloride and water under ice-cooling. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=100/0-95/5) to give ethyl 1-(3-chlorobenzyl)cyclopent-3-ene-1-carboxylate (3.50 g).

A mixture of the obtained compound (3.50 g), 2 mol/L aqueous solution of sodium hydroxide (33 mL), methanol (66 mL) and THF (33 mL) was stirred at 70 °C for 3.5 hours, and allowed to cool to room temperature. To the reaction mixture was added 2 mol/L hydrochloric acid. The mixture was extracted with ethyl acetate. The extract was washed with water and brine, dried over anhydrous sodium sulfate, and concentrated under reduced pressure to give 1-(3-chlorobenzyl)cyclopent-3-ene-1-carboxylic acid (3.06 g).

To a mixture of DMSO (1.31 g) and chloroform (25 mL) was added bromotrimethylsilane (2.57 g) under ice-cooling, and the mixture was stirred at the same temperature for 2 hours. To the reaction mixture was added a mixture of 1-(3-chlorobenzyl)cyclopent-3-ene-1-carboxylic acid (3.06 g) and chloroform (18 mL) under ice-cooling, and the mixture was stirred at the same temperature for 10 minutes. To the reaction mixture was added DIPEA (2.17 g) under ice-cooling. The mixture was stirred at 70 °C for 2 hours, and allowed to cool to room temperature. To the reaction mixture was added water. The mixture was extracted with DCM, and the extract was concentrated under reduced pressure. The residue was purified by column chromatography on silica gel (eluent: n-hexane/ethyl acetate=98/2-80/20) to give (1S*,4R*,6S*)-6-bromo-4-(3-chlorobenzyl)-2-oxabicyclo[2.2.1]heptan-3-one (2.20 g).

To a mixture of the obtained compound (2.20 g), 2,2'-azobis(isobutyronitrile) (0.115 g) and toluene (35 mL) was added tris(trimethylsilyl)silane (2.60 g) at room temperature. The mixture was stirred at 80 °C for 1 hour, and was allowed to cool to room temperature. To the reaction mixture was added a saturated aqueous solution of ammonium chloride. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=95/5-80/20) to give (1R*,4R*)-4-(3-chlorobenzyl)-2-oxabiscyclo[2.2.1]heptan-3-one (1.52 g).

A mixture of the obtained compound (1.52 g), hydrogen chloride (4 mol/L in 1,4-dioxane, 16 mL) and methanol (13 mL) was stirred at 70 °C for 1 hour. The reaction mixture was allowed to cool to room temperature, and then concentrated under reduced pressure. The residue was purified by column chromatography on silica gel (eluent: n-hexane/ethyl acetate=90/10-40/60) to give methyl (1R*,3R*)-1-(3-chlorobenzyl)-3-hydroxycyclopentane-1-carboxylate (1.29 g).

To a mixture of the obtained compound (1.29 g), TEA (0.968 g) and DCM (16 mL) was added methanesulfonyl chloride (0.822 g) under ice-cooling, and the mixture was stirred at the same temperature for 50 minutes. The reaction mixture was added to water, and extracted with DCM. The extract was concentrated under reduced pressure.

To a mixture of the residue and DMSO (16 mL) was added sodium azide (0.933 g) at room temperature. The mixture was stirred at 60 °C for 30 minutes, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=98/2-90/10) to give the title compound (1.24 g).

### Reference Example B-2

### Methyl (1R*,3S*)-1-(3-chlorobenzyl)-3-(methylsulfonamido)cyclopentane-1-carboxylate

To a mixture of Reference Example B-1 (1.00 g), methanol (8 mL) and water (0.123 mL) was added triphenylphosphine (1.34 g) at room temperature.

The mixture was stirred at the same temperature for 7 hours, and concentrated under reduced pressure. To a mixture of the residue, TEA (1.03 g) and DCM (8 mL) was added methanesulfonyl chloride (0.585 g) under ice-cooling, and the mixture was stirred at the same temperature for 15 minutes. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=70/30-50/50) to give the title compound (1.00 g).

### Reference Example B-3

### (1R*,3S*)-1-(3-Chlorobenzyl)-N-methoxy-N-methyl-3-(methylsulfonamido)cyclopentane-1-carboxamide

To a mixture of Reference Example B-2 (1.38 g) and methanol (15 mL) was added 2 mol/L aqueous solution of sodium hydroxide (10 mL) at room temperature. The mixture was stirred at 60 °C for 4 hours, and allowed to cool to room temperature. To the reaction mixture was added 2 mol/L hydrochloric acid. The mixture was extracted with ethyl acetate. The extract was washed with water and brine, dried over anhydrous sodium sulfate, and concentrated under reduced pressure.

To a mixture of the residue, N,O-dimethylhydroxylamine hydrochloride (1.51 g), DIPEA (2.50 g) and DMF (19 mL) was added HATU (1.62 g) at room temperature. The mixture was stirred at 70 °C for 2 hours, and allowed to cool to room temperature. To the reaction mixture was added water. The mixture was extracted with ethyl acetate. The extract was washed with water and brine, dried over anhydrous magnesium sulfate, and concentrated under reduced pressure. The residue was purified by column chromatography on silica gel (eluent: n-hexane/ethyl acetate=50/50-0/100) to give the title compound (1.25 g).

### Reference Example B-4

### (1R*,3S*)-N-Methoxy-N-methyl-3-(methylsulfonamido)-1-(3-(4,4,5,5-tetramethyl-1,3,2-dioxaborolan-2-yl)benzyl)cyclopentane-1-carboxamide

A mixture of Reference Example B-3 (0.177 g), bis(pinacolato)diboron (0.359 g), potassium acetate (0.139 g), Pd(dppf)Cl₂ DCM adduct (0.039 g), Xphos (0.045 g) and 1,4-dioxane (3 mL) was stirred at 110 °C under microwave irradiation for 1 hour. The reaction mixture was filtered through a pad of Celite, and the filtrate was concentrated under reduced pressure. The residue was purified by column chromatography on silica gel (eluent: n-hexane/ethyl acetate=50/50-0/100) to give the title compound (0.201 g).

### Reference Example B-5

Reference Example B-5 was synthesized in a manner similar to that described in Reference Example B-4 using Reference Example B-2 instead of Reference Example B-3.

### Reference Example B-6 to Reference Example B-9

Reference Example B-6 to Reference Example B-9 were synthesized in a manner similar to that described in Reference Example B-1 using corresponding reagents instead of 3-chlorobenzyl bromide.

### Reference Example B-10 to Reference Example B-13

Reference Example B-10 to Reference Example B-13 were synthesized in a manner similar to that described in Reference Example B-2 using the corresponding reference examples instead of Reference Example B-1.

### Reference Example B-14 to Reference Example B-17

Reference Example B-14 to Reference Example B-17 were synthesized in a manner similar to that described in Reference Example B-3 using the corresponding reference examples instead of Reference Example B-2.

### Reference Example B-18 to Reference Example B-21

Reference Example B-18 to Reference Example B-21 were synthesized in a manner similar to that described in Reference Example B-4 using the corresponding reference examples instead of Reference Example B-3.

### Reference Example B-22

Reference Example B-22 was synthesized in a manner similar to that described in Reference Example A-3 using Reference Example B-1 instead of Reference Example A-1.

### Reference Example C-1

### (1R*,3S*)-3-(Methylsulfonamido)-1-(3-(pyrimidin-2-yl)benzyl)cyclopentane-1-carboxylic acid

A mixture of Reference Example B-5 (0.030 g), 2-bromopyrimidine (0.022 g), sodium carbonate (0.022 g), Pd(dppf)Cl₂ DCM adduct (0.006 g), 1,4-dioxane (1 mL), ethanol (0.5 mL) and water (0.25 mL) was stirred at 90 °C under microwave irradiation for 1 hour. The reaction mixture was filtered through a pad of Celite, and the filtrate was concentrated under reduced pressure. The residue was purified by column chromatography on silica gel (eluent: n-hexane/ethyl acetate=50/50-0/100), and following column chromatography on APS (eluent: n-hexane/ethyl acetate=50/50-0/100) to give methyl (1R*,3S*)-3-(methylsulfonamido)-1-(3-(pyrimidin-2-yl)benzyl)cyclopentane-1-carboxylate (0.016 g).

To a mixture of the obtained compound (0.016 g) and methanol (1 mL) was added 2 mol/L aqueous solution of sodium hydroxide (0.421 mL) at room temperature. The mixture was stirred at 60 °C for 8.5 hours, and allowed to cool to room temperature. To the reaction mixture were added 2 mol/L hydrochloric acid and water. The mixture was extracted with ethyl acetate. The extract was washed with water and brine, dried over anhydrous sodium sulfate, and concentrated under reduced pressure. The residue was purified by column chromatography on silica gel (eluent: ethyl acetate/methanol=100/0-80/20) to give the title compound (0.015 g).

### Reference Example C-2

### (1R*,3S*)-3-Amino-N-methoxy-N-methyl- 1-(3-(pyrimidin-2-yl)benzyl)cyclopentane-1-carboxamide hydrochloride

To a mixture of Reference Example B-22 (0.136 g) and DCM (3 mL) were added oxalyl chloride (0.058 g) and DMF (4.8 mg) at room temperature, and the mixture was stirred at the same temperature for 30 minutes. To the reaction mixture were added N,O-dimethylhydroxylamine hydrochloride (0.187 g) and DIPEA(0.497 g) at room temperature, and the mixture was stirred at the same temperature for 30 minutes. To the reaction mixture was added water. The mixture was extracted with DCM, and the extract was concentrated under reduced pressure. The residue was purified by column chromatography on silica gel (eluent: n-hexane/ethyl acetate=30/70-0/100) to give tert-butyl ((1S*,3R*)-3-(3-chlorobenzyl)-3-(methoxy(methyl)carbamoyl)cyclopentyl)carbamate (0.104 g).

A mixture of the obtained compound (0.104 g), bis(pinacolato)diboron (0.203 g), potassium acetate (0.079 g), Pd(dppf)Cl₂ (0.020 g), Xphos (0.025 g) and 1,4-dioxane (2 mL) was stirred at 110 °C under microwave irradiation for 1 hour. To the reaction mixture was added water. The mixture was extracted with ethyl acetate. The extract was dried over anhydrous sodium sulfate, and concentrated under reduced pressure. The residue was purified by column chromatography on silica gel (eluent: n-hexane/ethyl acetate=80/20-0/100) to give tert-butyl ((1S*,3R*)-3-(methoxy(methyl)carbamoyl)-3-(3-(4,4,5,5-tetramethyl-1,3,2-dioxaborolan-2-yl)benzyl)cyclopentyl)carbamate (0.106 g).

A mixture of the obtained compound (0.052 g), 2-bromopyrimidine (0.068 g), sodium carbonate (0.068 g), Pd(dppf)Cl₂ DCM adduct (0.009 g), 1,4-dioxane (1 mL), ethanol (0.5 mL) and water (0.5 mL) was stirred at 90 °C under microwave irradiation for 1 hour. The reaction mixture was concentrated under reduced pressure. The residue was purified by column chromatography on silica gel (eluent: n-hexane/ethyl acetate=80/20-30/70) to give tert-butyl ((1S*,3R*)-3-(methoxy(methyl)carbamoyl)-3-(3-(pyrimidin-2-yl)benzyl)cyclopentyl)carbamate (0.026 g).

A mixture of the obtained compound (0.026 g) and hydrogen chloride (4 mol/L in 1,4-dioxane, 1 mL) was stirred at room temperature for 1 hour. The reaction mixture was concentrated under reduced pressure to give the title compound (0.021 g).

### Reference Example D-1

### 5-Bromo-2-(3-(bromomethyl)phenyl)pyrimidine

To a mixture of (3-(5-bromopyrimidin-2-yl)phenyl)methanol (1.07 g), TEA (0.531 g) and ethyl acetate (30 mL) was added methanesulfonyl chloride (0.509 g) under ice-cooling, and the mixture was stirred at room temperature for 2 hours. The reaction mixture was filtered. To the filtrate was added lithium bromide monohydrate (1.27 g), and the mixture was stirred at 60 °C for 1 hour. The reaction mixture was allowed to cool to room temperature, and filtered. The filtrate was added to water, and the mixture was extracted with ethyl acetate. The extract was dried over anhydrous sodium sulfate, and concentrated under reduced pressure to give the title compound (1.13 g).

### Reference Example D-2

### Reference Example D-2 was synthesized in a manner similar to that described in Reference Example D-1 using (3-(5-fluoropyrimidin-2-yl)phenyl)methanol instead of (3-(5-bromopyrimidin-2-yl)phenyl)methanol.

### Reference Example D-3

### (4-Fluoro-3-(5-fluoropyrimidin-2-yl)phenyl)methanol

A mixture of (2-fluoro-5-(hydroxymethyl)phenyl)boronic acid (3.07 g), 2-chloro-5-fluoropyrimidine (3.17 g), cesium carbonate (8.83 g), Pd(PPh₃)₄ (0.418 g), DMF (45 mL) and water (9 mL) was stirred at 100 °C for 2 hours, and allowed to cool to room temperature. To the reaction mixture was added water. The mixture was extracted with ethyl acetate. The extract was dried over anhydrous sodium sulfate, and concentrated under reduced pressure. The residue was purified by column chromatography on silica gel (eluent: n-hexane/ethyl acetate=50/50-20/80) to give the title compound (2.17 g).

### Reference Example D-4

### (2-Fluoro-5-(5-fluoropyrimidin-2-yl)phenyl)methanol

A mixture of (4-fluoro-3-(hydroxymethyl)phenyl)boronic acid (1.01 g), 2-chloro-5-fluoropyrimidine (1.02 g), cesium carbonate (2.90 g), Pd(PPh₃)₄ (0.137 g), DMF (15 mL) and water (3 mL) was stirred at 100 °C under microwave irradiation for 1.5 hours. The reaction mixture was added to water, and extracted with ethyl acetate. The extract was dried over anhydrous sodium sulfate, and concentrated under reduced pressure. The residue was purified by column chromatography on silica gel (eluent: n-hexane/ethyl acetate=90/10-85/15) to give the title compound (1.05 g).

### Reference Example D-5

### (3-(5-Fluoropyrimidin-2-yl)-4-methoxyphenyl)methanol

A mixture of (5-(hydroxymethyl)-2-methoxyphenyl)boronic acid (1.00 g), 2-chloro-5-fluoropyrimidine (0.947 g), cesium carbonate (2.69 g), Pd(PPh₃)₄ (0.127 g), DMF (15 mL) and water (2 mL) was stirred at 120 °C under microwave irradiation for 30 minutes. The reaction mixture was added to water, and extracted with ethyl acetate. The extract was washed with water, and then was concentrated under reduced pressure. The residue was purified by column chromatography on silica gel (eluent: n-hexane/ethyl acetate/methanol=50/50/0-0/100/0-0/80/20) to give the title compound (0.466 g).

### Reference Example D-6

### (5-(5-Bromopyrimidin-2-yl)-2-fluorophenyl)methanol

A mixture of (4-fluoro-3-(hydroxymethyl)phenyl)boronic acid (0.869 g), 5-bromo-2-iodopyrimidine (1.60 g), sodium carbonate (1.08 g), PdCl₂(PPh₃)₂ (0.179 g), toluene (6mL), ethanol (6 mL) and water (3 mL) was stirred at 90 °C under microwave irradiation for 2 hours. The reaction mixture was added to water, and extracted with ethyl acetate. The extract was dried over anhydrous sodium sulfate, and concentrated under reduced pressure. The residue was purified by column chromatography on silica gel (eluent: n-hexane/ethyl acetate=90/10-80/20) to give the title compound (0.932 g).

### Reference Example D-7

### (4-Chloro-3-(5-fluoropyrimidin-2-yl)phenyl)methanol

A mixture of (2-chloro-5-formylphenyl)boronic acid (0.300 g), 2-chloro-5-fluoropyrimidine (0.280 g), sodium carbonate (0.259 g), Pd(PPh₃)₄ (0.094 g), toluene (1.5 mL), ethanol (0.5 mL) and water (1.5 mL) was stirred at 110 °C under microwave irradiation for 3 hours. To the reaction mixture was added water. The mixture was extracted with ethyl acetate. The extract was washed with brine, dried over anhydrous magnesium sulfate, and concentrated under reduced pressure. The residue was purified by column chromatography on silica gel (eluent: n-hexane/ethyl acetate=90/10-60/40) to give the 4-chloro-3-(5-fluoropyrimidin-2-yl)benzaldehyde (0.081 g).

To a mixture of the obtained compound (0.080 g) and ethanol (2 mL) was added sodium borohydride (0.013 g) at room temperature, and the mixture was stirred at the same temperature for 20 minutes. To the reaction mixture were added a saturated aqueous solution of ammonium chloride and water. The mixture was extracted with DCM, and the extract was concentrated under reduced pressure. The residue was purified by column chromatography on silica gel (eluent: n-hexane/ethyl acetate=50/50-20/80) to give the title compound (0.055 g).

### Reference Example D-8

### (4-(Benzyloxy)-3-(5-fluoropyrimidin-2-yl)phenyl)methanol

A mixture of (2-(benzyloxy)-5-formylphenyl)boronic acid (3.00 g), 2-chloro-5-fluoropyrimidine (2.02 g), sodium carbonate (1.86 g), PdCl₂(PPh₃)₂ (0.411 g), toluene (10 mL), ethanol (20 mL) and water (10 mL) was stirred at 80 °C for 45 minutes, and allowed to cool to room temperature. To the reaction mixture were added a saturated aqueous solution of ammonium chloride and water. The mixture was extracted with ethyl acetate. The extract was dried over anhydrous sodium sulfate, and concentrated under reduced pressure. The residue was purified by column chromatography on silica gel (eluent: n-hexane/ethyl acetate=90/10-10/90) to give the 4-(benzyloxy)-3-(5-fluoropyrimidin-2-yl)benzaldehyde (3.23 g).

To a mixture of the obtained compound (3.15 g), ethanol (30 mL) and THF (30 mL) was added sodium borohydride (0.193 g) under ice-cooling, and the mixture was stirred at the same temperature for 5 minutes. To the reaction mixture was added water under ice-cooling. The mixture was extracted with ethyl acetate. The extract was dried over anhydrous sodium sulfate, and concentrated under reduced pressure. The residue was purified by Method A (eluent: n-hexane/ethyl acetate=60/40-40/60) to give the title compound (1.05 g).

### Reference Example D-9

### 2-(5-(Bromomethyl)-2-fluorophenyl)-5-fluoropyrimidine

To a mixture of Reference Example D-3 (2.17 g), TEA (1.98 g) and ethyl acetate (30 mL) was added methanesulfonyl chloride (1.68 g) under ice-cooling, and the mixture was stirred at room temperature for 1 hour. The reaction mixture was filtered, and the filtrate was concentrated under reduced pressure. To a mixture of the residue and ethyl acetate (30 mL) was added lithium bromide monohydrate (3.07 g) at room temperature and the mixture was stirred at 60 °C for 1 hour. The reaction mixture was allowed to cool to room temperature, and added to water. The mixture was extracted with ethyl acetate. The extract was dried over anhydrous sodium sulfate, and concentrated under reduced pressure to give the title compound (2.58 g).

### Reference Example D-10 to Reference Example D-14

Reference Example D-10 to Reference Example D-14 were synthesized in a manner similar to that described in Reference Example D-9 using the corresponding reference examples instead of Reference Example D-3.

### Reference Example E-1 and Reference Example E-2

### Ethyl (1R,3s,5S)-3-(3-(5-bromopyrimidin-2-yl)benzyl)-6-oxabicyclo[3.1.0]hexane-3-carboxylate (Reference Example E-1)

### Ethyl (1R,3r,5S)-3-(3-(5-bromopyrimidin-2-yl)benzyl)-6-oxabicyclo[3.1.0]hexane-3-carboxylate (Reference Example E-2)

Under an argon atmosphere, to a mixture of ethyl cyclopent-3-ene-1-carboxylate (1.00 g) and THF (20 mL) was added LDA (1.09 mol/L in THF/n-hexane, 7.2 mL) at - 78 °C, and the mixture was stirred at the same temperature for 30 minutes. To the reaction mixture was added a mixture of Reference Example D-1 (2.40 g) and THF (30 mL) at - 78 °C, and the mixture was stirred at room temperature for 1 hour. To the reaction mixture were added a saturated aqueous solution of ammonium chloride and water. The mixture was extracted with ethyl acetate. The extract was washed with brine, dried over anhydrous magnesium sulfate, and concentrated under reduced pressure. The residue was purified by column chromatography on silica gel (eluent: n-hexane/ethyl acetate=100/0-90/10) to give ethyl 1-(3-(5-bromopyrimidin-2-yl)benzyl)cyclopent-3-ene-1-carboxylate (2.06 g).

To a mixture of the obtained compound (2.00 g) and DCM (20 mL) was added m-chloroperbenzoic acid (1.92 g) under ice-cooling. The mixture was stirred at the same temperature for 1 hour, and then stirred at room temperature for 1 hour. To the reaction mixture were added 1 mol/L aqueous solution of sodium thiosulfate (10 mL), a saturated aqueous solution of sodium bicarbonate and water. The mixture was extracted with DCM. The extract was washed with a saturated aqueous solution of sodium bicarbonate and brine, dried over anhydrous sodium sulfate, and concentrated under reduced pressure. The residue was purified by column chromatography on silica gel (eluent: n-hexane/ethyl acetate=70/30) to give the title compounds.
Reference Example E-1 (0.889 g); ¹H-NMR (CDCl₃) δ ppm : 1.27 (3H, t, J=7.1Hz), 2.13-2.30 (4H, m), 3.17 (2H, s), 3.58 (2H, s), 4.16 (2H, q, J=7.1Hz), 7.16-7.31 (1H, m), 7.32-7.42 (1H, m), 8.14-8.21 (1H, m), 8.21-8.27 (1H, m), 8.81 (2H, s)
Reference Example E-2 (0.909 g); ¹H-NMR (CDCl₃) δ ppm : 1.06-1.17 (3H, m), 1.71 (2H, d, J=14.3Hz), 2.88 (2H, d, J=14.3Hz), 2.96 (2H, s), 3.42 (2H, s), 3.98-4.09 (2H, m), 7.15-7.23 (1H, m), 7.34-7.44 (1H, m), 8.10-8.18 (1H, m), 8.23-8.32 (1H, m), 8.82 (2H, s)

### Reference Example E-3

### Ethyl (1R*,3R*,4R*)-3-azido-1-(3-(5-bromopyrimidin-2-yl)benzyl)-4-hydroxycyclopentane-1-carboxylate

A mixture of Reference Example E-2 (0.900 g), sodium azide (0.435 g), ammonium chloride (0.358 g), ethanol (10 mL) and water (1 mL) was stirred at 110 °C under microwave irradiation for 1 hour. To the reaction mixture was added water. The mixture was extracted with ethyl acetate. The extract was washed with brine, dried over anhydrous magnesium sulfate, and concentrated under reduced pressure to give the title compound (0.936 g).

### Reference Example E-4

### (1R*,3R*,4S*)-3-Azido- 1-(3-(5-bromopyrimidin-2-yl)benzyl)-4-fluorocyclopentane-1-carboxylic acid

To a mixture of the Reference Example E-3 (0.150 g), pyridine (0.080 g) and 1,2-dichloroethane (3 mL) was added Deoxo-Fluor (0.112 g) at room temperature, and the mixture was stirred at 40 °C for 1 hour. To the reaction mixture were added a saturated aqueous solution of sodium bicarbonate and water under ice-cooling. The mixture was extracted with DCM. The extract was dried over anhydrous sodium sulfate, and concentrated under reduced pressure. The residue was purified by column chromatography on silica gel (eluent: n-hexane/ethyl acetate=100/0-80/20) to give Ethyl (1R*,3R*,4S*)-3-azido-1-(3-(5-bromopyrimidin-2-yl)benzyl)-4-fluorocyclopentane-1-carboxylate (0.066 g).

A mixture of the obtained compound (0.064 g), 2 mol/L aqueous solution of sodium hydroxide (0.357 mL) and ethanol (1.4 mL) was stirred at 80 °C for 2 hours, and allowed to cool to room temperature. To the reaction mixture were added 2 mol/L hydrochloric acid (0.393 mL), water and brine. The mixture was extracted with ethyl acetate. The extract was dried over anhydrous magnesium sulfate, and concentrated under reduced pressure to give the title compound (0.055 g).

### Reference Example E-5

### (1R*,3R*,4S*)-3-Azido-1-(3-(5-bromopyrimidin-2-yl)benzyl)-4-fluoro-N-methoxy-N-methylcyclopentane-1-carboxamide

To a mixture of Reference Example E-4 (0.054 g), DMF (1 mg) and DCM (1.5 mL) was added oxalyl chloride(0.033 g) under ice-cooling. The mixture was stirred at room temperature for 45 minutes, and concentrated under reduced pressure. A mixture of the residue and DCM (1mL) was added to a mixture of N,O-dimethylhydroxylamine hydrochloride (0.025 g), DIPEA (0.050 g) and DCM (1mL) under ice-cooling. The mixture was stirred at room temperature for 10 minutes. To the reaction mixture were added water and brine. The mixture was extracted with DCM, and the extract was concentrated under reduced pressure. The residue was purified by column chromatography on silica gel (eluent: n-hexane/ethyl acetate=70/30-50/50) to give the title compound (0.054 g).

### Reference Example E-6

### Ethyl (1R*,3R*,4S*)-3-azido-1-(3-(5-bromopyrimidin-2-yl)benzyl)-4-hydroxycyclopentane-1-carboxylate

To a mixture of Reference Example E-3 (0.500 g), 4-nitorobenzoic acid (0.225 g), triphenylphosphine (0.353 g) and THF (5 mL) was added diisopropyl azodicarboxylate (1.9 mol/L in toluene, 0.708 mL) at room temperature. The mixture was stirred at the same temperature for 22 hours, and concentrated under reduced pressure. The residue was purified by column chromatography on silica gel (eluent: n-hexane/ethyl acetate=100/0-80/20) to give (1S*,2R*,4R*)-2-azido-4-(3-(5-bromopyrimidin-2-yl)benzyl)-4-(ethoxycarbonyl)cyclopentyl 4-nitrobenzoate (0.526 g).

To a mixture of the obtained compound (0.520 g), ethanol (2 mL) and THF (2 mL) was added 2 mol/L aqueous solution of sodium hydroxide (0.873 mL), and the mixture was stirred at room temperature for 1 hour. To the reaction mixture were added water and a saturated aqueous solution of sodium bicarbonate. The mixture was extracted with ethyl acetate. The extract was washed with a saturated aqueous solution of sodium bicarbonate and brine, dried over anhydrous magnesium sulfate, and concentrated under reduced pressure to give the title compound (0.375 g).

### Reference Example E-7

### (1R*,3R*,4R*)-3-Azido-1-(3-(5-bromopyrimidin-2-yl)benzyl)-4-fluorocyclopentane-1-carboxylic acid

To a mixture of the Reference Example E-6 (0.370 g), pyridine (0.197 g) and DCM (7 mL) was added Deoxo-Fluor (0.275 g) under ice-cooling, and the mixture was stirred at room temperature for 16 hours. To the reaction mixture were added a saturated aqueous solution of sodium bicarbonate and water under ice-cooling. The mixture was extracted with DCM. The extract was dried over anhydrous sodium sulfate, and concentrated under reduced pressure. The residue was purified by column chromatography on silica gel (eluent: n-hexane/ethyl acetate=100/0-80/20) to give Ethyl (1R*,3R*,4R*)-3-azido-1-(3-(5-bromopyrimidin-2-yl)benzyl)-4-fluorocyclopentane-1-carboxylate (0.229 g).

A mixture of the obtained compound (0.225 g), 2 mol/L aqueous solution of sodium hydroxide (1.26 mL) and ethanol (5 mL) was stirred at 80 °C for 3 hours, and allowed to cool to room temperature. To the reaction mixture were added 2 mol/L hydrochloric acid (1.38 mL), water and brine. The mixture was extracted with ethyl acetate. The extract was dried over anhydrous magnesium sulfate, and concentrated under reduced pressure to give the title compound (0.192 g).

### Reference Example E-8

Reference Example E-8 was synthesized in a manner similar to that described in Reference Example E-5 using Reference Example E-7 instead of Reference Example E-4.

### Reference Example E-9 and Reference Example E-10

Reference Example E-9 and Reference Example E-10 were synthesized in a manner similar to that described in Reference Example E-1 and Reference Example E-2 using Reference Example D-2 instead of Reference Example D-1.

### Reference Example E-11

Reference Example E-11 was synthesized in a manner similar to that described in Reference Example E-3 using Reference Example E-10 instead of Reference Example E-2.

### Reference Example E-12

Reference Example E-12 was synthesized in a manner similar to that described in Reference Example E-7 using Reference Example E-11 instead of Reference Example E-6.

### Reference Example E-13

Reference Example E-13 was synthesized in a manner similar to that described in Reference Example E-5 using Reference Example E-12 instead of Reference Example E-4.

### Reference Example E-14 and Reference Example E-15

Reference Example E-14 and Reference Example E-15 were synthesized in a manner similar to that described in Reference Example E-1 and Reference Example E-2 using Reference Example D-9 instead of Reference Example D-1.

### Reference Example E-16

Reference Example E-16 was synthesized in a manner similar to that described in Reference Example E-3 using Reference Example E-15 instead of Reference Example E-2.

### Reference Example E-17

### (1R*,3R*,4S*)-3-Azido-4-fluoro-1-(4-fluoro-3-(5-fluoropyrimidin-2-yl)benzyl)cyclopentane-1-carboxylic acid

To a mixture of Reference Example E-16 (0.379 g), pyridine (0.223 g) and DCM (7 mL) was added Deoxo-Fluor (0.312 g) under ice-cooling. The mixture was stirred at the same temperature for 1 hour, and then stirred at room temperature for 2 hours. The reaction mixture was added to a saturated aqueous solution of sodium bicarbonate under ice-cooling, and extracted with DCM. The extract was dried over anhydrous sodium sulfate, and concentrated under reduced pressure. The residue was purified by column chromatography on silica gel (eluent: n-hexane/ethyl acetate=95/5-80/20) to give ethyl (1R*,3R*,4S*)-3-azido-4-fluoro-1-(4-fluoro-3-(5-fluoropyrimidin-2-yl)benzyl)cyclopentane-1-carboxylate (0.184 g).

A mixture of the obtained compound (0.184 g), 2 mol/L aqueous solution of sodium hydroxide (1.14 mL) and 2-propanol (2 mL) was stirred at 60 °C for 3.5 hours, and allowed to cool to room temperature. To the reaction mixture were added 2 mol/L hydrochloric acid (1.25 mL) and water. The mixture was extracted with DCM. The extract was dried over anhydrous magnesium sulfate, and concentrated under reduced pressure to give the title compound (0.196 g).

### Reference Example E-18

Reference Example E-18 was synthesized in a manner similar to that described in Reference Example E-5 using Reference Example E-17 instead of Reference Example E-4.

### Reference Example E-19

### (1R*,3R*,4S*)-3-Amino-4-fluoro-1-(4-fluoro-3-(5-fluoropyrimidin-2-yl)benzyl)-N-methoxy-N-methylcyclopentane-1-carboxamide

To a mixture of Reference Example E-18 (0.175 g), ethanol (1.5 mL) and THF (1.5 mL) was added 10% Pd/C (0.035 g) under ice-cooling. The mixture was stirred under a hydrogen atmosphere at room temperature for 1 hour. The reaction mixture was filtered through a pad of Celite. The filtrate was concentrated under reduced pressure to give the title compound (0.145 g).

### Reference Example E-20 and Reference Example E-21

Reference Example E-20 and Reference Example E-21 were synthesized in a manner similar to that described in Reference Example E-1 and Reference Example E-2 using Reference Example D-10 instead of Reference Example D-1.

### Reference Example E-22

Reference Example E-22 was synthesized in a manner similar to that described in Reference Example E-3 using Reference Example E-21 instead of Reference Example E-2.

### Reference Example E-23

Reference Example E-23 was synthesized in a manner similar to that described in Reference Example E-17 using Reference Example E-22 instead of Reference Example E-16.

### Reference Example E-24

Reference Example E-24 was synthesized in a manner similar to that described in Reference Example E-5 using Reference Example E-23 instead of Reference Example E-4.

### Reference Example E-25

Reference Example E-25 was synthesized in a manner similar to that described in Reference Example E-19 using Reference Example E-24 instead of Reference Example E-18.

### Reference Example E-26 and Reference Example E-27

Reference Example E-26 and Reference Example E-27 were synthesized in a manner similar to that described in Reference Example E-1 and Reference Example E-2 using Reference Example D-12 instead of Reference Example D-1.

### Reference Example E-28

### Ethyl (1R*,3R*,4R*)-3-azido-1-(5-(5-bromopyrimidin-2-yl)-2-fluorobenzyl)-4-hydroxycyclopentane-1-carboxylate

A mixture of Reference Example E-27 (1.01 g), sodium azide (0.468 g), ammonium chloride (0.385 g), ethanol (15 mL) and water (1.5 mL) was stirred at 110 °C under microwave irradiation for 5 hours. The reaction mixture was added to water, and extracted with ethyl acetate. The extract was dried over anhydrous sodium sulfate, and concentrated under reduced pressure. The residue was purified by column chromatography on silica gel (eluent: n-hexane/ethyl acetate=80/20) to give the title compound (1.10 g).

### Reference Example E-29

Reference Example E-29 was synthesized in a manner similar to that described in Reference Example E-7 using Reference Example E-28 instead of Reference Example E-6.

### Reference Example E-30

Reference Example E-30 was synthesized in a manner similar to that described in Reference Example E-5 using Reference Example E-29 instead of Reference Example E-4.

### Reference Example E-31

Reference Example E-31 was synthesized in a manner similar to that described in Reference Example E-19 using Reference Example E-30 instead of Reference Example E-18.

### Reference Example E-32 and Reference Example E-33

Reference Example E-32 and Reference Example E-33 were synthesized in a manner similar to that described in Reference Example E-1 and Reference Example E-2 using Reference Example D-14 instead of Reference Example D-1.

### Reference Example E-34

### Ethyl (1R*,3S*,4R*)-3-fluoro-1-(3-(5-fluoropyrimidin-2-yl)-4-hydroxybenzyl)-4-(methylsulfonamido)cyclopentane-1-carboxylate

A mixture of Reference Example E-33 (0.152 g), sodium azide (0.054 g), ammonium chloride (0.045 g), 1,4-dioxane (1.5 mL) and water (0.15 mL) was stirred at 110 °C under microwave irradiation for 2 hours. The reaction mixture was added to water, and extracted with ethyl acetate. The extract was dried over anhydrous magnesium sulfate, and concentrated under reduced pressure. The residue was purified by column chromatography on silica gel (eluent: n-hexane/ethyl acetate=80/20-70/30) to give ethyl (1R*,3R*,4R*)-3-azido-1-(4-(benzyloxy)-3-(5-fluoropyrimidin-2-yl)benzyl)-4-hydroxycyclopentane-1-carboxylate as a mixture with Reference Example E-33. A mixture of the obtained compound, sodium azide (0.054 g), ammonium chloride (0.045 g), 1,4-dioxane (1.5 mL) and water (0.15 mL) was stirred at 110 °C under microwave irradiation for 1 hour. To the reaction mixture was added water, and the mixture was extracted with ethyl acetate. The extract was dried over anhydrous magnesium sulfate, and concentrated under reduced pressure.

To a mixture of the residue, ethanol (0.9 mL) and THF (0.9 mL) was added 10% Pd/C (0.014 g) under ice-cooling. The mixture was stirred under a hydrogen atmosphere at room temperature for 2 hours. The reaction mixture was filtered through a pad of Celite, and the filtrate was concentrated under reduced pressure.

To a mixture of the residue, TEA (0.021 g) and DCM (1 mL) was added methanesulfonyl chloride (0.015 g) under ice-cooling, and the mixture was stirred at room temperature for 15 minutes. To the reaction mixture was added water, and the mixture was extracted with DCM. The extract was dried over anhydrous sodium sulfate, and concentrated under reduced pressure. The residue was purified by column chromatography on silica gel (eluent: n-hexane/ethyl acetate=70/30-60/40) to give ethyl (1R*,3R*,4R*)-1-(3-(5-fluoropyrimidin-2-yl)-4-hydroxybenzyl)-3-hydroxy-4-(methylsulfonamido)cyclopentane-1-carboxylate (0.035 g)

To a mixture of the obtained compound (0.035 g), pyridine (0.018 g) and DCM (0.8 mL) was added Deoxo-Fluor (0.051 g) under ice-cooling. The mixture was stirred at the same temperature for 20 minutes, and then the mixture was stirred at room temperature for 1 hour. The reaction mixture was added to a saturated aqueous solution of sodium bicarbonate under ice-cooling, and extracted with DCM. The extract was dried over anhydrous sodium sulfate, and concentrated under reduced pressure. The residue was purified by column chromatography on silica gel (eluent: n-hexane/ethyl acetate=80/20-60/40) to give the title compound (0.010 g).

### Reference Example E-35

### Ethyl (1R*,3S*,4R*)-1-(3-(5-fluoropyrimidin-2-yl)benzyl)-3-hydroxy-4-methylcyclopentane-1-carboxylate

Under an argon atmosphere, to a mixture of copper (I) cyanide (0.173 g) and THF (5 mL) was added methyllithium (3.1 mol/L in 1,2-diethoxyethane, 1.24 mL) at - 78 °C, and the mixture was stirred at room temperature for 5 minutes. To the reaction mixture was added a mixture of Reference Example E-9 (0.300 g) and THF (5 mL) at - 78 °C, and then boron trifluoride-diethyl ether complex (0.497 g) was added to the mixture. The mixture was stirred at the same temperature for 1 hour. The reaction mixture was added to a saturated aqueous solution of ammonium chloride at room temperature, and extracted with ethyl acetate. The extract was dried over anhydrous sodium sulfate, and concentrated under reduced pressure. The residue was purified by column chromatography on silica gel (eluent: n-hexane/ethyl acetate=100/0-55/45) to give ethyl (1R*,3R*,4R*)-1-(3-(5-fluoropyrimidin-2-yl)benzyl)-3-hydroxy-4-methylcyclopentane-1-carboxylate (0.250 g).

To a mixture of the obtained compound (0.250 g), 4-nitrobenzoic acid (0.280 g), triphenylphosphine (0.439 g) and THF (7 mL) was added diisopropyl azodicarboxylate (1.9 mol/L in toluene, 0.881 mL) at room temperature. The mixture was stirred at room temperature for 1 hour, and concentrated under reduced pressure. The residue was purified by column chromatography on silica gel (eluent: n-hexane/ethyl acetate=100/0-50/50) to give (1S*,2R*,4R*)-4-(ethoxycarbonyl)-4-(3-(5-fluoropyrimidin-2-yl)benzyl)-2-methylcyclopentyl 4-nitrobenzoate (0.353 g).

To a mixture of the obtained compound (0.353 g), an aqueous sodium hydroxide solution (2 mol/L, 0.698 mL) and ethanol (7 mL) was stirred at room temperature for 1.5 hours. To the reaction mixture were added a saturated aqueous solution of ammonium chloride and water. The mixture was extracted with DCM. The extract was dried over anhydrous sodium sulfate, and concentrated under reduced pressure. The residue was purified by column chromatography on silica gel (eluent: n-hexane/ethyl acetate=100/0-0/100) to give the title compound (0.078 g).

### Reference Example E-36

### Ethyl (1R*,3R*,4R*)-1-(3-(5-fluoropyrimidin-2-yl)benzyl)-3-methyl-4-(methylsulfonamido)cyclopentane-1-carboxylate

To a mixture of Reference Example E-35 (0.078 g), TEA (0.044 g) and DCM (1 mL) was added methanesulfonyl chloride (0.037 g) under ice-cooling, and the mixture was stirred at room temperature for 30 minutes. The reaction mixture was added to water, and extracted with DCM. The extract was dried over anhydrous sodium sulfate, and concentrated under reduced pressure.

A mixture of the residue, sodium azide (0.042 g) and DMSO (1 mL) was stirred at 100 °C for 2 hours. The reaction mixture was allowed to cool to room temperature, and then added to water. The mixture was extracted with ethyl acetate. The extract was dried over anhydrous sodium sulfate, and concentrated under reduced pressure. The residue was purified by column chromatography on silica gel (eluent: n-hexane/ethyl acetate=100/0-90/10) to give ethyl (1R*,3R*,4R*)-3-azido-1-(3-(5-fluoropyrimidin-2-yl)benzyl)-4-methylcyclopentane-1-carboxylate (0.058 g).

To a mixture of the obtained compound (0.058 g), methanol (1 mL) and water (0.005 mL) was added triphenylphosphine (0.060 g) at room temperature. The mixture was stirred at the same temperature for 13 hours, and concentrated under reduced pressure. The residue was purified by column chromatography on APS (eluent: n-hexane/ethyl acetate=100/0-10/90).

To a mixture of obtained ethyl (1R*,3R*,4R*)-3-amino-1-(3-(5-fluoropyrimidin-2-yl)benzyl)-4-methylcyclopentane-1-carboxylate, TEA (0.046 g) and DCM (1 mL) was added methanesulfonyl chloride (0.026 g) under ice-cooling, and the mixture was stirred at the same temperature for 15 minutes. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=100/0-70/30) to give the title compound (0.018 g).

### Reference Example E-37

### Ethyl (1R*,3S*,4S*)-1-(3-(5-fluoropyrimidin-2-yl)benzyl)-3-hydroxy-4-methylcyclopentane-1-carboxylate

Under an argon atmosphere, to a mixture of copper (I) cyanide (0.270 g) and THF (5 mL) was added methyllithium (3.1 mol/L in 1,2-diethoxyethane, 1.95 mL) at - 78 °C, and the mixture was stirred at room temperature for 5 minutes. To the reaction mixture was added a mixture of Reference Example E-10 (0.470 g) and THF (5 mL) at - 78 °C, and then boron trifluoride-diethyl ether complex (0.779 g) was added to the mixture. The mixture was stirred at the same temperature for 1 hour. The reaction mixture was added to a saturated aqueous solution of ammonium chloride at room temperature, and extracted with ethyl acetate. The extract was dried over anhydrous sodium sulfate, and concentrated under reduced pressure. The residue was purified by column chromatography on silica gel (eluent: n-hexane/ethyl acetate=100/0-55/45) to give the title compound (0.355 g).

### Reference Example E-38

Reference Example E-38 was synthesized in a manner similar to that described in Reference Example E-36 using Reference Example E-37 instead of Reference Example E-35.

### Reference Example F-1

### (1R*,4R*)-4-Azido-3,3-difluoro-N-methoxy-N-methyl-1-(3-(pyrimidin-2-yl)benzyl)cyclopentane-1-carboxamide

To a mixture of Reference Example E-1 (0.950 g), benzyl alcohol (0.509 g) and DCM (10 mL) was added boron trifluoride-diethyl ether complex (0.033 g) under ice-cooling. The mixture was stirred at 0 °C for 16 hours, and concentrated under reduced pressure. The residue was purified by column chromatography on silica gel (eluent: n-hexane/ethyl acetate=75/25-55/45) to give ethyl (1S*,3S*,4S*)-3-(benzyloxy)-1-(3-(5-bromopyrimidin-2-yl)benzyl)-4-hydroxycyclopentane-1-carboxylate (0.739 g).

To a mixture of the obtained compound (0.670 g), iodobenzene diacetate (0.633 g) and DCM (10 mL) was added AZADOL (0.010 g) at room temperature, and the mixture was stirred at the same temperature for 1 hour. To the reaction mixture were added 1 mol/L aqueous solution of sodium thiosulfate (2 mL), a saturated aqueous solution of sodium bicarbonate and water. The mixture was extracted with DCM. The extract was dried over anhydrous sodium sulfate, and concentrated under reduced pressure. The residue was purified by column chromatography on silica gel (eluent: n-hexane/ethyl acetate=90/10-70/30) to give ethyl (1R*,3S*)-3-(benzyloxy)-1-(3-(5-bromopyrimidin-2-yl)benzyl)-4-oxocyclopentane-1-carboxylate (0.530 g).

To a mixture of the obtained compound (0.570 g) and DCM (3 mL) was added Deoxo-Fluor (0.545 g) under ice-cooling, and the mixture was stirred at room temperature for 15 hours. To the reaction mixture were added a saturated aqueous solution of sodium bicarbonate and water under ice-cooling. The mixture was extracted with DCM. The extract was dried over anhydrous sodium sulfate, and concentrated under reduced pressure. The residue was purified by column chromatography on silica gel (eluent: n-hexane/ethyl acetate=100/0-80/20) to give ethyl (1R*,4S*)-4-(benzyloxy)-1-(3-(5-bromopyrimidin-2-yl)benzyl)-3,3-difluorocyclopentane-1-carboxylate (0.550 g).

To a mixture of the obtained compound (0.550 g), ethanol (5 mL) and THF (5 mL) was added 10% Pd/C (0.110 g) under ice-cooling. The mixture was stirred under a hydrogen atmosphere at room temperature for 13 hours. The reaction mixture was filtered through a pad of Celite, and the filtrate was concentrated under reduced pressure. The residue was purified by column chromatography on silica gel (eluent: n-hexane/ethyl acetate=80/20-60/40) to give ethyl (1R*,4S*)-4-(benzyloxy)-3,3-difluoro-1-(3-(pyrimidin-2-yl)benzyl)cyclopentane-1-carboxylate (0.259 g).

Under an argon atmosphere, to a mixture of the obtained compound (0.200 g) and DCM (8 mL) was added boron tribromide (1 mol/L in DCM, 1.77 mL) at -78 °C, and the mixture was stirred at the same temperature for 1.5 hours. To the reaction mixture were added ethanol (0.2 mL), a saturated aqueous solution of sodium bicarbonate and water at -78 °C. The mixture was extracted with DCM. The extract was dried over anhydrous sodium sulfate, and concentrated under reduced pressure. The residue was purified by column chromatography on silica gel (eluent: n-hexane/ethyl acetate=70/30-50/50) to give ethyl (1R*,4S*)-3,3-difluoro-4-hydroxy-1-(3-(pyrimidin-2-yl)benzyl)cyclopentane-1-carboxylate (0.151 g).

To a mixture of the obtained compound (0.165 g), TEA (0.092 g) and DCM (2 mL) was added methanesulfonyl chloride (0.078 g) under ice-cooling, and the mixture was stirred at room temperature for 1 hour. To the reaction mixture was added water. The mixture was extracted with DCM, and the extract was concentrated under reduced pressure.

To a mixture of the residue and DMSO (2 mL) was added sodium azide (0.118 g) at room temperature. The mixture was stirred at 100 °C for 12 hours, and then stirred at 150 °C for 3 hours. The mixture was allowed to cool to room temperature. To the reaction mixture was added water. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=70/30-50/50) to give ethyl (1R*,4R*)-4-azido-3,3-difluoro-1-(3-(pyrimidin-2-yl)benzyl)cyclopentane-1-carboxylate (0.048 g).

A mixture of the obtained compound (0.045 g), 2 mol/L aqueous solution of sodium hydroxide (0.290 mL) and ethanol (1 mL) was stirred at 80 °C for 3 hours, and allowed to cool to room temperature. To the reaction mixture were added 2 mol/L hydrochloric acid (0.319 mL), water and brine. The mixture was extracted with ethyl acetate. The extract was dried over anhydrous magnesium sulfate, and concentrated under reduced pressure.

To a mixture of the residue, DMF (1 mg) and DCM (1 mL) was added oxalyl chloride (0.030 g) under ice-cooling. The mixture was stirred at the same temperature for 30 minutes, and concentrated under reduced pressure. A mixture of the residue and DCM (1 mL) was added to a mixture of N,O-dimethylhydroxylamine hydrochloride (0.023 g), DIPEA (0.045 g) and DCM (1 mL) under ice-cooling. The mixture was stirred at room temperature for 30 minutes. To the reaction mixture were added water and brine. The mixture was extracted with DCM, and the extract was concentrated under reduced pressure. The residue was purified by column chromatography on silica gel (eluent: n-hexane/ethyl acetate=50/50-0/100) to give the title compound (0.043 g).

### Reference Example F-2

### (1R*,4R*)-4-Amino-3,3-difluoro-1-(3-(5-fluoropyrimidin-2-yl)benzyl)-N-methoxy-N-methylcyclopentane-1-carboxamide

A mixture of Reference Example E-11 (0.700 g), triphenylphosphine (0.572 g), THF (14 mL) and water (0.7 mL) was stirred at 70 °C for 6 hours, and allowed to cool to room temperature. To the reaction mixture were added a mixture of Boc₂O (0.476 g) and THF (7 mL). To the mixture was added TEA (0.221 g). The mixture was stirred at room temperature for 30 minutes, and concentrated under reduced pressure. The residue was purified by column chromatography on silica gel (eluent: n-hexane/ethyl acetate=70/30-50/50) to give ethyl (1R*,3R*,4R*)-3-((tert-butoxycarbonyl)amino)-1-(3-(5-fluoropyrimidin-2-yl)benzyl)-4-hydroxycyclopentane-1-carboxylate (0.773 g).

To a mixture of the obtained compound (0.770 g), iodobenzene diacetate (0.810 g) and DCM (15 mL) was added AZADOL (0.013 g) at room temperature, and the mixture was stirred at the same temperature for 2.5 hours. To the reaction mixture were added a saturated aqueous solution of sodium bicarbonate and water. The mixture was extracted with DCM. The extract was washed with brine, dried over anhydrous sodium sulfate, and concentrated under reduced pressure. The residue was purified by column chromatography on silica gel (eluent: n-hexane/ethyl acetate=80/20-60/40) to give ethyl (1R*,3R*)-3-((tert-butoxycarbonyl)amino)-1-(3-(5-fluoropyrimidin-2-yl)benzyl)-4-oxocyclopentane-1-carboxylate (0.704 g).

To a mixture of the obtained compound (0.600 g), and 1,2-dichloroethane (3 mL) was added Deoxo-Fluor (1.16 g) at room temperature, and the mixture was stirred at 30 °C for 24 hours. The reaction mixture was added to a mixture of a saturated aqueous solution of sodium bicarbonate (20 mL) and water (10 mL) under ice-cooling. The mixture was stirred at room temperature for 10 minutes, and extracted with DCM. The extract was washed with brine, dried over anhydrous sodium sulfate, and concentrated under reduced pressure. The residue was purified by column chromatography on silica gel (eluent: n-hexane/ethyl acetate=90/10-70/30) to give ethyl (1R*,4R*)-4-((tert-butoxycarbonyl)amino)-3,3-difluoro-1-(3-(5-fluoropyrimidin-2-yl)benzyl)cyclopentane-1-carboxylate (0.309 g).

A mixture of the obtained compound (0.300 g), 2 mol/L aqueous solution of sodium hydroxide (1.56 mL) and 2-propanol (6 mL) was stirred at 60 °C for 3 hours, and allowed to cool to room temperature. To the reaction mixture were added 2 mol/L hydrochloric acid (1.72 mL), water and brine. The mixture was extracted with DCM. The extract was dried over anhydrous magnesium sulfate, and concentrated under reduced pressure.

To a mixture of the residue, N,O-dimethylhydroxylamine hydrochloride (0.277 g), DIPEA (0.458 g) and DMF (3.2 mL) was added HATU (0.350 g) at room temperature. The mixture was stirred at 70 °C for 1.5 hours, and allowed to cool to room temperature. To the reaction mixture were added water and a saturated aqueous solution of sodium bicarbonate. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=70/30-50/50) to give tert-butyl ((1R*,4R*)-2,2-difluoro-4-(3-(5-fluoropyrimidin-2-yl)benzyl)-4-(methoxy(methyl)carbamoyl)cyclopentyl)carbamate (0.271 g).

A mixture of the obtained compound (0.270 g), hydrogen chloride (4 mol/L in 1,4-dioxane, 1.5 mL) and 1,4-dioxane (1.5 mL) was stirred at room temperature for 13 hours, and the mixture was diluted with DCM. To the mixture were added a saturated aqueous solution of sodium bicarbonate, water and brine. The mixture was extracted with DCM. The extract was dried over anhydrous sodium sulfate, and concentrated under reduced pressure to give the title compound (0.209 g).

### Reference Example F-3 and Reference Example F-4

Reference Example F-3 and Reference Example F-4 were synthesized in a manner similar to that described in Reference Example F-2 using the corresponding reference examples instead of Reference Example E-11.

### Reference Example G-1

### Methyl (1S,4R)-4-((diphenylmethylene)amino)cyclopent-2-ene-1-carboxylate

A mixture of methyl (1S,4R)-4-((tert-butoxycarbonyl)amino)cyclopent-2-ene-1-carboxylate (3.00 g), hydrogen chloride (4 mol/L in 1,4-dioxane, 31 mL) and methanol (30 mL) was stirred at room temperature for 1 hour, and concentrated under reduced pressure.

A mixture of the residue, diphenylmethanimine (2.20 g) and DCM (24 mL) was stirred at room temperature for 24 hours. The reaction mixture was filtered, and the filtrate was concentrated under reduced pressure. To the residue was added diethyl ether, and the mixture was filtered. The filtrate was concentrated under reduced pressure to give the title compound (3.74 g).

### Reference Example G-2

### Methyl (1R,3S)-3-((diphenylmethylene)amino)cyclopentane-1-carboxylate

A mixture of methyl (1R,3S)-3-((tert-butoxycarbonyl)amino)cyclopentane-1-carboxylate (0.770 g), hydrogen chloride (4 mol/L in 1,4-dioxane, 8 mL) and 1,4-dioxane (8 mL) was stirred at room temperature for 6 hours, and concentrated under reduced pressure. To the residue was added toluene, and the mixture was concentrated under reduced pressure.

A mixture of the residue, diphenylmethanimine (0.545 g) and DCM (16 mL) was stirred at room temperature for 16 hours. The reaction mixture was filtered, and the filtrate was concentrated under reduced pressure. The residue was purified by column chromatography on APS (eluent: n-hexane/ethyl acetate=90/10-70/30) to give the title compound (0.921 g).

### Reference Example G-3

### Methyl (4R)-4-((diphenylmethylene)amino)-3,3-difluorocyclopentane-1-carboxylate

To a mixture of methyl (3R,4R)-4-((tert-butoxycarbonyl)amino)-3-hydroxycyclopent-1-ene-1-carboxylate (1.36 g) and ethanol (20 mL) was added 10% Pd/C (0.408 g) at room temperature. The mixture was stirred under a hydrogen atmosphere at room temperature for 2 hours. The reaction mixture was filtered through a pad of Celite, and the filtrate was concentrated under reduced pressure.

To a mixture of the residue, iodobenzene diacetate (2.55 g) and DCM (21 mL) was added AZADOL (0.081 g) at room temperature, and the mixture was stirred at the same temperature for 2 hours. To the reaction mixture was added a saturated aqueous solution of sodium bicarbonate. The mixture was extracted with DCM. The extract was dried over anhydrous sodium sulfate, and concentrated under reduced pressure. The residue was purified by column chromatography on silica gel (eluent: n-hexane/ethyl acetate=100/0-60/40) to give methyl (3R)-3-((tert-butoxycarbonyl)amino)-4-oxocyclopentane-1-carboxylate (1.06 g).

To a mixture of the obtained compound (1.06 g) and 1,2-dichloroethane (15 mL) was added Deoxo-Fluor (3.65 g) at room temperature. The mixture was stirred at 60 °C for 13 hours, and allowed to cool to room temperature. The reaction mixture was diluted with DCM. The mixture was added to a saturated aqueous solution of sodium bicarbonate under ice-cooling, and extracted with DCM. The extract was washed with brine, dried over anhydrous sodium sulfate, and concentrated under reduced pressure. The residue was purified by column chromatography on silica gel (eluent: n-hexane/ethyl acetate=100/0-60/40) to give methyl (4R)-4-((tert-butoxycarbonyl)amino)-3,3-difluorocyclopentane-1-carboxylate (0.499 g).

A mixture of the obtained compound (0.499 g), hydrogen chloride (4 mol/L in 1,4-dioxane, 8.93 mL) and methanol (7.5 mL) was stirred at room temperature for 1 hour, and concentrated under reduced pressure. The residue was purified by column chromatography on APS (eluent: n-hexane/ethyl acetate=100/0-0/100) to give methyl (4R)-4-amino-3,3-difluorocyclopentane-1-carboxylate (0.237 g).

A mixture of the obtained compound (0.224 g), diphenylmethanimine (0.227 g) and DCM (3 mL) was stirred at room temperature for 40 hours. The reaction mixture was filtered, and the filtrate was concentrated under reduced pressure. To the residue was added DCM, and the mixture was filtered. The filtrate was concentrated under reduced pressure to give the title compound (0.337 g).

### Reference Example H-1

### Methyl (1R,4R)-4-((diphenylmethylene)amino)-1-(4-fluoro-3-(5-fluoropyrimidin-2-yl)benzyl)cyclopent-2-ene-1-carboxylate

Under an argon atmosphere, to a mixture of Reference Example G-1 (2.26 g), Reference Example D-9 (2.22 g) and THF (23 mL) was added lithium bis(trimethylsilyl)amide (1.11 mol/L in THF, 8 mL) in ice-salt bath. The mixture was stirred at the same temperature for 30 minutes. To the mixture was added lithium bis(trimethylsilyl)amide (1.11 mol/L in THF, 2 mL) in ice-salt bath. The mixture was stirred at the same temperature for 15 minutes. To the reaction mixture were added a saturated aqueous solution of ammonium chloride, brine and water. The mixture was extracted with ethyl acetate. The extract was dried over anhydrous magnesium sulfate, and concentrated under reduced pressure. The residue was purified by column chromatography on silica gel (eluent: n-hexane/ethyl acetate=100/0-80/20) to give the title compound (2.13 g).

### Reference Example H-2

### Methyl (1R,4R)-4-((tert-butoxycarbonyl)amino)-1-(4-fluoro-3-(5-fluoropyrimidin-2-yl)benzyl)cyclopent-2-ene-1-carboxylate

A mixture of Reference Example H-1 (2.10 g), 6 mol/L hydrochloric acid (2.06 mL) and methanol (10 mL) was stirred at room temperature for 18 hours. To the reaction mixture was added 5 mol/L aqueous solution of sodium hydroxide (3.3 mL) under ice-cooling, and then a mixture of Boc₂O (1.17 g) and THF (10 mL) was added to the mixture. The mixture was stirred at room temperature for 1 hour. To the reaction mixture was added 2 mol/L aqueous solution of sodium hydroxide (2.06 mL) at room temperature, and then a mixture of Boc₂O (0.450 g) and THF (2 mL) was added to the mixture. The mixture was stirred at the same temperature for 30 minutes. To the reaction mixture were added water and brine. The mixture was extracted with ethyl acetate. The extract was dried over anhydrous magnesium sulfate, and concentrated under reduced pressure. The residue was purified by column chromatography on silica gel (eluent: n-hexane/ethyl acetate=80/20-60/40) to give the title compound (1.72 g).

### Reference Example H-3

### Methyl (1R,3S)-3-((tert-butoxycarbonyl)amino)-1-(4-fluoro-3-(5-fluoropyrimidin-2-yl)benzyl)cyclopentane-1-carboxylate

To a mixture of Reference Example H-2 (1.71 g), ethanol (8.5 mL) and THF (8.5 mL) was added platinum on carbon (5%, 0.342 g) under ice-cooling. The mixture was stirred under a hydrogen atmosphere at room temperature for 3 hours. The reaction mixture was filtered through a pad of Celite, and the filtrate was concentrated under reduced pressure. The residue was purified by column chromatography on silica gel (eluent: n-hexane/ethyl acetate=80/20-60/40) to give the title compound (1.63 g).

### Reference Example H-4

### (1R,3S)-1-(4-Fluoro-3-(5-fluoropyrimidin-2-yl)benzyl)-3-(methylsulfonamido)cyclopentane-1-carboxylic acid

A mixture of Reference Example H-3 (0.057 g), hydrogen chloride (4 mol/L in 1,4-dioxane, 0.5 mL) and 1,4-dioxane (0.5 mL) was stirred at 40 °C for 1.5 hours, and allowed to cool to room temperature. The reaction mixture was concentrated under reduced pressure. To a mixture of the residue, TEA (0.039 g) and DCM (1 mL) was added methanesulfonyl chloride (0.022 g) under ice-cooling, and the mixture was stirred at room temperature for 15 minutes. To the reaction mixture was added water. The mixture was extracted with DCM, and the extract was concentrated under reduced pressure.

A mixture of the residue, 6 mol/L hydrochloric acid (0.5 mL) and 1,4-dioxane (0.5 mL) was stirred at 80 °C for 2 hours, and then stirred at room temperature for 15 hours. The reaction mixture was stirred at 100 °C for 4 hours, and allowed to cool to room temperature. To the reaction mixture was added water. The mixture was extracted with DCM, and the extract was concentrated under reduced pressure to give the title compound (0.054 g).

### Reference Example H-5

### (1R,3S)-3-Amino-1-(4-fluoro-3-(5-fluoropyrimidin-2-yl)benzyl)-N-methoxy-N-methylcyclopentane-1-carboxamide

A mixture of Reference Example H-3 (0.210 g), 5 mol/L aqueous solution of sodium hydroxide (0.939 mL) and 1,4-dioxane (4.2 mL) was stirred at 100 °C for 6 hours, and allowed to cool to room temperature. Under water-cooling, to the reaction mixture were added 6 mol/L hydrochloric acid (0.939 mL), water and brine. The mixture was extracted with ethyl acetate. The extract was dried over anhydrous magnesium sulfate, and concentrated under reduced pressure.

To a mixture of the residue, N,O-dimethylhydroxylamine hydrochloride (0.062 g), DIPEA (0.165 g) and DMF (2 mL) was added (1-cyano-2-ethoxy-2-oxoethylidenaminooxy)dimethylamino-morpholino-carbenium hexafluorophosphate (0.274 g) at room temperature, and the mixture was stirred at the same temperature for 18 hours. To the reaction mixture were added a saturated aqueous solution of sodium bicarbonate and water. The mixture was extracted with ethyl acetate. The extract was washed with water and brine, dried over anhydrous magnesium sulfate, and concentrated under reduced pressure. The residue was purified by column chromatography on silica gel (eluent: n-hexane/ethyl acetate=60/40-40/60) to give tert-butyl ((1S,3R)-3-(4-fluoro-3-(5-fluoropyrimidin-2-yl)benzyl)-3-(methoxy(methyl)carbamoyl)cyclopentyl)carbamate (0.136 g).

A mixture of the obtained compound (0.135 g), hydrogen chloride (4 mol/L in 1,4-dioxane, 1 mL) and 1,4-dioxane (1 mL) was stirred at room temperature for 18 hours, and concentrated under reduced pressure. To the residue was added toluene, and the mixture was concentrated under reduced pressure. To the residue was added a saturated aqueous solution of sodium bicarbonate, and the mixture was extracted with DCM. The extract was dried over anhydrous sodium sulfate, and concentrated under reduced pressure to give the title compound (0.083 g).

### Reference Example H-6

### Methyl (1R,3S)-1-(4-chloro-3-(5-fluoropyrimidin-2-yl)benzyl)-3-(methylsulfonamido)cyclopentane-1-carboxylate

Under an argon atmosphere, to a mixture of Reference Example G-2 (0.060 g), Reference Example D-13 (0.060 g) and THF (1 mL) was added lithium bis(trimethylsilyl)amide (1.1 mol/L in THF, 0.248 mL) in ice-salt bath. The mixture was stirred at the same temperature for 1 hour. To the mixture was added lithium bis(trimethylsilyl)amide (1.1 mol/L in THF, 0.177 mL) in ice-salt bath. The mixture was stirred at the same temperature for 30 minutes. To the reaction mixture were added a saturated aqueous solution of ammonium chloride, brine and water. The mixture was extracted with ethyl acetate. The extract was dried over anhydrous magnesium sulfate, and concentrated under reduced pressure. The residue was purified by column chromatography on APS (eluent: n-hexane/ethyl acetate=100/0-50/50) to give methyl (1R,3S)-1-(4-chloro-3-(5-fluoropyrimidin-2-yl)benzyl)-3-((diphenylmethylene)amino)cyclopentane-1-carboxylate (0.037 g).

A mixture of the obtained compound (0.035 g), 2 mol/L hydrochloric acid (0.331 mL) and THF (1 mL) was stirred at room temperature for 1 hour. To the reaction mixture were added a saturated aqueous solution of sodium bicarbonate and water. The mixture was extracted with DCM, and the extract was concentrated under reduced pressure.

To a mixture of the residue, TEA (0.020 g) and DCM (1 mL) was added methanesulfonyl chloride (0.011 g) under ice-cooling, and the mixture was stirred at room temperature for 10 minutes. To the reaction mixture was added water. The mixture was extracted with DCM, and the extract was concentrated under reduced pressure. The residue was purified by column chromatography on silica gel (eluent: n-hexane/ethyl acetate=50/50-0/100) to give the title compound (0.019 g).

### Reference Example H-7

### Methyl (1R,4R)-4-((diphenylmethylene)amino)-1-(3-(5-fluoropyrimidin-2-yl)-4-methoxybenzyl)cyclopent-2-ene-1-carboxylate

Under an argon atmosphere, to a mixture of Reference Example G-1 (0.493 g), Reference Example D-11 (0.505 g) and THF (7.3 mL) was added lithium bis(trimethylsilyl)amide (1.11 mol/L in THF, 0.184 mL) in ice-salt bath. The mixture was stirred at the same temperature for 1 hour. To the reaction mixture were added a saturated aqueous solution of ammonium chloride and water. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=100/0-40/60) to give the title compound (0.62 g).

### Reference Example H-8

### Methyl (1R,4R)-1-(3-(5-fluoropyrimidin-2-yl)-4-methoxybenzyl)-4-(methylsulfonamido)cyclopent-2-ene-1-carboxylate

To a mixture of Reference Example H-7 (0.620 g) and THF (6 mL) was added 2 mol/L hydrochloric acid (1.78 mL) at room temperature. The mixture was stirred at the same temperature for 1 hour. To the reaction mixture was added 2 mol/L aqueous solution of sodium hydroxide (1.78 mL). The mixture was extracted with DCM. The extract was dried over anhydrous magnesium sulfate, and concentrated under reduced pressure.

To a mixture of the residue and DCM (6 mL) were added TEA (0.241 g) and methanesulfonyl chloride (0.204 g) under ice-cooling, and the mixture was stirred at room temperature for 30 minutes. To the reaction mixture was added water. The mixture was extracted with DCM, and the extract was concentrated under reduced pressure. The residue was purified by column chromatography on silica gel (eluent: n-hexane/ethyl acetate/methanol=50/50/0-0/100/0-0/88/12) to give the title compound (0.476 g).

### Reference Example H-9

Reference Example H-9 was synthesized in a manner similar to that described in Reference Example H-3 using Reference Example H-8 instead of Reference Example H-2.

### Reference Example H-10

### (1R,3S)-1-(3-(5-Fluoropyrimidin-2-yl)-4-methoxybenzyl)-3-(methylsulfonamido)cyclopentane-1-carboxylic acid

To a mixture of the Reference Example H-9 (0.466 g) and 1,4-dioxane (5 mL) was added 5 mol/L aqueous solution of sodium hydroxide (2.13 mL) at room temperature. The mixture was stirred at 100 °C for 30 minutes, and allowed to cool to room temperature. Under ice-cooling, to the reaction mixture was added concentrated hydrochloric acid (0.9 mL). The mixture was extracted with DCM, and the extract was concentrated under reduced pressure to give the title compound (0.125 g).

### Reference Example I-1

### Methyl (3aR,5R,6aS)-5-(3-(5-fluoropyrimidin-2-yl)benzyl)-2-oxohexahydro-2H-cyclopenta[d]oxazole-5-carboxylate

Under an argon atmosphere, to a mixture of Reference Example G-1 (2.80 g), Reference Example D-2 (2.57 g) and THF (42 mL) was added lithium bis(trimethylsilyl)amide (1.1 mol/L in THF, 16.5 mL) at -78 °C. The mixture was stirred at the same temperature for 1.5 hours, and then stirred at room temperature for 30 minutes. To the reaction mixture were added a saturated aqueous solution of ammonium chloride and water. The mixture was extracted with ethyl acetate. The extract was dried over anhydrous sodium sulfate, and concentrated under reduced pressure. The residue was purified by column chromatography on silica gel (eluent: n-hexane/ethyl acetate=100/0-85/15) to give methyl (1R,4R)-4-((diphenylmethylene)amino)-1-(3-(5-fluoropyrimidin-2-yl)benzyl)cyclopent-2-ene-1-carboxylate (2.35 g).

A mixture of the obtained compound (2.35 g), 2 mol/L hydrochloric acid (7.17 mL) and THF (28 mL) was stirred at room temperature for 30 minutes. To the reaction mixture was added 2 mol/L aqueous solution of sodium hydroxide (9.56 mL) at room temperature, and then a mixture of Boc₂O (1.25 g) and THF (28 mL) was added to the mixture. The mixture was stirred at room temperature for 20 minutes. To the reaction mixture was added water. The mixture was extracted with ethyl acetate. The extract was washed with brine, dried over anhydrous sodium sulfate, and concentrated under reduced pressure. The residue was purified by column chromatography on silica gel (eluent: n-hexane/ethyl acetate=90/10-50/50) to give methyl (1R,4R)-4-((tert-butoxycarbonyl)amino)-1-(3-(5-fluoropyrimidin-2-yl)benzyl)cyclopent-2-ene-1-carboxylate (2.04 g).

To a mixture of the obtained compound (2.04 g), THF (24 mL) and water (2.4 mL) was added N-bromosuccinimide (1.11 g) at room temperature, and the mixture was stirred at the same temperature for 30 minutes. To the reaction mixture were added 1 mol/L aqueous solution of sodium thiosulfate and a saturated aqueous solution of sodium bicarbonate at room temperature. The mixture was extracted with ethyl acetate. The extract was washed with a saturated aqueous solution of sodium bicarbonate and brine, dried over anhydrous sodium sulfate, and concentrated under reduced pressure. The residue was purified by column chromatography on silica gel (eluent: n-hexane/ethyl acetate=70/30-30/70) to give methyl (3aR,5S,6R,6aR)-6-bromo-5-(3-(5-fluoropyrimidin-2-yl)benzyl)-2-oxohexahydro-2H-cyclopenta[d]oxazole-5-carboxylate (1.59 g).

Under an argon atmosphere, a mixture of the obtained compound (1.59 g), tris(trimethylsilyl)silane (1.32 g), 2,2'-azobis(isobutyronitrile) (0.058 g) and toluene (20 mL) was stirred at 90 °C for 2 hours. The reaction mixture was allowed to cool to room temperature, and concentrated under reduced pressure. The residue was purified by column chromatography on silica gel (eluent: n-hexane/ethyl acetate=30/70-0/100) to give the title compound (1.16 g).

### Reference Example I-2

### Methyl (3aR,5R,6aS)-5-(3-(5-fluoropyrimidin-2-yl)benzyl)-3-(methylsulfonyl)-2-oxohexahydro-2H-cyclopenta[d]oxazole-5-carboxylate

To a mixture of Reference Example I-1 (0.392 g) and THF (20 mL) was added lithium bis(trimethylsilyl)amide (1.1 mol/L in THF, 2.0 mL) at -78 °C, and the mixture was stirred at the same temperature for 30 minutes. To the reaction mixture was added methanesulfonyl chloride (0.206 g) at -78 °C, and the mixture was stirred at the same temperature for 30 minutes. To the reaction mixture was added methanesulfonyl chloride (0.132 g) at -78 °C, and the mixture was stirred at room temperature for 20 minutes. To the reaction mixture were added water and a saturated aqueous solution of ammonium chloride. The mixture was extracted with ethyl acetate. The extract was dried over anhydrous sodium sulfate, and concentrated under reduced pressure. The residue was purified by column chromatography on silica gel (eluent: n-hexane/ethyl acetate=60/40) to give the title compound (0.364 g).

### Reference Example I-3

### Methyl (1R,3S,4R)-1-(3-(5-fluoropyrimidin-2-yl)benzyl)-3-hydroxy-4-(methylsulfonamido)cyclopentane-1-carboxylate

A mixture of Reference Example I-2 (0.364 g), cesium carbonate (0.792 g) and methanol (9 mL) was stirred at room temperature for 1 hour, and concentrated under reduced pressure. To the residue were added DCM and water. The mixture was extracted with DCM. The extract was dried over anhydrous sodium sulfate, and concentrated under reduced pressure. The residue was purified by column chromatography on silica gel (eluent: n-hexane/ethyl acetate=30/70-20/80) to give the title compound (0.244 g).

### Reference Example I-4

### Methyl (1R,3R,4R)-1-(3-(5-fluoropyrimidin-2-yl)benzyl)-3-hydroxy-4-(methylsulfonamido)cyclopentane-1-carboxylate

Under an argon atmosphere, to a mixture of Reference Example I-3 (0.244 g), 4-nitrobenzoic acid (0.231 g), triphenylphosphine (0.363 g) and THF (5 mL) was added diisopropyl azodicarboxylate (1.9 mol/L in toluene, 0.728 mL) under ice-cooling. The mixture was stirred at room temperature for 1 hour, and concentrated under reduced pressure. The residue was purified by column chromatography on silica gel (eluent: n-hexane/ethyl acetate=60/40-50/50) to give (1R,2R,4R)-4-(3-(5-fluoropyrimidin-2-yl)benzyl)-4-(methoxycarbonyl)-2-(methylsulfonamido)cyclopentyl 4-nitrobenzoate (0.299 g).

A mixture of the obtained compound (0.287 g), 2 mol/L aqueous solution of sodium hydroxide (0.501 mL), methanol (5 mL) and THF (6 mL) was stirred at room temperature for 1 hour, and concentrated under reduced pressure. To the residue were added DCM and water. The mixture was extracted with DCM. The extract was dried over anhydrous sodium sulfate, and concentrated under reduced pressure. The residue was purified by column chromatography on silica gel (eluent: n-hexane/ethyl acetate=60/40-0/100), and then purified by column chromatography on silica gel (eluent: n-hexane/ethyl acetate=60/40-20/80) to give the title compound (0.140 g).

### Reference Example I-5

### (1R,2R,4R)-2- Amino-4-(3-(5-fluoropyrimidin-2-yl)benzyl)-4-(methoxycarbonyl)cyclopentyl 4-nitrobenzoate hydrochloride

To a mixture of Reference Example I-1 (1.16 g), DMAP (0.038 g), TEA (0.915 g) and THF (14 mL) was added Boc₂O (0.818 g) at room temperature. The mixture was stirred at the same temperature for 30 minutes, and concentrated under reduced pressure. The residue was purified by column chromatography on silica gel (eluent: n-hexane/ethyl acetate=80/20-50/50) to give 5-methyl 3-(tert-butyl) (3aR,5R,6aS)-5-(3-(5-fluoropyrimidin-2-yl)benzyl)-2-oxotetrahydro-2H-cyclopenta[d]oxazole-3,5(3aH)-dicarboxylate (1.38 g).

A mixture of the obtained compound (1.38 g), cesium carbonate (3.05 g) and methanol (28 mL) was stirred at room temperature for 1 hour. To the reaction mixture were added 2 mol/L hydrochloric acid (4.69 mL) and water. The mixture was extracted with DCM. The extract was dried over anhydrous sodium sulfate, and concentrated under reduced pressure. The residue was purified by column chromatography on silica gel (eluent: n-hexane/ethyl acetate=70/30-0/100) to give methyl (1R,3R,4S)-3-((tert-butoxycarbonyl)amino)-1-(3-(5-fluoropyrimidin-2-yl)benzyl)-4-hydroxycyclopentane-1-carboxylate (0.910 g).

To a mixture of the obtained compound (0.910 g), 4-nitrobenzoic acid (0.819 g), triphenylphosphine (1.29 g) and THF (10 mL) was added diisopropyl azodicarboxylate (1.9 mol/L in toluene, 2.58 mL) at room temperature. The mixture was stirred at the same temperature for 1 hour, and concentrated under reduced pressure. The residue was purified by column chromatography on silica gel (eluent: n-hexane/ethyl acetate=80/20-50/50) to give (1R,2R,4R)-2-((tert-butoxycarbonyl)amino)-4-(3-(5-fluoropyrimidin-2-yl)benzyl)-4-(methoxycarbonyl)cyclopentyl 4-nitrobenzoate (0.770 g).

A mixture of the obtained compound (0.353 g) and hydrogen chloride (4 mol/L in 1,4-dioxane, 3 mL) was stirred at room temperature for 30 minutes. The reaction mixture was concentrated under reduced pressure to give the title compound (0.315 g).

### Reference Example I-6

### Methyl (1R,3R,4R)-1-(3-(5-fluoropyrimidin-2-yl)benzyl)-3-hydroxy-4-((1-methylethyl)sulfonamido)cyclopentane-1-carboxylate

To a mixture of Reference Example I-5 (0.100 g), 1,8-diazabicyclo[5.4.0]-7-undecene (0.051 g) and DCM (2 mL) was added isopropylsulfonyl chloride (0.024 g) at room temperature, the mixture was stirred at the same temperature for 1 hour. To the reaction mixture were added 1,8-diazabicyclo[5.4.0]-7-undecene (0.152 g) and isopropylsulfonyl chloride (0.071 g) at room temperature, and the mixture was stirred at the same temperature for 2 hours. To the reaction mixture was added water. The mixture was extracted with DCM, and the extract was concentrated under reduced pressure.

To a mixture of the residue and methanol (2 mL) was added 2 mol/L aqueous solution of sodium hydroxide (0.111 mL) at room temperature, and the mixture was stirred at the same temperature for 30 minutes. To the reaction mixture were added a saturated aqueous solution of ammonium chloride and water. The mixture was extracted with DCM. The extract was dried over anhydrous sodium sulfate, and concentrated under reduced pressure. The residue was purified by column chromatography on silica gel (eluent: n-hexane/ethyl acetate=50/50-0/100) to give the title compound (0.032 g).

### Reference Example I-7

### Methyl (1R,3R,4R)-3-(ethylsulfonamido)-1-(3-(5-fluoropyrimidin-2-yl)benzyl)-4-hydroxycyclopentane-1-carboxylate

To a mixture of Reference Example I-5 (0.500 g), TEA (0.168 g) and DCM (7 mL) was added ethanesulfonyl chloride (0.107 g) under ice-cooling, and the mixture was stirred at the same temperature for 30 minutes. To the reaction mixture was added water. The mixture was extracted with DCM, and the extract was concentrated under reduced pressure.

To a mixture of the residue and methanol (7 mL) was added 2 mol/L aqueous solution of sodium hydroxide (0.553 mL) under ice-cooling, and the mixture was stirred at the same temperature for 20 minutes. To the reaction mixture were added a saturated aqueous solution of ammonium chloride and water. The mixture was extracted with DCM. The extract was dried over anhydrous sodium sulfate, and concentrated under reduced pressure. The residue was purified by column chromatography on silica gel (eluent: n-hexane/ethyl acetate=50/50-30/70) to give the title compound (0.191 g).

### Reference Example I-8

Reference Example I-8 was synthesized in a manner similar to that described in Reference Example I-6 using cyclopropanesulfonyl chloride instead of isopropylsulfonyl chloride.

### Reference Example I-9

Reference Example I-9 was synthesized in a manner similar to that described in Reference Example I-1 using Reference Example D-12 instead of Reference Example D-2.

### Reference Example I-10

Reference Example I-10 was synthesized in a manner similar to that described in Reference Example I-2 using Reference Example I-9 and ethanesulfonyl chloride instead of Reference Example I-1 and methanesulfonyl chloride, respectively.

### Reference Example I-11

Reference Example I-11 was synthesized in a manner similar to that described in Reference Example I-3 using Reference Example I-10 instead of Reference Example I-2.

### Reference Example I-12

Reference Example I-12 was synthesized in a manner similar to that described in Reference Example I-4 using Reference Example I-11 instead of Reference Example I-3.

### Reference Example I-13

Reference Example I-13 was synthesized in a manner similar to that described in Reference Example I-1 using Reference Example D-13 instead of Reference Example D-2.

### Reference Example I-14

Reference Example I-14 was synthesized in a manner similar to that described in Reference Example I-2 using Reference Example I-13 instead of Reference Example I-1.

### Reference Example I-15

Reference Example I-15 was synthesized in a manner similar to that described in Reference Example I-2 using Reference Example I-13 and ethanesulfonyl chloride instead of Reference Example I-1 and methanesulfonyl chloride, respectively.

### Reference Example I-16 and Reference Example I-17

Reference Example I-16 and Reference Example I-17 were synthesized in a manner similar to that described in Reference Example I-3 using the corresponding reference examples instead of Reference Example I-2.

### Reference Example I-18 and Reference Example I-19

Reference Example I-18 and Reference Example I-19 were synthesized in a manner similar to that described in Reference Example I-4 using the corresponding reference examples instead of Reference Example I-3.

### Reference Example I-20

### Methyl (3aR,5R,6aS)-5-(4-fluoro-3-(5-fluoropyrimidin-2-yl)benzyl)-2-oxohexahydro-2H-cyclopenta[d]oxazole-5-carboxylate

To a mixture of Reference Example H-2 (0.550 g), THF (5 mL) and water (0.5 mL) was added N-bromosuccinimide (0.286 g) at room temperature, and the mixture was stirred at the same temperature for 30 minutes. To the reaction mixture were added 1 mol/L aqueous solution of sodium thiosulfate and a saturated aqueous solution of sodium bicarbonate at room temperature. The mixture was extracted with ethyl acetate. The extract was washed with a saturated aqueous solution of sodium bicarbonate and brine, dried over anhydrous sodium sulfate, and concentrated under reduced pressure. The residue was purified by column chromatography on silica gel (eluent: n-hexane/ethyl acetate=100/0-30/70) to give methyl (3aR,5S,6R,6aR)-6-bromo-5-(4-fluoro-3-(5-fluoropyrimidin-2-yl)benzyl)-2-oxohexahydro-2H-cyclopenta[d]oxazole-5-carboxylate (0.421 g).

Under an argon atmosphere, a mixture of the obtained compound (0.421 g), tris(trimethylsilyl)silane (0.335 g), 2,2'-azobis(isobutyronitrile) (0.015 g) and toluene (9 mL) was stirred at 90 °C for 1 hour. The reaction mixture was allowed to cool to room temperature, and concentrated under reduced pressure. The residue was purified by column chromatography on silica gel (eluent: n-hexane/ethyl acetate=100/0-0/100) to give the title compound (0.117 g).

### Reference Example I-21

Reference Example I-21 was synthesized in a manner similar to that described in Reference Example I-2 using Reference Example I-20 instead of Reference Example I-1.

### Reference Example I-22

Reference Example I-22 was synthesized in a manner similar to that described in Reference Example I-3 using Reference Example I-21 instead of Reference Example I-2.

### Reference Example I-23

### Methyl (1R,3S,4R)-1-(3-(5-fluoropyrimidin-2-yl)benzyl)-3-methoxy-4-(N-(4-methoxybenzyl)methylsulfonamido)cyclopentane-1-carboxylate

Under an argon atmosphere, to a mixture of Reference Example I-3 (0.110 g), imidazole (0.032 g) and DCM (1 mL) was added tert-butyldimethylsilyl triflate (0.094 g) under ice-cooling, and the mixture was stirred at room temperature for 2 hours. The reaction mixture was added to water, and extracted with DCM. The extract was dried over anhydrous sodium sulfate, and concentrated under reduced pressure. The residue was purified by column chromatography on silica gel (eluent: n-hexane/ethyl acetate=100/0-60/40) to give methyl (1R,3S,4R)-3-((tert-butyldimethylsilyl)oxy)-1-(3-(5-fluoropyrimidin-2-yl)benzyl)-4-(methylsulfonamido)cyclopentane-1-carboxylate (0.087 g).

To a mixture of the obtained compound (0.087 g), potassium carbonate (0.067 g), tetrabutylammonium iodide (0.012 g) and DMF (1 mL) was added 4-methoxybenzyl chloride (0.038 g) at room temperature. The mixture was stirred at 80 °C for 1 hour, and allowed to cool to room temperature. To the reaction mixture was added water, and the mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=100/0-80/20) to give methyl (1R,3S,4R)-3-((tert-butyldimethylsilyl)oxy)-1-(3-(5-fluoropyrimidin-2-yl)benzyl)-4-(N-(4-methoxybenzyl)methylsulfonamido)cyclopentane-1-carboxylate (0.066 g).

A mixture of the obtained compound (0.066 g), tetrabutylammonium fluoride (1 mol/L in THF, 0.12 mL), and THF (1 mL) was stirred at room temperature for 5 hours, and concentrated under reduced pressure. The residue was purified by column chromatography on silica gel (eluent: n-hexane/ethyl acetate=100/0-60/40) to give methyl (1R,3S,4R)-1-(3-(5-fluoropyrimidin-2-yl)benzyl)-3-hydroxy-4-(N-(4-methoxybenzyl)methylsulfonamido)cyclopentane-1-carboxylate (0.041 g).

To a mixture of the obtained compound (0.041 g), methyl iodide (0.054 g) and THF (0.5 mL) was added sodium hydride (60% in mineral oil, 0.009 g) under ice-cooling, and the mixture was stirred at room temperature for 3 hours. The reaction mixture was added to water, and extracted with DCM. The extract was dried over anhydrous sodium sulfate, and concentrated under reduced pressure. The residue was purified by column chromatography on silica gel (eluent: n-hexane/ethyl acetate=100/0-67/33) to give the title compound (0.021 g).

### Reference Example I-24

Reference Example I-24 was synthesized in a manner similar to that described in Reference Example I-23 using Reference Example I-4 instead of Reference Example I-3.

### Reference Example J-1

### Methyl (1R,4R)-1-(3-(5-bromopyrimidin-2-yl)benzyl)-3,3-difluoro-4-(methylsulfonamido)cyclopentane-1-carboxylate

Under an argon atmosphere, to a mixture of Reference Example G-3 (0.337 g) and THF (5 mL) was added LDA (1.09 mol/L in THF/n-hexane, 1.35 mL) at -78 °C, and the mixture was stirred at the same temperature for 30 minutes. To the reaction mixture was added Reference Example D-1 (0.338 g) at -78 °C. The mixture was stirred at the same temperature for 10 minutes, and then stirred at room temperature for 40 minutes. To the reaction mixture was added a saturated aqueous solution of ammonium chloride. The mixture was extracted with DCM. The extract was dried over anhydrous sodium sulfate, and concentrated under reduced pressure.

A mixture of the residue, hydrogen chloride (4 mol/L in 1,4-dioxane, 2.45 mL) and methanol (5 mL) was stirred at room temperature for 1 hour, and concentrated under reduced pressure. The residue was purified by column chromatography on APS (eluent: n-hexane/ethyl acetate=100/0-90/10) to give methyl (1R,4R)-4-amino-1-(3-(5-bromopyrimidin-2-yl)benzyl)-3,3-difluorocyclopentane-1-carboxylate (0.154 g) as a mixture with (1S,4R)-isomer.

To a mixture of the obtained compound (0.154 g), TEA (0.073 g) and DCM (2.3 mL) was added methanesulfonyl chloride (0.062 g) under ice-cooling, and the mixture was stirred at room temperature for 1 hour. The reaction mixture was added to water, and extracted with DCM. The extract was dried over anhydrous sodium sulfate, and concentrated under reduced pressure. The residue was purified by column chromatography on silica gel (eluent: n-hexane/ethyl acetate=100/0-40/60) to give the title compound (0.153 g) as a mixture with (1S,4R)-isomer.

### Reference Example J-2

### Methyl (1R,4R)-1-(3-chlorobenzyl)-3,3-difluoro-4-(methylsulfonamido)cyclopentane-1-carboxylate

Under an argon atmosphere, to a mixture of Reference Example G-3 (0.206 g) and THF (6 mL) was added LDA (1.09 mol/L in THF/n-hexane, 0.826 mL) at -40 °C, and the mixture was stirred at the same temperature for 30 minutes. To the reaction mixture was added 1-(bromomethyl)-3-chlorobenzene (0.129 g) at -40 °C. The mixture was stirred at the same temperature for 10 minutes, and then stirred at room temperature for 30 minutes. The reaction mixture was added to a saturated aqueous solution of ammonium chloride, and extracted with DCM. The extract was dried over anhydrous sodium sulfate, and concentrated under reduced pressure. The residue was purified by column chromatography on APS (eluent: n-hexane/ethyl acetate=100/0-90/10) to give methyl (1R,4R)-1-(3-chlorobenzyl)-4-((diphenylmethylene)amino)-3,3-difluorocyclopentane-1-carboxylate (0.185 g) as a mixture with (1S,4R)-isomer.

A mixture of the obtained compound (0.185 g), hydrogen chloride (4 mol/L in 1,4-dioxane, 4.94 mL) and DCM (1 mL) was stirred at room temperature for 3 hours, and concentrated under reduced pressure.

To a mixture of the residue, TEA (0.160 g) and DCM (1 mL) was added methanesulfonyl chloride (0.136 g) at room temperature. The mixture was stirred at the same temperature for 1 hour, and concentrated under reduced pressure. The residue was purified by column chromatography on silica gel (eluent: n-hexane/ethyl acetate=100/0-50/50) to give the title compound (0.101 g) as a mixture with (1S,4R)-isomer.

### Reference Example J-3

### Methyl (1R,4R)-3,3-difluoro-4-(methylsulfonamido)-1-(3-(4,4,5,5-tetramethyl-1,3,2-dioxaborolan-2-yl)benzyl)cyclopentane-1-carboxylate

A mixture of Reference Example J-2 (0.018 g), bis(pinacolato)diboron (0.036 g), potassium acetate (0.014 g), Pd₂(dba)₃ (2.2 mg), Xphos (4.5 mg) and 1,4-dioxane (0.5 mL) was stirred at 110 °C under microwave irradiation for 1 hour. The reaction mixture was filtered through a pad of Celite, and the filtrate was concentrated under reduced pressure. The residue was purified by column chromatography on silica gel (eluent: n-hexane/ethyl acetate=100/0-0/100) to give the title compound (0.025 g) as a mixture with (1S,4R)-isomer.

Structures of Reference Examples are shown in the following tables.

**[Table 1]**

| Ref. No. | Str. | Ref. No. | Str. |
|---|---|---|---|
| A-1 | 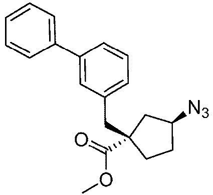 | A-2 | 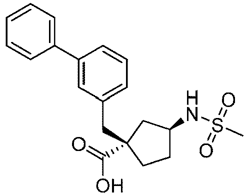 |
| A-3 | 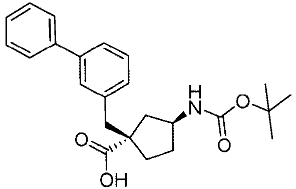 | A-4 | 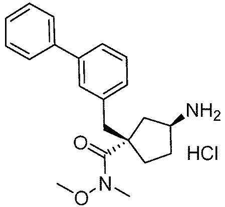 |

**[Table 2]**

| Ref. No. | Str. | Ref. No. | Str. |
|---|---|---|---|
| B-1 | 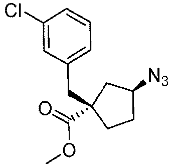 | B-2 | 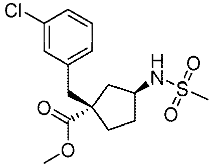 |
| B-3 | 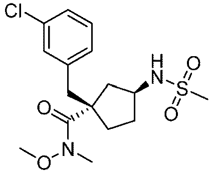 | B-4 | 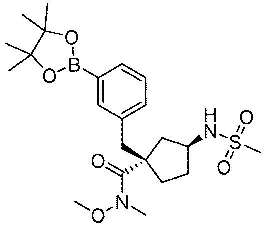 |
| B-5 | 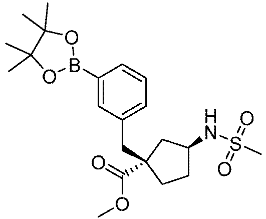 | B-6 | 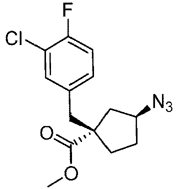 |
| B-7 | 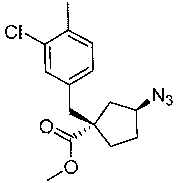 | B-8 | 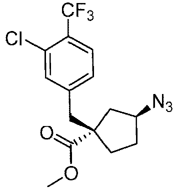 |
| B-9 | 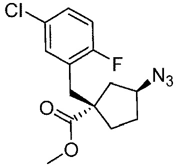 | B-10 | 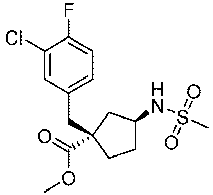 |

**[Table 3]**

| Ref. No. | Str. | Ref. No. | Str. |
|---|---|---|---|
| B-11 | 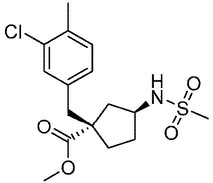 | B-12 | 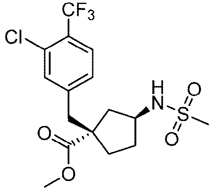 |
| B-13 | 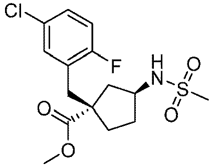 | B-14 | 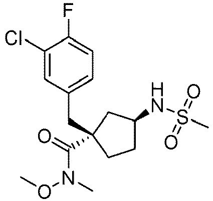 |
| B-15 | 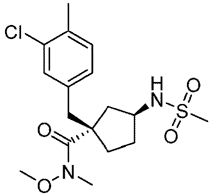 | B-16 | 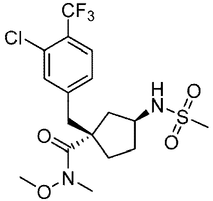 |
| B-17 | 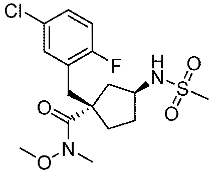 | B-18 | 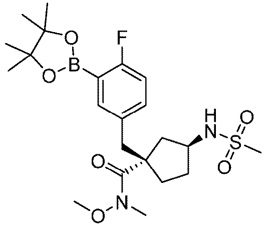 |
| B-19 | 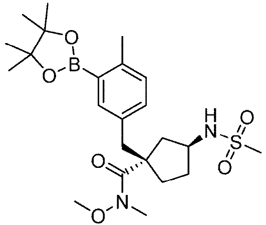 | B-20 | 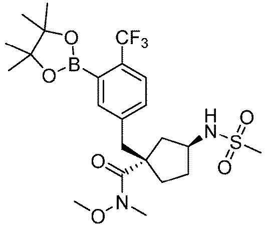 |

**[Table 4]**

| Ref. No. | Str. | Ref. No. | Str. |
|---|---|---|---|
| B-21 | 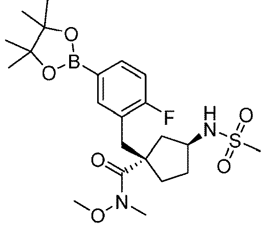 | B-22 | 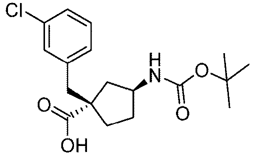 |
| C-1 | 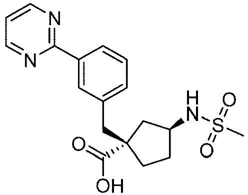 | C-2 | 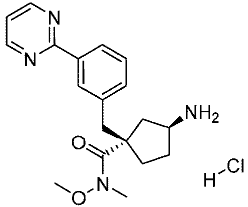 |
| D-1 | 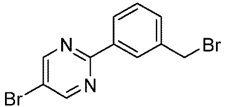 | D-2 | 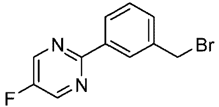 |
| D-3 | 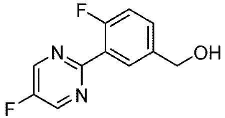 | D-4 | 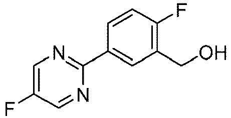 |
| D-5 | 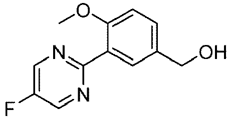 | D-6 | 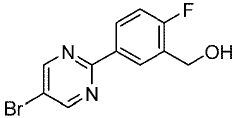 |
| D-7 | 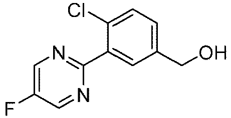 | D-8 | 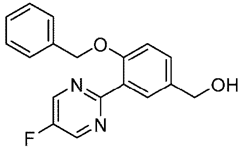 |

**[Table 5]**

| Ref. No. | Str. | Ref. No. | Str. |
|---|---|---|---|
| D-9 | 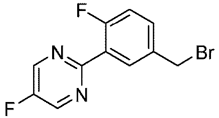 | D-10 | 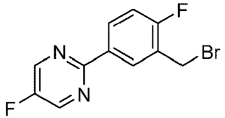 |
| D-11 | 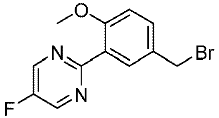 | D-12 | 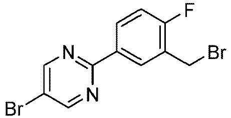 |
| D-13 | 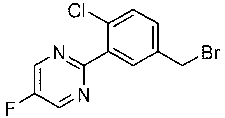 | D-14 | 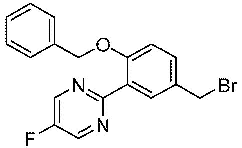 |
| E-1 | 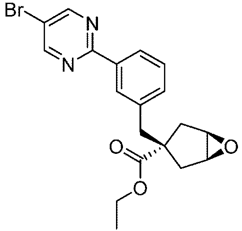 | E-2 | 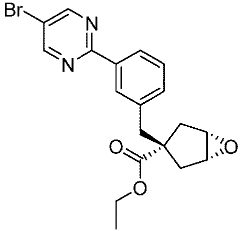 |
| E-3 | 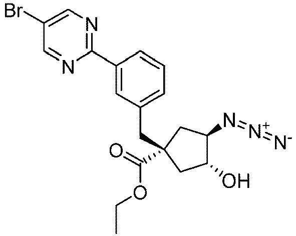 | E-4 | 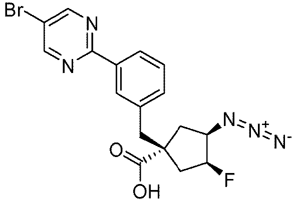 |

**[Table 6]**

| Ref. No. | Str. | Ref. No. | Str. |
|---|---|---|---|
| E-5 | 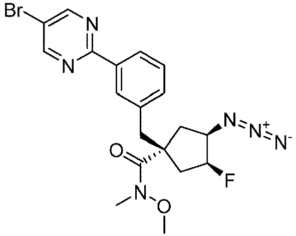 | E-6 | 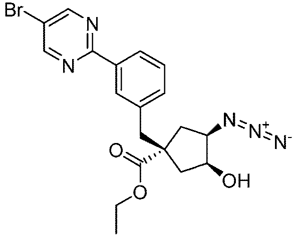 |
| E-7 | 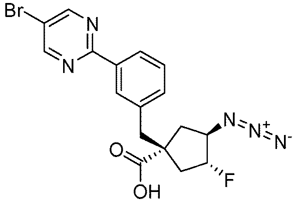 | E-8 | 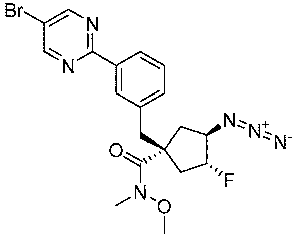 |
| E-9 | 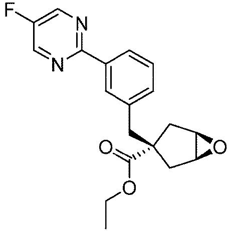 | E-10 | 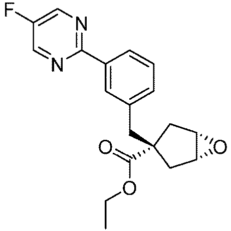 |
| E-11 | 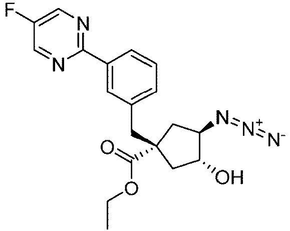 | E-12 | 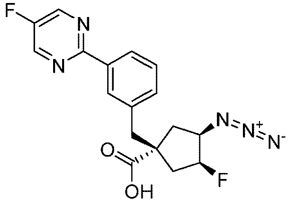 |

**[Table 7]**

| Ref. No. | Str. | Ref. No. | Str. |
|---|---|---|---|
| E-13 | 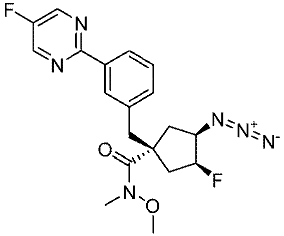 | E-14 | 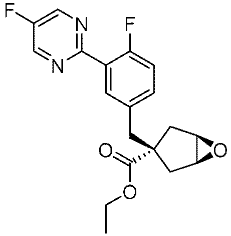 |
| E-15 | 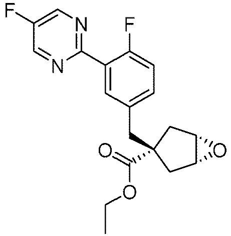 | E-16 | 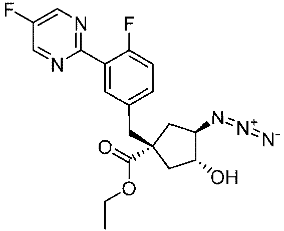 |
| E-17 | 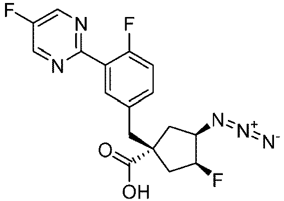 | E-18 | 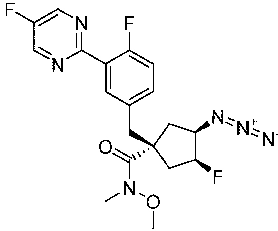 |
| E-19 | 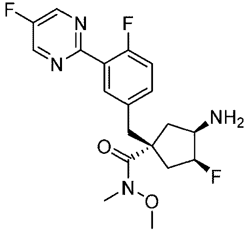 | E-20 | 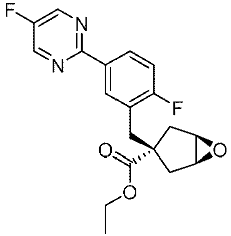 |

**[Table 8]**

| Ref. No. | Str. | Ref. No. | Str. |
|---|---|---|---|
| E-21 | 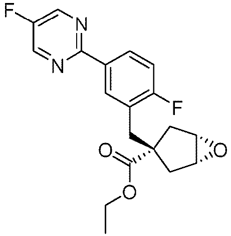 | E-22 | 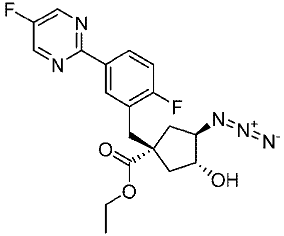 |
| E-23 | 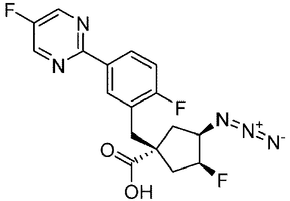 | E-24 | 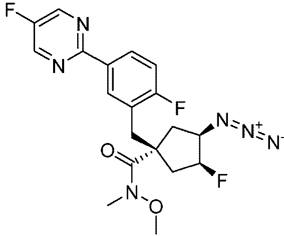 |
| E-25 | 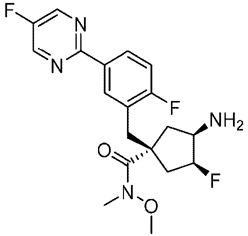 | E-26 | 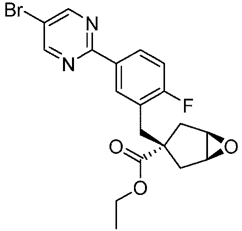 |
| E-27 | 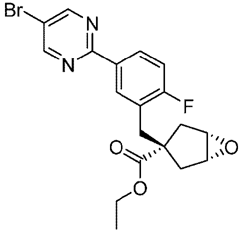 | E-28 | 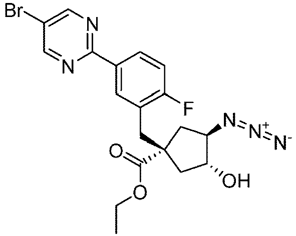 |

**[Table 9]**

| Ref. No. | Str. | Ref. No. | Str. |
|---|---|---|---|
| E-29 | 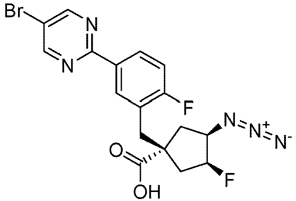 | E-30 | 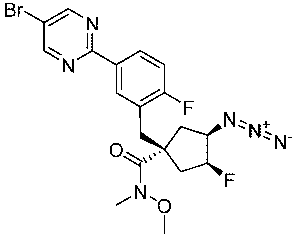 |
| E-31 | 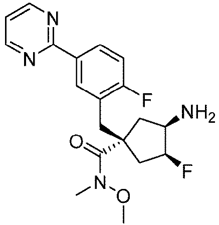 | E-32 | 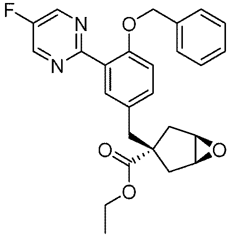 |
| E-33 | 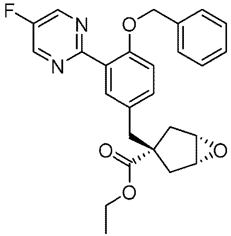 | E-34 | 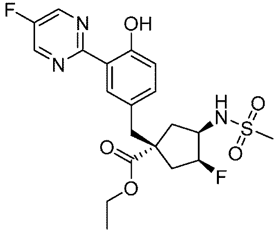 |
| E-35 | 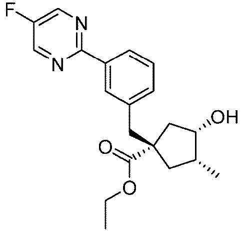 | E-36 | 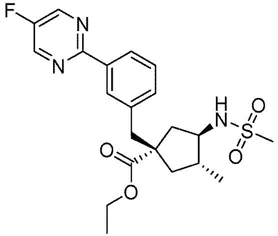 |

**[Table 10]**

| Ref. No. | Str. | Ref. No. | Str. |
|---|---|---|---|
| E-37 | 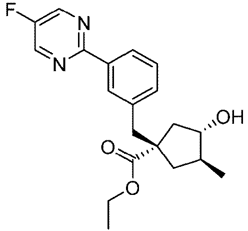 | E-38 | 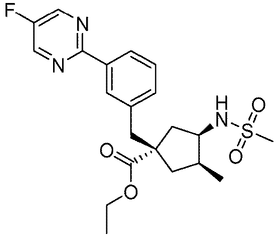 |
| F-1 | 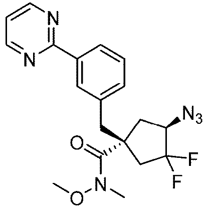 | F-2 | 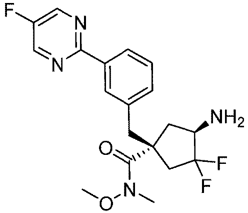 |
| F-3 | 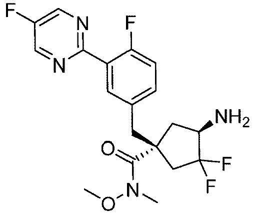 | F-4 | 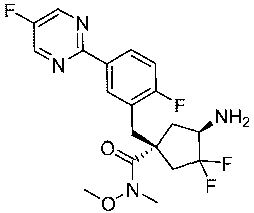 |
| G-1 | 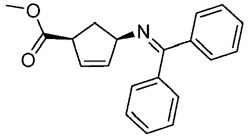 | G-2 | 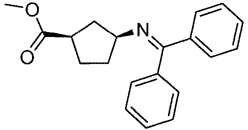 |
| G-3 | 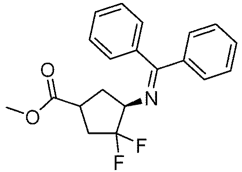 | | |

**[Table 11]**

| Ref. No. | Str. | Ref. No. | Str. |
|---|---|---|---|
| H-1 | 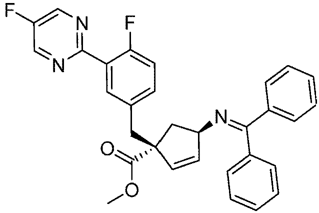 | H-2 | 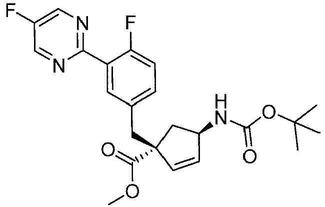 |
| H-3 | 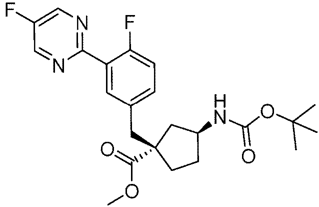 | H-4 | 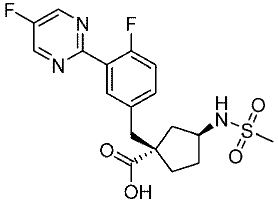 |
| H-5 | 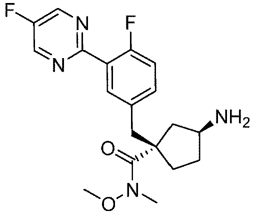 | H-6 | 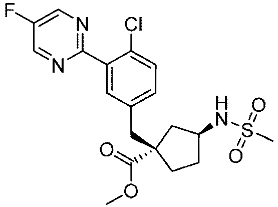 |
| H-7 | 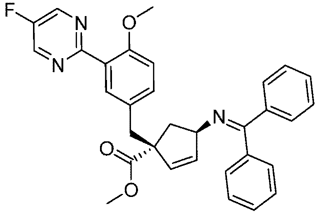 | H-8 | 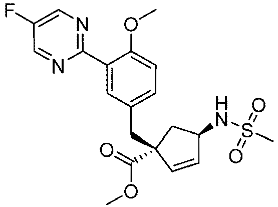 |
| H-9 | 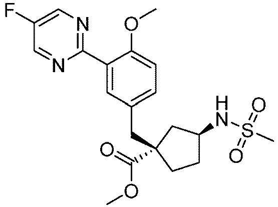 | H-10 | 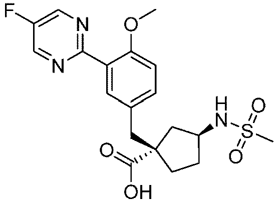 |

**[Table 12]**

| Ref. No. | Str. | Ref. No. | Str. |
|---|---|---|---|
| I-1 | 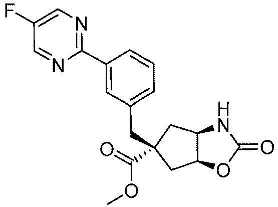 | I-2 | 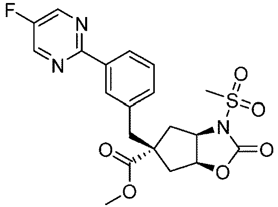 |
| I-3 | 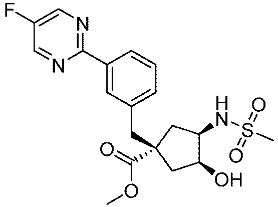 | I-4 | 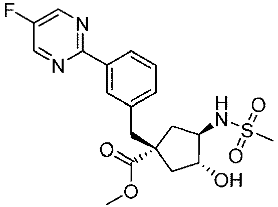 |
| I-5 | 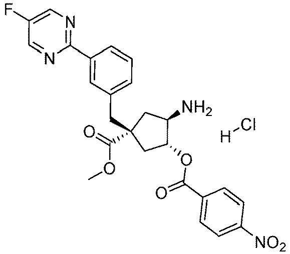 | I-6 | 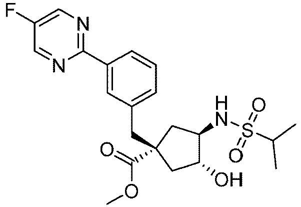 |
| I-7 | 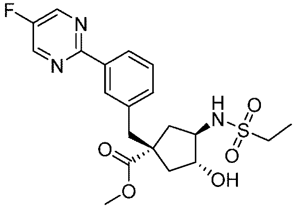 | I-8 | 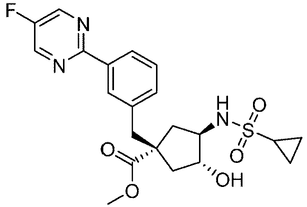 |

**[Table 13]**

| Ref. No. | Str. | Ref. No. | Str. |
|---|---|---|---|
| I-9 | 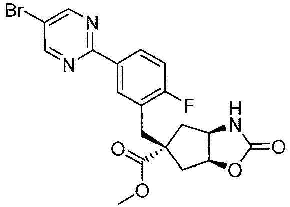 | I-10 | 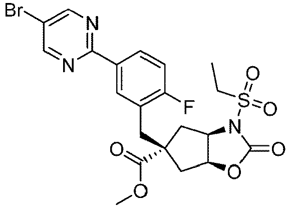 |
| I-11 | 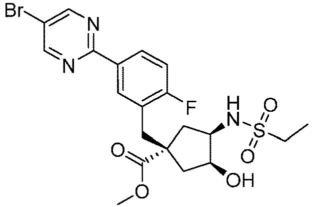 | I-12 | 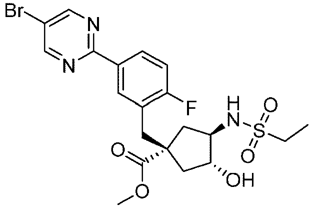 |
| I-13 | 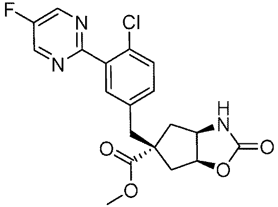 | I-14 | 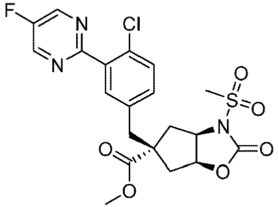 |
| I-15 | 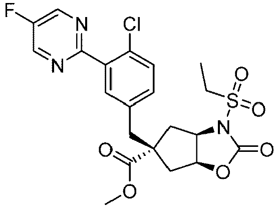 | I-16 | 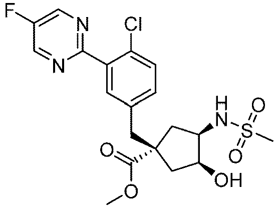 |
| I-17 | 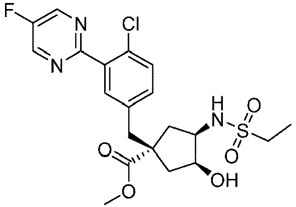 | I-18 | 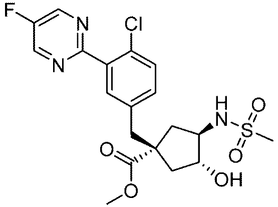 |

**[Table 14]**

| Ref. No. | Str. | Ref. No. | Str. |
|---|---|---|---|
| I-19 | 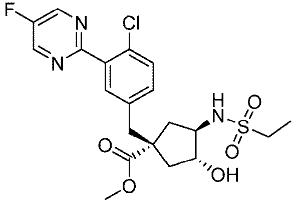 | I-20 | 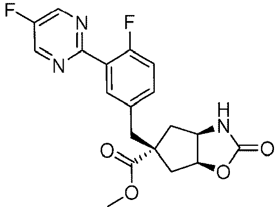 |
| I-21 | 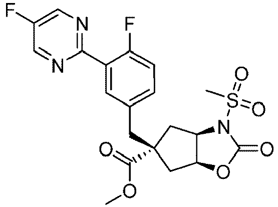 | I-22 | 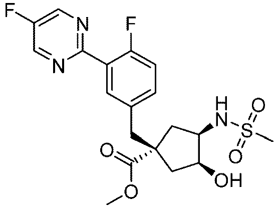 |
| I-23 | 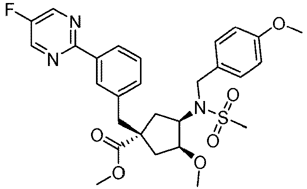 | I-24 | 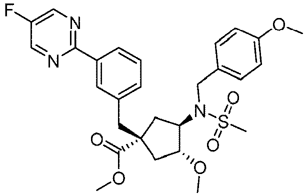 |
| J-1 | 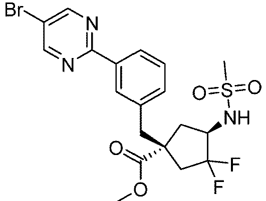 | J-2 | 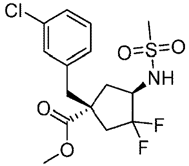 |
| J-3 | 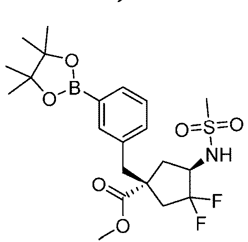 | | |

The stereochemical notations in the formulae of Reference Example A-1 to F-4 represent relative configuration.

### Example 1

### (1R*,3S*)-1-([1,1'-Biphenyl]-3-ylmethyl)-N-ethyl-3-(methylsulfonamido)cyclopentane-1-carboxamide

To a mixture of Reference Example A-2 (0.006 g), ethylamine hydrochloride (0.003 g), DIPEA (0.010 g) and DMF (2 mL) was added HATU (0.007 g) at room temperature, and the mixture was stirred at the same temperature for 30 minutes. To the reaction mixture were added water and ethyl acetate. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=40/60-0/100) to give the title compound (0.006 g).

### Example 2

### (1R*,3S*)-1-([1,1'-Biphenyl]-3-ylmethyl)-N-methoxy-N-methyl-3-(methylsulfonamido)cyclopentane-1-carboxamide

A mixture of Reference Example A-2 (0.015 g), N,O-dimethylhydroxylamine hydrochloride (0.008 g), DIPEA (0.016 g), 1-hydroxybenzotriazole monohydrate (0.009 g), 1-ethyl-3-(3-dimethylaminopropyl)carbodiimide hydrochloride (0.012 g) and DMF (0.5 mL) was stirred at 50 °C for 24 hours, and allowed to cool to room temperature. To the reaction mixture was added water. The mixture was extracted with ethyl acetate. The extract was washed with water and brine, dried over anhydrous magnesium sulfate, and concentrated under reduced pressure. The residue was purified by column chromatography on silica gel (eluent: n-hexane/ethyl acetate=50/50-0/100) to give the title compound (0.013 g).

### Example 3

### (1R*,3S*)-1-((2',6-Difluoro-[1,1'-biphenyl]-3-yl)methyl)-N-methoxy-N-methyl-3-(methylsulfonamido)cyclopentane-1-carboxamide

A mixture of Reference Example B-14 (0.015 g), 2-fluorophenylboronic acid (0.010 g), tripotassium phosphate (0.024 g), Xphos Pd G3 (3.2 mg), 1,2-dimethoxyethane (0.25 mL) and water (0.25 mL) was stirred at 80 °C under microwave irradiation for 30 minutes. The reaction mixture was added to a saturated aqueous solution of ammonium chloride. The mixture was extracted with ethyl acetate. The extract was dried over anhydrous sodium sulfate, and concentrated under reduced pressure. To the residue was added ethyl acetate. The mixture was filtered through a pad of Celite, and the filtrate was concentrated under reduced pressure. The residue was purified by column chromatography on silica gel (eluent: n-hexane/ethyl acetate=100/0-0/100) to give the title compound (0.016 g).

### Example 4 and Example 5

Example 4 and Example 5 were synthesized in a manner similar to that described in Example 3 using the corresponding reagents instead of 2-fluorophenylboronic acid.

### Example 6

### (1R*,3S*)-1-([1,1'-Biphenyl]-3-ylmethyl)-N-methoxy-N-methyl-3-((N-methylsulfamoyl)amino)cyclopentane-1-carboxamide

To a mixture of Reference Example A-4 (0.014 g), DIPEA (0.030 g) and DCM (2mL) was added N-methylsulfamoyl chloride (0.005 mL) at room temperature, and the mixture was stirred at the same temperature for 1 hour. To the reaction mixture were added water and DCM. The mixture was extracted with DCM, and the extract was concentrated under reduced pressure. The residue was purified by column chromatography on silica gel (eluent: n-hexane/ethyl acetate=50/50-0/100) to give the title compound (0.016 g).

### Example 7

### N-((1S*,3R*)-3-([1,1'-Biphenyl]-3-ylmethyl)-3-(isoxazolidine-2-carbonyl)cyclopentyl)methanesulfonamide

To a mixture of Reference Example A-2 (0.015 g) and DCM (1 mL) were added oxalyl chloride (0.016 g) and DMF (4.7 mg) at room temperature, and the mixture was stirred at the same temperature for 30 minutes. To the reaction mixture were added isoxazolidine hydrochloride (0.022 g) and DIPEA (0.104 g) at room temperature, and the mixture was stirred at the same temperature for 30 minutes. To the reaction mixture was added water. The mixture was extracted with DCM, and the extract was concentrated under reduced pressure. The residue was purified by column chromatography on silica gel (eluent: n-hexane/ethyl acetate=20/80-0/100) to give the title compound (0.013 g).

### Example 8 and Example 9

Example 8 and Example 9 were synthesized in a manner similar to that described in Example 7 using the corresponding reagents instead of isoxazolidine hydrochloride.

### Example 10

### (1R*,3S*)-1-((2'-Cyano-[1,1'-biphenyl]-3-yl)methyl)-N-methoxy-N-methyl-3-(methylsulfonamido)cyclopentane-1-carboxamide

A mixture of Reference Example B-4 (0.019 g), 2-bromobenzonitrile (0.011 g), potassium carbonate (0.017 g), Pd(PPh₃)₄ (0.005 g), toluene (1 mL), ethanol (0.5 mL) and water (0.25 mL) was stirred at 120 °C under microwave irradiation for 1 hour. The reaction mixture was filtered through a pad of Celite, and the filtrate was concentrated under reduced pressure. The residue was purified by column chromatography on silica gel (eluent: n-hexane/ethyl acetate=50/50-0/100) to give the title compound (0.013 g).

### Example 11

Example 11 was synthesized in a manner similar to that described in Example 10 using 2-bromophenol instead of 2-bromobenzonitrile.

### Example 12

### (1R*,3S*)-1-(3-(5-Fluoropyridin-2-yl)benzyl)-N-methoxy-N-methyl-3-(methylsulfonamido)cyclopentane-1-carboxamide

A mixture of Reference Example B-4 (0.010 g), 2-bromo-5-fluoropyridine (0.006 g), sodium carbonate (0.007 g), Pd(dppf)Cl₂ (2.0 mg), 1,4-dioxane (0.25 mL), ethanol (0.125 mL) and water (0.125 mL) was stirred at 100 °C under microwave irradiation for 30 minutes. The reaction mixture was concentrated under reduced pressure. The residue was purified by column chromatography on silica gel (eluent: n-hexane/ethyl acetate=50/50-0/100) to give the title compound (0.007 g).

### Example 13 and Example 14

Examples 13 and Examples 14 were synthesized in a manner similar to that described in Example 12 using the corresponding reagents instead of 2-bromo-5-fluoropyridine.

### Example 15

### (1R*,3S*)-1-(3-(5-Chloropyrimidin-2-yl)benzyl)-N-methoxy-N-methyl-3-(methylsulfonamido)cyclopentane-1-carboxamide

A mixture of Reference Example B-4 (0.052 g), 2-bromo-5-chloropyrimidine (0.043 g), sodium carbonate (0.036 g), Pd(dppf)Cl₂ DCM adduct (0.009 g), 1,4-dioxane (0.5 mL), ethanol (0.25 mL) and water (0.25 mL) was stirred at 90 °C under microwave irradiation for 1 hour. The reaction mixture was added to a saturated aqueous solution of ammonium chloride. The mixture was extracted with ethyl acetate. The extract was dried over anhydrous sodium sulfate, and concentrated under reduced pressure. The residue was purified by column chromatography on silica gel (eluent: n-hexane/ethyl acetate=100/0-40/60) to give the title compound (0.050 g).

### Example 16 to Example 20

Example 16 to Example 20 were synthesized in a manner similar to that described in Example 15 using the corresponding reagents instead of 2-bromo-5-chloropyrimidine.

### Example 21

### (1R*,3S*)-1-(3-(5-(Dimethylamino)pyrimidin-2-yl)benzyl)-N-methoxy-N-methyl-3-(methylsulfonamido)cyclopentane-1-carboxamide

A mixture of Example 15 (0.010 g), 9.5 mol/L aqueous solution of dimethylamine (0.005 mL), Pd(dba)₂ (0.6 mg), sodium tert-butoxide (0.006 g), 2-dicyclohexylphosphino-2',6'-diisopropoxy-1,1'-biphenyl (0.5 mg) and 1,4-dioxane (0.5 mL) was stirred at 110 °C under microwave irradiation for 2 hours. The reaction mixture was added to water, and extracted with ethyl acetate. The extract was dried over anhydrous sodium sulfate, and concentrated under reduced pressure. The residue was purified by column chromatography on silica gel (eluent: n-hexane/ethyl acetate=100/0-0/100), and then purified by column chromatography on ODS (eluent: water/MeCN=70/30-10/90) to give the title compound (2.4 mg).

### Example 22 to Example 26

Example 22 to Example 26 were synthesized in a manner similar to that described in Reference Example 15 using corresponding Reference Examples and reagents instead of Reference Example B-4 and 2-bromo-5-chloropyrimidine, respectively.

### Example 27

### (1R*,3S*)-1-(3-(2H-1,2,3-Triazol-2-yl)benzyl)-N-methoxy-N-methyl-3-(methylsulfonamido)cyclopentane-1-carboxamide

A mixture of Reference Example B-3 (0.034 g), 2H-1,2,3-triazol (0.019 g), tripotassium phosphate (0.096 g), Pd₂(dba)₃ chloroform adduct (0.019 g), Me4tBuXPhos (0.017 g) and toluene (2 mL) was stirred at 140 °C under microwave irradiation for 3 hours. The reaction mixture was concentrated under reduced pressure. The residue was purified by column chromatography on silica gel (eluent: n-hexane/ethyl acetate=50/50-0/100), and then purified by column chromatography on ODS (eluent: water/MeCN=70/30-10/90) to give the title compound (2.0 mg).

### Example 28

### (1R*,3S*)-N-Ethyl-N-methyl-3-(methylsulfonamido)-1-(3-(pyrimidin-2-yl)benzyl)cyclopentane-1-carboxamide

A mixture of Reference Example C-1 (0.035 g), N-methylethylamine (0.022 g), HATU (0.046 g), DIPEA (0.060 g) and NMP(0.6 mL) was stirred at 90 °C under microwave irradiation for 1 hour. The reaction mixture was added to 1 mol/L hydrochloric acid, and extracted with ethyl acetate. The extract was washed with brine, dried over anhydrous sodium sulfate, and concentrated under reduced pressure. The residue was purified by column chromatography on silica gel (eluent: n-hexane/ethyl acetate=100/0-0/100) to give the title compound (0.032 g).

### Example 29

### (1R*,3S*)-N-Methoxy-3-(methylsulfonamido)-1-(3-(pyrimidin-2-yl)benzyl)cyclopentane-1-carboxamide

To a mixture of Reference Example C-1 (0.008 g), DMF (0.009 g) and DCM (0.5 mL) was added oxalyl chloride (0.006 g) at room temperature, and the mixture was stirred at the same temperature for 30 minutes. To the reaction mixture was added a mixture of methoxyamine hydrochloride (0.012 g), DIPEA (0.056 g) and DCM (0.5 mL) at room temperature. The mixture was stirred at the same temperature for 30 minutes. To the reaction mixture was added water. The mixture was extracted with DCM, and the extract was concentrated under reduced pressure. The residue was purified by column chromatography on silica gel (eluent: ethyl acetate/methanol=100/0-80/20) to give the title compound (0.006 g).

### Example 30

### (1R*,3S*)-N-Hydroxy-N-methyl-3-(methylsulfonamido)-1-(3-(pyrimidin-2-yl)benzyl)cyclopentane-1-carboxamide

To a mixture of Reference Example C-1 (0.015 g), DMF (0.009 g) and DCM (0.5 mL) was added oxalyl chloride (0.011 g) at room temperature, and the mixture was stirred at the same temperature for 30 minutes. To the reaction mixture was added a mixture of N-methylhydroxylamine hydrochloride (0.024 g), DIPEA (0.104 g) and DCM (0.5 mL) at room temperature. The mixture was stirred at the same temperature for 30 minutes. To the reaction mixture was added 2 mol/L aqueous solution of sodium hydroxide (1 mL), and the mixture was stirred for 5 minutes at room temperature. To the reaction mixture was added water. The mixture was extracted with DCM, and the extract was concentrated under reduced pressure. The residue was purified by column chromatography on silica gel (eluent: ethyl acetate/methanol=100/0-95/5) to give the title compound (1.3 mg).

### Example 31

### (1R*,3S*)-N-Methoxy-N-methyl-3-((1-methylcyclopropane)-1-sulfonamido)-1-(3-(pyrimidin-2-yl)benzyl)cyclopentane-1-carboxamide

To a mixture of Reference Example C-2 (0.006 g), DIPEA (0.012 g) and DCM (2 mL) was added 1-methylcyclopropane-1-sulfonyl chloride (0.010 g) at room temperature, and the mixture was stirred at the same temperature for 30 minutes. To the reaction mixture were added DIPEA (0.082 g) and 1-methylcyclopropane-1-sulfonyl chloride (0.048 g) at room temperature, and the mixture was stirred at the same temperature for 30 minutes. To the reaction mixture was added water. The mixture was extracted with DCM, and the extract was concentrated under reduced pressure. The residue was purified by column chromatography on silica gel (eluent: n-hexane/ethyl acetate=30/70-0/100), and then purified by column chromatography on APS (eluent: n-hexane/ethyl acetate=30/70-0/100) to give the title compound (1.0 mg).

### Example 32

### (1R*,3R*,4R*)-3-Fluoro-N-methoxy-N-methyl-4-(methylsulfonamido)-1-(3-(pyrimidin-2-yl)benzyl)cyclopentane-1-carboxamide

To a mixture of Reference Example E-8 (0.053 g), TEA (0.017 g), ethanol (0.5 mL) and THF (0.5 mL) was added 10% Pd/C (0.011 g) under ice-cooling. The mixture was stirred under a hydrogen atmosphere at room temperature for 2.5 hours. The reaction mixture was filtered through a pad of Celite, and the filtrate was concentrated under reduced pressure.

To a mixture of the residue, TEA (0.035 g) and DCM (1 mL) was added methanesulfonyl chloride (0.020 g) under ice-cooling, and the mixture was stirred at room temperature for 30 minutes. To the reaction mixture were added water and brine. The mixture was extracted with DCM. The extract was dried over anhydrous sodium sulfate, and concentrated under reduced pressure. The residue was purified by column chromatography on silica gel (eluent: n-hexane/ethyl acetate/methanol=50/50/0-0/100/0-0/90/10) to give the title compound (0.035 g).

### Example 33

Example 33 was synthesized in a manner similar to that described in Example 32 using Reference Example E-5 instead of Reference Example E-8.

### Example 34

### (1R*,3R*,4S*)-3-((N,N-Dimethylsulfamoyl)amino)-4-fluoro-1-(4-fluoro-3-(5-fluoropyrimidin-2-yl)benzyl)-N-methoxy-N-methylcyclopentane-1-carboxamide

A mixture of Reference Example E-19 (0.013 g), dimethylsulfamoyl chloride (0.270 g), DMAP (0.012 g) and DIPEA (0.012 g) was stirred at 60 °C for 5 hours. The reaction mixture was allowed to cool to room temperature, and then added to a saturated aqueous solution of sodium bicarbonate. The mixture was extracted with ethyl acetate. The extract was dried over anhydrous sodium sulfate, and concentrated under reduced pressure. The residue was purified by column chromatography on silica gel (eluent: n-hexane/ethyl acetate=40/60) to give the title compound (0.008 g).

### Example 35

### (1R*,3R*,4S*)-3-((N,N-Dimethylsulfamoyl)amino)-4-fluoro-1-(3-(5-fluoropyrimidin-2-yl)benzyl)-N-methoxy-N-methylcyclopentane-1-carboxamide

To a mixture of Reference Example E-13 (0.022 g), ethanol (0.5 mL) and THF (0.5 mL) was added 10% Pd/C (0.004 g) under ice-cooling. The mixture was stirred under a hydrogen atmosphere at room temperature for 1 hour. The reaction mixture was filtered through a pad of Celite, and the filtrate was concentrated under reduced pressure.

A mixture of the residue, dimethylsulfamoyl chloride (0.405 g), DMAP (0.020 g) and DIPEA (0.021 g) was stirred at 60 °C for 3 hours, and allowed to cool to room temperature. To the reaction mixture were added water, brine and a saturated aqueous solution of sodium bicarbonate. The mixture was extracted with ethyl acetate. The extract was dried over anhydrous magnesium sulfate, and concentrated under reduced pressure. The residue was purified by column chromatography on silica gel (eluent: n-hexane/ethyl acetate=50/50-20/80) to give the title compound (0.008 g).

### Example 36

### (1R*,3R*,4S*)-3-(Ethylsulfonamido)-4-fluoro-1-(4-fluoro-3-(5-fluoropyrimidin-2-yl)benzyl)-N-methoxy-N-methylcyclopentane-1-carboxamide

Under an argon atmosphere, to a mixture of Reference Example E-19 (0.067 g) and pyridine (1 mL) was added ethanesulfonyl chloride (0.065 g) at room temperature, and the mixture was stirred at the same temperature for 6 hours. The reaction mixture was added to water. The mixture was extracted with DCM. The extract was dried over anhydrous sodium sulfate, and concentrated under reduced pressure. To the residue was added toluene, and the mixture was concentrated under reduced pressure. The residue was purified by column chromatography on silica gel (eluent: n-hexane/ethyl acetate=50/50-40/60) to give the title compound (0.039 g).

### Example 37

### (1R*,3S*,4R*)-3-Fluoro-1-(2-fluoro-5-(5-fluoropyrimidin-2-yl)benzyl)-N-methoxy-N-methyl-4-(methylsulfonamido)cyclopentane-1-carboxamide

Under an argon atmosphere, to a mixture of Reference Example E-25 (0.051 g), TEA (0.052 g) and DCM (1 mL) was added methanesulfonyl chloride (0.044 g) under ice-cooling, and the mixture was stirred at room temperature for 15 minutes. The reaction mixture was added to a saturated aqueous solution of sodium bicarbonate, and extracted with DCM. The extract was dried over anhydrous sodium sulfate, and concentrated under reduced pressure. The residue was purified by column chromatography on silica gel (eluent: n-hexane/ethyl acetate=60/40), and then purified by column chromatography on APS (eluent: n-hexane/ethyl acetate=50/50) to give the title compound (0.042 g).

### Example 38

### (1R*,3S*,4R*)-3-Fluoro-1-(2-fluoro-5-(pyrimidin-2-yl)benzyl)-N-methoxy-N-methyl-4-((trifluoromethyl)sulfonamido)cyclopentane-1-carboxamide

Under an argon atmosphere, to a mixture of Reference Example E-31 (0.015 g), TEA (0.008 g) and DCM (0.4 mL) was added a mixture of trifluoromethanesulfonyl chloride (0.010 g) and DCM (0.05 mL) under ice-cooling, and the mixture was stirred at the same temperature for 20 minutes. To the reaction mixture was added TEA (0.008 g) under ice-cooling, and then a mixture of trifluoromethanesulfonyl chloride (0.010 g) and DCM (0.05 mL) was added to the mixture. The mixture was stirred at the same temperature for 30 minutes. To the reaction mixture was added TEA (0.020 g) under ice-cooling, and then a mixture of trifluoromethanesulfonyl chloride (0.027 g) and DCM (0.05 mL) was added to the mixture. The mixture was stirred at the same temperature for 20 minutes. To the reaction mixture was added water under ice-cooling. The mixture was extracted with DCM. The extract was dried over anhydrous sodium sulfate, and concentrated under reduced pressure. The residue was purified by column chromatography on silica gel (eluent: n-hexane/ethyl acetate=70/30-50/50) to give the title compound (0.003 g).

### Example 39

### (1R*,3S*,4R*)-3-Fluoro-1-(3-(5-fluoropyrimidin-2-yl)-4-hydroxybenzyl)-N-methoxy-N-methyl-4-(methylsulfonamido)cyclopentane-1-carboxamide

A mixture of Reference Example E-34 (0.010 g), 6 mol/L hydrochloric acid (0.1 mL) and 1,4-dioxane (0.5 mL) was stirred at 100 °C for 5 hours, and then stirred at room temperature for 18 hours. To the reaction mixture was added concentrated hydrochloric acid (0.1 mL) at room temperature. The mixture was stirred at 100 °C for 4 hours, and allowed to cool to room temperature. To the reaction mixture were added concentrated hydrochloric acid (0.1 mL) and 1,4-dioxane (0.5 mL) at room temperature. The mixture was stirred at 100 °C for 1 hour, and allowed to cool to room temperature. To the reaction mixture was added a saturated aqueous solution of sodium bicarbonate to basify. The mixture was washed with DCM. To the aqueous layer was added 2 mol/L hydrochloric acid to adjust the pH to 1 to 2. The mixture was extracted with DCM. The extract was dried over anhydrous sodium sulfate, and concentrated under reduced pressure.

To a mixture of the residue, N,O-dimethylhydroxylamine hydrochloride (0.007 g), DIPEA (0.012 g) and DMF (0.3 ml) was added HATU (0.009 g) at room temperature. The mixture was stirred at 70 °C for 1 hour, and allowed to cool to room temperature. To the reaction mixture was added water. The mixture was extracted with ethyl acetate. The extract was dried over anhydrous sodium sulfate, and concentrated under reduced pressure. The residue was purified by column chromatography on silica gel (eluent: n-hexane/ethyl acetate=60/40-20/80). To the obtained objective substance were added ethyl acetate and 2 mol/L hydrochloric acid, and the mixture was extracted with ethyl acetate. The extract was dried over anhydrous sodium sulfate, and concentrated under reduced pressure to give the title compound (0.006 g).

### Example 40

### (1R*,4R*)-3,3-Difluoro-N-methoxy-N-methyl-4-(methylsulfonamido)-1-(3-(pyrimidin-2-yl)benzyl)cyclopentane-1-carboxamide

To a mixture of Reference Example F-1 (0.043 g), ethanol (0.5 mL) and THF (0.5 mL) was added 10% Pd/C (0.009 g) under ice-cooling. The mixture was stirred under a hydrogen atmosphere at room temperature for 1 hour. The reaction mixture was filtered through a pad of Celite, and the filtrate was concentrated under reduced pressure.

To a mixture of the residue, TEA (0.022 g) and DCM (1 mL) was added methanesulfonyl chloride (0.012 g) under ice-cooling, and the mixture was stirred at room temperature for 15 minutes. To the reaction mixture was added water. The mixture was extracted with DCM, and the extract was concentrated under reduced pressure. The residue was purified by column chromatography on APS (eluent: n-hexane/ethyl acetate=50/50-0/100) to give the title compound (0.011 g).

### Example 41

Example 41 was synthesized in a manner similar to that described in Example 34 using Reference Example F-2 instead of Reference Example E-19.

### Example 42 and Example 43

Example 42 and Example 43 was synthesized in a manner similar to that described in Example 37 using corresponding Reference Examples instead of Reference Example E-25.

### Example 44

### (1R*,3R*,4R*)-1-(3-(5-Fluoropyrimidin-2-yl)benzyl)-N-methoxy-N,3-dimethyl-4-(methylsulfonamido)cyclopentane-1-carboxamide

A mixture of Reference Example E-36 (0.018 g), 6 mol/L hydrochloric acid (1 mL) and 1,4-dioxane (1 mL) was stirred at 100 °C for 8 hours, and allowed to cool to room temperature. To the reaction mixture was added water. The mixture was extracted with DCM. The extract was dried over anhydrous sodium sulfate, and concentrated under reduced pressure.

To a mixture of the residue, DMF (0.3 mg) and DCM (1 mL) was added oxalyl chloride (0.008 g) under ice-cooling, and the mixture was stirred at room temperature for 30 minutes. The reaction mixture was concentrated under reduced pressure. A mixture of the residue and DCM (0.5 mL) was added to a mixture of N,O-dimethylhydroxylamine hydrochloride (0.008 g), DIPEA (0.016 g) and DCM (0.5 mL) under ice-cooling. The mixture was stirred at room temperature for 15 minutes, and concentrated under reduced pressure. The residue was purified by column chromatography on silica gel (eluent: n-hexane/ethyl acetate=100/0-70/30) to give the title compound (0.008 g).

### Example 45

### (1R*,3S*,4R*)-1-(3-(5-Fluoropyrimidin-2-yl)benzyl)-N-methoxy-N,3-dimethyl-4-(methylsulfonamido)cyclopentane-1-carboxamide

A mixture of Reference Example E-38 (0.245 g), 6 mol/L hydrochloric acid (4 mL) and 1,4-dioxane (4 mL) was stirred at 100 °C for 8 hours, and allowed to cool to room temperature. To the reaction mixture was added water. The mixture was extracted with DCM. The extract was dried over anhydrous sodium sulfate, and concentrated under reduced pressure.

To a mixture of the residue (0.051 g), DMF (0.9 mg) and DCM (1 mL) was added oxalyl chloride (0.048 g) under ice-cooling, and the mixture was stirred at room temperature for 30 minutes. The reaction mixture was concentrated under reduced pressure. A mixture of the residue and DCM (0.5 mL) was added to a mixture of N,O-dimethylhydroxylamine hydrochloride (0.024 g), DIPEA (0.113 g) and DCM (0.5 mL) under ice-cooling. The mixture was stirred at room temperature for 15 minutes, and concentrated under reduced pressure. The residue was purified by column chromatography on silica gel (eluent: n-hexane/ethyl acetate=100/0-70/30) to give the title compound (0.036 g).

### Example 46 and Example 47

### N-Methoxy-N-methyl-3-(methylsulfonamido)-1-(3-(pyrimidin-2-yl)benzyl)cyclopentane-1-carboxamide (Example 46, first peak)

### N-Methoxy-N-methyl-3-(methylsulfonamido)-1-(3-(pyrimidin-2-yl)benzyl)cyclopentane-1-carboxamide (Example 47, second peak)

A mixture of Reference Example B-4 (0.305 g), 2-bromopyrimidine (0.208 g), sodium carbonate (0.208 g), Pd(dppf)Cl₂ DCM adduct (0.053 g), 1,4-dioxane (5 mL), ethanol (2.5 mL) and water (2.5 mL) was stirred at 90 °C under microwave irradiation for 1 hour. The reaction mixture was filtered through a pad of Celite, and the filtrate was concentrated under reduced pressure. The residue was purified by column chromatography on silica gel (eluent: n-hexane/ethyl acetate=100/0-0/100), and then purified by chiral preparative column chromatography (eluent: n-hexane/ethanol=50/50) to give the Example 46 (0.060 g) as a first peak, and Example 47 (0.056 g) as a second peak.

### Example 48

### (1R,3S)-1-(4-Fluoro-3-(5-fluoropyrimidin-2-yl)benzyl)-N-methoxy-N-methyl-3-(methylsulfonamido)cyclopentane-1-carboxamide

To a mixture of Reference Example H-4 (0.151 g), DMF (2.7 mg) and DCM (1.5 mL) was added oxalyl chloride (0.070 g) under ice-cooling. The mixture was stirred at room temperature for 45 minutes, and concentrated under reduced pressure. A mixture of the residue and DCM (1mL) was added to a mixture of N,O-dimethylhydroxylamine hydrochloride (0.071 g), DIPEA (0.142 g) and DCM (1mL) under ice-cooling. The mixture was stirred at room temperature overnight. To the reaction mixture was added water. The mixture was extracted with DCM, and the extract was concentrated under reduced pressure. The residue was purified by column chromatography on silica gel (eluent: n-hexane/ethyl acetate=50/50-0/100) to give the title compound (0.066 g).

### Example 49

### (1R,3S)-1-(4-Chloro-3-(5-fluoropyrimidin-2-yl)benzyl)-N-methoxy-N-methyl-3-(methylsulfonamido)cyclopentane-1-carboxamide

A mixture of Reference Example H-6 (0.019 g), 2 mol/L aqueous solution of sodium hydroxide (0.215 mL) and 1,4-dioxane (1 mL) was stirred at 80 °C for 2 hours, and allowed to cool to room temperature. To the reaction mixture were added 2 mol/L hydrochloric acid (0.236 mL) and brine. The mixture was extracted with DCM, and the extract was concentrated under reduced pressure.

A mixture of the residue, DIPEA (0.028 g), HATU (0.021 g) and DMF (1 mL) was stirred at 60 °C for 1 hour, and allowed to cool to room temperature. To the reaction mixture was added N,O-dimethylhydroxylamine hydrochloride (0.017 g) at room temperature. The mixture was stirred at 70 °C for 2 hours, and then stirred at room temperature for 22 hours. To the reaction mixture were added water and a saturated aqueous solution of sodium bicarbonate. The mixture was extracted with ethyl acetate. The extract was washed with brine, dried over anhydrous magnesium sulfate, and concentrated under reduced pressure. The residue was purified by Method A (eluent: n-hexane/ethyl acetate=50/50-0/100), and then purified by column chromatography on ODS (eluent: water/MeCN=90/10-10/90) to give the title compound (0.004 g).

### Example 50

### (1R,3S)-1-(4-Fluoro-3-(5-fluoropyrimidin-2-yl)benzyl)-3-((fluoromethyl)sulfonamido)-N-methoxy-N-methylcyclopentane-1-carboxamide

Under an argon atmosphere, to a mixture of Reference Example H-5 (0.014 g), TEA (0.008 g) and DCM (0.4 mL) was added fluoromethanesulfonyl chloride (0.007 g) under ice-cooling, and the mixture was stirred at room temperature for 30 minutes. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=60/40-50/50) to give the title compound (0.010 g).

### Example 51

### (1R,3S)-3-((Difluoromethyl)sulfonamido)-1-(4-fluoro-3-(5-fluoropyrimidin-2-yl)benzyl)-N-methoxy-N-methylcyclopentane-1-carboxamide

Under an argon atmosphere, to a mixture of Reference Example H-5 (0.017 g), pyridine (0.018 g) and MeCN (1 mL) was added difluoromethanesulfonyl chloride (0.008 g) under ice-cooling, and the mixture was stirred at room temperature for 17 hours. To the reaction mixture was added difluoromethanesulfonyl chloride (0.008 g) under ice-cooling, and the mixture was stirred at room temperature for 3 hours. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=60/40-50/50) to give the title compound (0.004 g).

### Example 52

### (1R,3S)-3-(Ethylsulfonamido)-1-(4-fluoro-3-(5-fluoropyrimidin-2-yl)benzyl)-N-methoxy-N-methylcyclopentane-1-carboxamide

Under an argon atmosphere, to a mixture of Reference Example H-5 (0.032 g) and pyridine (0.8 mL) was added ethanesulfonyl chloride (0.033 g) under ice-cooling, and the mixture was stirred at room temperature for 2.5 hours. To the reaction mixture was added water. The mixture was extracted with DCM. The extract was dried over anhydrous sodium sulfate, and concentrated under reduced pressure. To the residue was added toluene, and the mixture was concentrated under reduced pressure. The residue was purified by column chromatography on silica gel (eluent: n-hexane/ethyl acetate=70/30-40/60) to give the title compound (0.011 g).

### Example 53

### (1R,3S)-1-(4-Fluoro-3-(5-fluoropyrimidin-2-yl)benzyl)-N-methoxy-N-methyl-3-(vinylsulfonamido)cyclopentane-1-carboxamide

Under an argon atmosphere, to a mixture of Reference Example H-5 (0.016 g), TEA (0.009 g) and DCM (0.4 mL) was added 2-fluoroethane-1-sulfonyl chloride (0.009 g) under ice-cooling, and the mixture was stirred at room temperature for 2.5 hours. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=60/40-40/60) to give the title compound (0.005 g).

### Example 54

### (1R,3S)-1-(4-Fluoro-3-(5-fluoropyrimidin-2-yl)benzyl)-N-methoxy-N-methyl-3-(oxetane-3-sulfonamido)cyclopentane-1-carboxamide

Under an argon atmosphere, to a mixture of Reference Example H-5 (0.022 g), 1,8-diazabicyclo[5.4.0]-7-undecene (0.036 g) and DCM (0.5 mL) was added oxetane-3-sulfonyl chloride (0.018 g) under ice-cooling, and the mixture was stirred at room temperature for 50 minutes. To the reaction mixture was added oxetane-3-sulfonyl chloride (0.018 g) at room temperature, and the mixture was stirred at the same temperature for 25 minutes. To the reaction mixture were added 1,8-diazabicyclo[5.4.0]-7-undecene (0.018 g) and oxetane-3-sulfonyl chloride (0.018 g) at room temperature, and the mixture was stirred at the same temperature for 15 minutes. To the reaction mixture was added water. The mixture was extracted with DCM. The extract was dried over anhydrous sodium sulfate, and concentrated under reduced pressure. The residue was purified by column chromatography on silica gel (eluent: n-hexane/ethyl acetate=80/20-60/40) to give the title compound (0.007 g).

### Example 55

### (1R,3S)-1-(3-(5-Fluoropyrimidin-2-yl)-4-hydroxybenzyl)-N-methoxy-N-methyl-3-(methylsulfonamido)cyclopentane-1-carboxamide

To a mixture of Reference Example H-10 (0.050 g) and DCM (1 mL) was added boron tribromide (1 mol/L in DCM, 0.236 mL) at -78 °C, and the mixture was stirred at the same temperature for 1 hour. To the mixture was added water at the same temperature, and the mixture was stirred at room temperature for 10 minutes. The mixture was extracted with DCM. The extract was dried over anhydrous magnesium sulfate, and concentrated under reduced pressure.

To a mixture of the residue, N,O-dimethylhydroxylamine hydrochloride (0.018 g), DIPEA (0.030 g) and DMF (1 mL) was added HATU (0.019 g) at room temperature. The mixture was stirred at 70 °C for 1 hour, and allowed to cool to room temperature. To the reaction mixture was added water. The mixture was extracted with ethyl acetate, and the extract was concentrated under reduced pressure. The residue was purified by column chromatography on silica gel (eluent: n-hexane/ethyl acetate/methanol=50/50/0-0/100/0-0/80/20) to give the title compound (0.002 g).

### Example 56

### (1R,3S)-1-(3-(5-Fluoropyrimidin-2-yl)-4-methoxybenzyl)-N-methoxy-N-methyl-3-(methylsulfonamido)cyclopentane-1-carboxamide

To a mixture of Reference Example H-10 (0.010 g), N,O-dimethylhydroxylamine hydrochloride (0.009 g), DIPEA (0.015 g) and DMF (1 mL) was added HATU (0.010 g) at room temperature. The mixture was stirred at the same temperature for 1 hour, and then stirred at 70 °C for 2 hours. The mixture was allowed to cool to room temperature. To the reaction mixture was added water. The mixture was extracted with ethyl acetate, and the extract was concentrated under reduced pressure. The residue was purified by column chromatography on silica gel (eluent: n-hexane/ethyl acetate/methanol=50/50/0-0/100/0-0/80/20) to give the title compound (0.010 g).

### Example 57

### (1R,3S,4R)-3-Fluoro-1-(3-(5-fluoropyrimidin-2-yl)benzyl)-N-methoxy-N-methyl-4-(methylsulfonamido)cyclopentane-1-carboxamide

To a mixture of Reference Example I-4 (0.201 g) and DCM (10 mL) was added Deoxo-Fluor (0.158 g) under ice-cooling. The mixture was stirred at the same temperature for 30 minutes, and then stirred at room temperature for 2 hours. To the reaction mixture was added Deoxo-Fluor (0.158 g) at room temperature. The mixture was stirred at the same temperature for 15 hours. The reaction mixture was added to a saturated aqueous solution of sodium bicarbonate. The mixture was extracted with DCM, and the extract was concentrated under reduced pressure. The residue was purified by column chromatography on silica gel (eluent: n-hexane/ethyl acetate=70/30-0/100) to give methyl (1R,3S,4R)-3-fluoro-1-(3-(5-fluoropyrimidin-2-yl)benzyl)-4-(methylsulfonamido)cyclopentane-1-carboxylate (0.145 g).

A mixture of the obtained compound (0.145 g), 2 mol/L aqueous solution of sodium hydroxide (0.852 mL) and 2-propanol (4 mL) was stirred at 70 °C for 2 hours, and allowed to cool to room temperature. To the reaction mixture was added 2 mol/L hydrochloric acid (0.937 mL). The mixture was extracted with DCM. The extract was dried over anhydrous sodium sulfate, and concentrated under reduced pressure.

To a mixture of the residue, N,O-dimethylhydroxylamine hydrochloride (0.100 g), DIPEA (0.308 g) and DMF (2 mL) was added HATU (0.143 g) at room temperature. The mixture was stirred at 70 °C for 1 hour, and allowed to cool to room temperature. To the reaction mixture was added water. The mixture was extracted with ethyl acetate. The extract was washed with water and brine, dried over anhydrous sodium sulfate, and concentrated under reduced pressure. The residue was successively purified by column chromatography on silica gel (eluent: n-hexane/ethyl acetate=50/50-0/100), column chromatography on silica gel (eluent: n-hexane/ethyl acetate=40/60), column chromatography on APS (eluent: n-hexane/ethyl acetate=40/60) and column chromatography on ODS (eluent: water/MeCN=90/10-10/90) to give the title compound (0.042 g).

### Example 58

### (1R,3S,4R)-3-Fluoro-1-(3-(5-fluoropyrimidin-2-yl)benzyl)-N-methoxy-N-methyl-4-((1-methylethyl)sulfonamido)cyclopentane-1-carboxamide

To a mixture of Reference Example I-6 (0.032 g) and DCM (2 mL) was added Deoxo-Fluor (0.024 g) under ice-cooling. The mixture was stirred at the same temperature for 30 minutes, and then stirred at room temperature for 1 hour. The reaction mixture was added to a saturated aqueous solution of sodium bicarbonate. The mixture was extracted with DCM, and the extract was concentrated under reduced pressure. The residue was purified by column chromatography on silica gel (eluent: n-hexane/ethyl acetate=80/20-50/50) to give methyl (1R,3S,4R)-3-fluoro-1-(3-(5-fluoropyrimidin-2-yl)benzyl)-4-((1-methylethyl)sulfonamido)cyclopentane-1-carboxylate (0.020 g).

A mixture of the obtained compound (0.020 g), 2 mol/L aqueous solution of sodium hydroxide (0.110 mL) and 2-propanol (2 mL) was stirred at 70 °C for 1 hour, and allowed to cool to room temperature. To the reaction mixture was added 2 mol/L hydrochloric acid (0.121 mL). The mixture was extracted with DCM. The extract was dried over anhydrous sodium sulfate, and concentrated under reduced pressure.

To a mixture of the residue, N,O-dimethylhydroxylamine hydrochloride (0.013 g), DIPEA (0.040 g) and DMF (2 mL) was added HATU (0.018 g) at room temperature. The mixture was stirred at 70 °C for 1 hour, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=50/50-30/70), and then purified by column chromatography on ODS (eluent: water/MeCN=70/30-10/90) to give the title compound (0.010 g).

### Example 59

### (1R,3R,4S)-3-(Ethylsulfonamido)-4-fluoro-1-(3-(5-fluoropyrimidin-2-yl)benzyl)-N-methoxy-N-methylcyclopentane-1-carboxamide

To a mixture of Reference Example I-7 (0.191 g), pyridine (0.104 g) and DCM (4 mL) was added Deoxo-Fluor (0.145 g) under ice-cooling. The mixture was stirred at the same temperature for 30 minutes, and then stirred at room temperature for 20 hours. To the reaction mixture were added a saturated aqueous solution of sodium bicarbonate and water. The mixture was extracted with DCM. The extract was dried over anhydrous sodium sulfate, and concentrated under reduced pressure. The residue was purified by column chromatography on silica gel (eluent: n-hexane/ethyl acetate=80/20-50/50) to give methyl (1R,3R,4S)-3-(ethylsulfonamido)-4-fluoro-1-(3-(5-fluoropyrimidin-2-yl)benzyl)cyclopentane-1-carboxylate (0.168 g).

A mixture of the obtained compound (0.168 g), 2 mol/L aqueous solution of sodium hydroxide (0.437 mL) and 2-propanol (4 mL) was stirred at 70 °C for 2 hours, and allowed to cool to room temperature. To the reaction mixture was added 2 mol/L hydrochloric acid. The mixture was extracted with DCM. The extract was dried over anhydrous sodium sulfate, and concentrated under reduced pressure.

To a mixture of the residue, N,O-dimethylhydroxylamine hydrochloride (0.128 g), DIPEA (0.282 g) and DMF (4 mL) was added HATU (0.183 g) at room temperature. The mixture was stirred at 80 °C for 1 hour, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=50/50-20/80), and then purified by column chromatography on ODS (eluent: water/MeCN=70/30-10/90) to give the title compound (0.041 g).

### Example 60

### (1R,3R,4S)-3-(Cyclopropanesulfonamido)-4-fluoro-1-(3-(5-fluoropyrimidin-2-yl)benzyl)-N-methoxy-N-methylcyclopentane-1-carboxamide

To a mixture of Reference Example I-8 (0.027 g), pyridine (0.014 g) and DCM (2 mL) was added Deoxo-Fluor (0.020 g) under ice-cooling. The mixture was stirred at the same temperature for 30 minutes, and then stirred at room temperature for 17 hours. To the reaction mixture were added a saturated aqueous solution of sodium bicarbonate and water. The mixture was extracted with DCM. The extract was dried over anhydrous sodium sulfate, and concentrated under reduced pressure. The residue was purified by column chromatography on silica gel (eluent: n-hexane/ethyl acetate=70/30-50/50) to give methyl (1R,3R,4S)-3-(cyclopropanesulfonamido)-4-fluoro-1-(3-(5-fluoropyrimidin-2-yl)benzyl)cyclopentane-1-carboxylate (0.023 g).

A mixture of the obtained compound (0.023 g), 2 mol/L aqueous solution of sodium hydroxide (0.060 mL) and 2-propanol (2 mL) was stirred at 70 °C for 2 hours, and allowed to cool to room temperature. To the reaction mixture was added 2 mol/L hydrochloric acid. The mixture was extracted with DCM. The extract was dried over anhydrous sodium sulfate, and concentrated under reduced pressure.

To a mixture of the residue, N,O-dimethylhydroxylamine hydrochloride (0.018 g), DIPEA (0.039 g) and DMF (2 mL) was added HATU (0.025 g) at room temperature. The mixture was stirred at 80 °C for 1 hour, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=50/50-20/80), and then purified by column chromatography on ODS (eluent: water/MeCN=70/30-10/90) to give the title compound (0.005 g).

### Example 61

### (1R,3R,4S)-3-(Ethylsulfonamido)-4-fluoro-1-(2-fluoro-5-(pyrimidin-2-yl)benzyl)-N-methoxy-N-methylcyclopentane-1-carboxamide

To a mixture of Reference Example I-12 (0.169 g), pyridine (0.078 g) and DCM (7 mL) was added Deoxo-Fluor (0.109 g) under ice-cooling. The mixture was stirred at the same temperature for 5 minutes, and then stirred at room temperature for 3 hours. To the reaction mixture were added a saturated aqueous solution of sodium bicarbonate and water. The mixture was extracted with DCM. The extract was dried over anhydrous sodium sulfate, and concentrated under reduced pressure. The residue was purified by column chromatography on silica gel (eluent: n-hexane/ethyl acetate=80/20-50/50) to give methyl (1R,3R,4S)-1-(5-(5-bromopyrimidin-2-yl)-2-fluorobenzyl)-3-(ethylsulfonamido)-4-fluorocyclopentane-1-carboxylate (0.126 g).

A mixture of the obtained compound (0.126 g), 2 mol/L aqueous solution of sodium hydroxide (0.327 mL) and 2-propanol (4 mL) was stirred at 70 °C for 2 hours, and allowed to cool to room temperature. To the reaction mixture was added 2 mol/L hydrochloric acid. The mixture was extracted with DCM. The extract was dried over anhydrous sodium sulfate, and concentrated under reduced pressure.

To a mixture of the residue, N,O-dimethylhydroxylamine hydrochloride (0.096 g), DIPEA (0.211 g) and DMF (4 mL) was added HATU (0.137 g) at room temperature. The mixture was stirred at 100 °C for 1.5 hours, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=70/30-30/70), and then purified by column chromatography on ODS (eluent: water/MeCN=70/30-10/90) to give (1R,3R,4S)-1-(5-(5-bromopyrimidin-2-yl)-2-fluorobenzyl)-3-(ethylsulfonamido)-4-fluoro-N-methoxy-N-methylcyclopentane-1-carboxamide (0.081 g).

A mixture of the obtained compound (0.073 g), TEA (0.020 g), ethanol (1.5 mL), THF (1.5 mL) and 10% Pd/C (0.014 g) was stirred under a hydrogen atmosphere at room temperature for 1 hour. The reaction mixture was filtered through a pad of Celite, and the filtrate was concentrated under reduced pressure. The residue was purified by column chromatography on silica gel (eluent: n-hexane/ethyl acetate=70/30-0/100) to give the title compound (0.055 g).

### Example 62

### (1R,3S,4R)-1-(4-Chloro-3-(5-fluoropyrimidin-2-yl)benzyl)-3-fluoro-N-methoxy-N-methyl-4-(methylsulfonamido)cyclopentane-1-carboxamide

To a mixture of Reference Example I-18 (0.237 g), pyridine (0.098 g) and DCM (7.6 mL) was added Deoxo-Fluor (0.137 g) under ice-cooling. The mixture was stirred at the same temperature for 30 minutes, and then stirred at room temperature for 1 hour. The reaction mixture was added to a saturated aqueous solution of sodium bicarbonate, and extracted with DCM. The extract was dried over anhydrous sodium sulfate, and concentrated under reduced pressure. The residue was purified by column chromatography on silica gel (eluent: n-hexane/ethyl acetate=100/0-50/50) to give methyl (1R,3S,4R)-1-(4-chloro-3-(5-fluoropyrimidin-2-yl)benzyl)-3-fluoro-4-(methylsulfonamido)cyclopentane-1-carboxylate (0.091 g).

A mixture of the obtained compound (0.091 g), 5 mol/L aqueous solution of sodium hydroxide (0.394 mL) and 1,4-dioxane (2 mL) was stirred at 80 °C for 1.5 hours, and allowed to cool to room temperature. To the reaction mixture was added 6 mol/L hydrochloric acid (0.394 mL). The mixture was extracted with DCM. The extract was dried over anhydrous sodium sulfate, and concentrated under reduced pressure.

To a mixture of the residue, N,O-dimethylhydroxylamine hydrochloride (0.058 g), DIPEA (0.172 g) and DMF (2 mL) was added HATU (0.083 g) at room temperature. The mixture was stirred at 100 °C for 1.5 hour, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=100/0-0/100), and then purified by column chromatography on ODS (eluent: water/MeCN=90/10-10/90) to give the title compound (0.033 g).

### Example 63

### (1R,3R,4S)-1-(4-Chloro-3-(5-fluoropyrimidin-2-yl)benzyl)-3-(ethylsulfonamido)-4-fluoro-N-methoxy-N-methylcyclopentane-1-carboxamide

To a mixture of Reference Example I-19 (0.251 g), pyridine (0.126 g) and DCM (2.5 mL) was added Deoxo-Fluor (0.177 g) under ice-cooling. The mixture was stirred at the same temperature for 30 minutes, and then stirred at room temperature for 30 minutes. To the reaction mixture were added a saturated aqueous solution of sodium bicarbonate and water. The mixture was extracted with DCM. The extract was dried over anhydrous sodium sulfate, and concentrated under reduced pressure. The residue was purified by column chromatography on silica gel (eluent: n-hexane/ethyl acetate=100/0-50/50) to give methyl (1R,3R,4S)-1-(4-chloro-3-(5-fluoropyrimidin-2-yl)benzyl)-3-(ethylsulfonamido)-4-fluorocyclopentane-1-carboxylate (0.200 g).

A mixture of the obtained compound (0.200 g), 5 mol/L aqueous solution of sodium hydroxide (0.571 mL) and 1,4-dioxane (2 mL) was stirred at 80 °C for 1.5 hours, and allowed to cool to room temperature. To the reaction mixture was added 6 mol/L hydrochloric acid (0.571 mL). The mixture was extracted with DCM. The extract was dried over anhydrous sodium sulfate, and concentrated under reduced pressure. To a mixture of the residue, N,O-dimethylhydroxylamine hydrochloride (0.083 g), DIPEA (0.184 g) and DMF (3 mL) was added HATU (0.119 g) at room temperature. The mixture was stirred at 100 °C for 1.5 hour, and allowed to cool to room temperature. To the reaction mixture was added water. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=100/0-0/100), and then purified by column chromatography on ODS (eluent: water/MeCN=90/10-10/90) to give the title compound (0.060 g).

### Example 64

### (1R,3R,4R)-3-Fluoro-1-(4-fluoro-3-(5-fluoropyrimidin-2-yl)benzyl)-N-methoxy-N-methyl-4-(methylsulfonamido)cyclopentane-1-carboxamide

To a mixture of the Reference Example I-22 (0.033 g), pyridine (0.017 g) and DCM (1 mL) was added Deoxo-Fluor (0.024 g) under ice-cooling, and the mixture was stirred at room temperature for 3 hours. To the reaction mixture were added a saturated aqueous solution of sodium bicarbonate and water under ice-cooling. The mixture was extracted with DCM. The extract was dried over anhydrous sodium sulfate, and concentrated under reduced pressure. The residue was purified by column chromatography on silica gel (eluent: n-hexane/ethyl acetate=100/0-40/60) to give methyl (1R,3R,4R)-3-fluoro-1-(4-fluoro-3-(5-fluoropyrimidin-2-yl)benzyl)-4-(methylsulfonamido)cyclopentane-1-carboxylate (0.023 g).

A mixture of the obtained compound (0.023 g), 6 mol/L hydrochloric acid (1 mL) and 1,4-dioxane (1 mL) was stirred at 100 °C for 8 hours, and allowed to cool to room temperature. To the reaction mixture was added water. The mixture was extracted with DCM. The extract was dried over anhydrous sodium sulfate, and concentrated under reduced pressure.

To a mixture of the residue, DMF (0.4 mg) and DCM (1 mL) was added oxalyl chloride (0.010 g) under ice-cooling, and the mixture was stirred at room temperature for 30 minutes. The reaction mixture was concentrated under reduced pressure. A mixture of the residue and DCM (0.5 mL) was added to a mixture of N,O-dimethylhydroxylamine hydrochloride (0.010 g), DIPEA (0.020 g) and DCM (0.5 mL) under ice-cooling. The mixture was stirred at room temperature for 15 minutes, and concentrated under reduced pressure. The residue was purified by column chromatography on silica gel (eluent: n-hexane/ethyl acetate=100/0-70/30) to give the title compound (0.011 g).

### Example 65

### (1R,4R)-3,3-Difluoro-N-methoxy-N-methyl-4-(methylsulfonamido)-1-(3-(pyrimidin-2-yl)benzyl)cyclopentane-1-carboxamide

To a mixture of Reference Example J-1 (0.153 g), TEA (0.031 g) and ethanol (3 mL) was added 10% Pd/C (0.046 g) at room temperature. The mixture was stirred under a hydrogen atmosphere at the same temperature for 1 hour. The reaction mixture was filtered through a pad of Celite, and the filtrate was concentrated under reduced pressure. To the residue were added a saturated aqueous solution of sodium bicarbonate and DCM, and the mixture was extracted with DCM. The extract was dried over anhydrous sodium sulfate, and concentrated under reduced pressure.

A mixture of the residue, 5 mol/L aqueous solution of sodium hydroxide (0.310 mL), THF (0.5 mL) and methanol (1 mL) was stirred at 110 °C under microwave irradiation for 1 hour. To the reaction mixture were added 6 mol/L hydrochloric acid (0.259 mL) and water. The mixture was extracted with DCM. The extract was dried over anhydrous sodium sulfate, and concentrated under reduced pressure.

A mixture of the residue, DIPEA (0.201 g), HATU (0.154 g), and NMP (3 mL) was stirred at 90 °C under microwave irradiation for 30 minutes. To the reaction mixture were added N,O-dimethylhydroxylamine hydrochloride (0.121 g) and DIPEA (0.201 g) at room temperature, and the mixture was stirred at 90 °C under microwave irradiation for 1 hour. To the reaction mixture were added N,O-dimethylhydroxylamine hydrochloride (0.121 g) and DIPEA (0.161 g) at room temperature, and the mixture was stirred at 90 °C under microwave irradiation for 1 hour. The reaction mixture was added to water, and extracted with ethyl acetate. The extract was dried over anhydrous sodium sulfate, and concentrated under reduced pressure. The residue was purified by column chromatography on silica gel (eluent: n-hexane/ethyl acetate=100/0-0/100), and then purified by column chromatography on ODS (eluent: water/MeCN=70/30-10/90) to give the title compound (0.020 g).

### Example 66

### (1R,4R)-3,3-Difluoro-1-(3-(5-fluoropyrimidin-2-yl)benzyl)-N-methoxy-N-methyl-4-(methylsulfonamido)cyclopentane-1-carboxamide

A mixture of Reference Example J-3 (0.665 g), 2-bromo-5-fluoropyrimidine (0.497 g), sodium carbonate (0.447 g), Pd(dppf)Cl₂ DCM adduct (0.115 g), 1,4-dioxane (5 mL), ethanol (2.5 mL) and water (2.5 mL) was stirred at 90 °C under microwave irradiation for 1 hour. The mixture was filtered through a pad of Celite, and the filtrate was concentrated under reduced pressure. The residue was purified by column chromatography on silica gel (eluent: n-hexane/ethyl acetate=100/0-50/50) to give methyl (1R,4R)-3,3-difluoro-1-(3-(5-fluoropyrimidin-2-yl)benzyl)-4-(methylsulfonamido)cyclopentane-1-carboxylate (0.703 g) as a mixture with (1S,4R)-isomer.

A mixture of the obtained compound (0.703 g), 2 mol/L aqueous solution of sodium hydroxide (3.57 mL) and 2-propanol (20 mL) was stirred at 60 °C for 1 hour, and allowed to cool to room temperature. To the reaction mixture were added 2 mol/L hydrochloric acid (3.96 mL), water and brine. The mixture was extracted with DCM. The extract was dried over anhydrous sodium sulfate, and concentrated under reduced pressure.

A mixture of the residue, DIPEA (1.05 g), HATU (0.805 g) and DMF (1 mL) was stirred at 60 °C for 40 minutes. To the reaction mixture was added N,O-dimethylhydroxylamine hydrochloride (0.635 g) at room temperature. The mixture was stirred at 60 °C for 40 minutes, and allowed to cool to room temperature. The reaction mixture was added to water, and extracted with ethyl acetate. The extract was dried over anhydrous sodium sulfate, and concentrated under reduced pressure. The residue was purified by column chromatography on silica gel (eluent: n-hexane/ethyl acetate=100/0-0/100), and then purified by chiral preparative column chromatography (eluent: n-hexane/ethanol=70/30) to give the title compound (0.124 g).

### Example 67

### (1R,3S,4R)-1-(3-(5-Fluoropyrimidin-2-yl)benzyl)-N,3-dimethoxy-N-methyl-4-(methylsulfonamido)cyclopentane-1-carboxamide

A mixture of Reference Example I-23 (0.021 g), 2 mol/L aqueous solution of sodium hydroxide (0.190 mL), THF (0.5 mL) and 2-propanol (0.5 mL) was stirred at 60 °C for 2 hours, and allowed to cool to room temperature. To the reaction mixture were added 2 mol/L hydrochloric acid (0.228 mL), water and brine. The mixture was extracted with DCM. The extract was dried over anhydrous sodium sulfate, and concentrated under reduced pressure.

To a mixture of the residue, DMF (0.2 mg) and DCM (1 mL) was added oxalyl chloride (0.006 g) under ice-cooling, and the mixture was stirred at room temperature for 30 minutes. The reaction mixture was concentrated under reduced pressure. A mixture of the residue and DCM (0.5 mL) was added to a mixture of N,O-dimethylhydroxylamine hydrochloride (0.006 g), DIPEA (0.012 g) and DCM (0.5 mL) under ice-cooling. The mixture was stirred at room temperature for 15 minutes, and concentrated under reduced pressure. The residue was purified by column chromatography on silica gel (eluent: n-hexane/ethyl acetate=100/0-70/30) to give (1R,3S,4R)-1-(3-(5-fluoropyrimidin-2-yl)benzyl)-N,3-dimethoxy-4-(N-(4-methoxybenzyl)methylsulfonamido)-N-methylcyclopentane-1-carboxamide (0.013 g).

A mixture of the obtained compound (0.013 g), triethylsilane (0.008 g), TFA (1.5 mL) and DCM (0.5 mL) was stirred at 40 °C for 30 minutes. The reaction mixture was allowed to cool to room temperature, and then concentrated under reduced pressure. The residue was purified by column chromatography on silica gel (eluent: n-hexane/ethyl acetate=100/0-20/80) to give the title compound (0.009 g).

### Example 68

Example 68 was synthesized in a manner similar to that described in Example 67 using Reference Example I-24 instead of Reference Example I-23.

### Example 69 and Example 70

Example 69 and Example 70 were synthesized in a manner similar to that described in Example 1 using the corresponding reagents instead of ethylamine hydrochloride.

### Example 71

### N-((1S*,3R*)-3-([1,1'-Biphenyl]-3-ylmethyl)-3-(2-(trifluoromethyl)pyrrolidine-1-carbonyl)cyclopentyl)methanesulfonamide

To a mixture of Reference Example A-2 (0.011 g) and DCM (1 mL) were added oxalyl chloride (0.007 g) and DMF (4.7 mg) at room temperature, and the mixture was stirred at the same temperature for 30 minutes. To the reaction mixture were added 2-(trifluoromethyl)pyrrolidine hydrochloride (0.010 g) and DIPEA (0.074 g) at room temperature, and the mixture was stirred at the same temperature for 30 minutes. To the reaction mixture was added water. The mixture was extracted with DCM, and the extract was concentrated under reduced pressure. The residue was purified by column chromatography on silica gel (eluent: n-hexane/ethyl acetate=50/50-0/100) to give the title compound (0.007 g).

### Example 72

### (1R*,3S*)-1-([1,1'-Biphenyl]-3-ylmethyl)-3-(methylsulfonamido)cyclopentane-1-carboxamide

To a mixture of Reference Example A-2 (0.035 g), ammonium chloride (0.025 g), DIPEA (0.097 g) and DMF (1.5 mL) was added HATU (0.039 g) at room temperature, and the mixture was stirred at the same temperature for 30 minutes. To the reaction mixture were added DIPEA (0.048 g), ammonium chloride (0.013 g) and HATU (0.018 g) at room temperature, and the mixture was stirred at the same temperature for 30 minutes. To the reaction mixture were added water and ethyl acetate. The mixture was extracted with ethyl acetate. The extract was washed with water and brine, dried over anhydrous sodium sulfate, and concentrated under reduced pressure. The residue was purified by column chromatography on silica gel (eluent: n-hexane/ethyl acetate=20/80-0/100), and then purified by column chromatography on APS (eluent: ethyl acetate) to give the title compound (0.029 g).

### Example 73

### (1R*,3S*)-1-([1,1'-Biphenyl]-3-ylmethyl)-N-(2-hydroxyethyl)-3-(methylsulfonamido)cyclopentane-1-carboxamide

A mixture of Reference Example A-2 (0.030 g), 2-(benzyloxy)-1-ethanamine hydrochloride (0.030 g), DIPEA (0.031 g), 1-hydroxybenzotriazole monohydrate (0.018 g), 1-ethyl-3-(3-dimethylaminopropyl)carbodiimide hydrochloride (0.023 g) and DMF (1.0 mL) was stirred at 50 °C for 13 hours, and allowed to cool to room temperature. To the reaction mixture was added water. The mixture was extracted with a mixed solvent of n-hexane/ethyl acetate (3/1). The extract was washed with water and brine, dried over anhydrous magnesium sulfate, and concentrated under reduced pressure. The residue was purified by column chromatography on silica gel (eluent: ethyl acetate/methanol=100/0-90/10) to give (1R*,3S*)-1-([1,1'-biphenyl]-3-ylmethyl)-N-(2-(benzyloxy)ethyl)-3-(methylsulfonamido)cyclopentane-1-carboxamide (0.033 g).

To a mixture of the obtained compound (0.030 g) and ethanol (1.0 mL) was added 10% Pd/C (0.006 g) under ice-cooling. The mixture was stirred under a hydrogen atmosphere at room temperature for 6 hours. The reaction mixture was filtered through a pad of Celite, and the filtrate was concentrated under reduced pressure. The residue was purified by column chromatography on silica gel (eluent: ethyl acetate/methanol=100/0-90/10) to give the title compound (0.019 g).

### Example 74

### (1R*,3S*)-1-([1,1'-Biphenyl]-3-ylmethyl)-3-(methylsulfonamido)-N-(2,2,2-trifluoroethyl)cyclopentane-1-carboxamide

To a mixture of 2,2,2-trifluoroethylamine (0.011 g), TEA (0.047 g) and DCM (0.5 mL) was added phosphoryl chloride (0.016 g) under ice-cooling. To the mixture was added a mixture of Reference Example A-2 (0.027 g) and DCM (0.5 mL) under ice-cooling. The reaction mixture was stirred at room temperature for 3 hours. To the reaction mixture was added water. The mixture was extracted with DCM, and the extract was concentrated under reduced pressure. The residue was purified by column chromatography on silica gel (eluent: n-hexane/ethyl acetate=40/60-20/80) to give the title compound (0.010 g).

### Example 75

Examples 75 was synthesized in a manner similar to that described in Example 71 using 3,3-difluoropyrrolidine hydrochloride instead of 2-(trifluoromethyl)pyrrolidine hydrochloride.

The following tables show structure, physical property and OX2R agonist activity (see, Test Example 1) of Examples.

**[Table 15]**

| Ex. No. | Str. | Phys. data | Act. (%) |
|---|---|---|---|
| 1 | 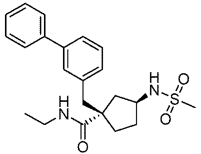 | MS m/z : 401 (M+H)⁺ | 50 |
| 2 | 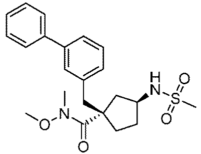 | MS m/z : 417 (M+H)⁺ | 84 |
| 3 | 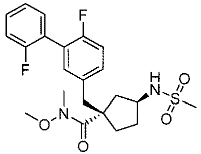 | MS m/z : 453 (M+H)⁺ | 93 |
| 4 | 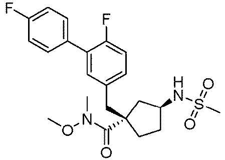 | MS m/z : 453 (M+H)⁺ | 82 |
| 5 | 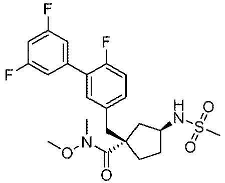 | MS m/z : 471 (M+H)⁺ | 73 |
| 6 | 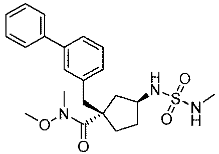 | MS m/z : 432 (M+H)⁺ | 92 |

**[Table 16]**

| Ex. No. | Str. | Phys. data | Act. (%) |
|---|---|---|---|
| 7 | 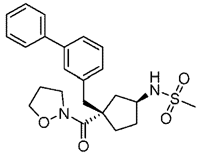 | MS m/z : 429 (M+H)⁺ | 70 |
| 8 | 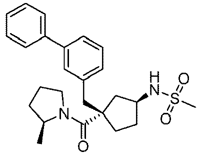 | MS m/z : 441 (M+H)⁺ | 75 |
| 9 | 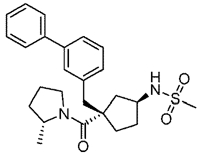 | MS m/z : 441 (M+H)⁺ | 58 |
| 10 | 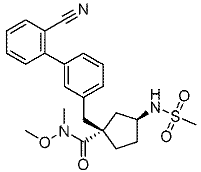 | MS m/z : 442 (M+H)⁺ | 41 |
| 11 | 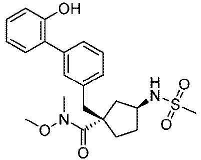 | MS m/z : 433 (M+H)⁺ | 71 |
| 12 | 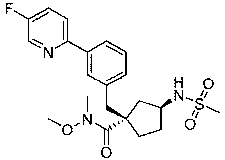 | MS m/z : 436 (M+H)⁺ | 86 |

**[Table 17]**

| Ex. No. | Str. | Phys. data | Act. (%) |
|---|---|---|---|
| 13 | 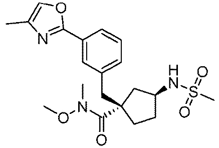 | MS m/z : 422 (M+H)⁺ | 90 |
| 14 | 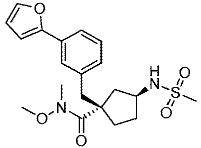 | MS m/z : 407 (M+H)⁺ | 93 |
| 15 | 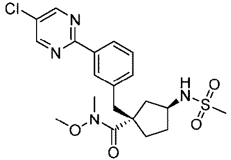 | MS m/z : 453 (M+H)⁺ | 86 |
| 16 | 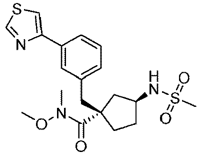 | MS m/z : 424 (M+H)⁺ | 91 |
| 17 | 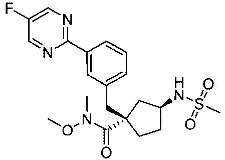 | ¹H-NMR (CDCl₃) δ ppm : 1. 35-1. 50 (1H, m), 1. 54-1. 75 (1H, m), 1. 82-2. 34 (3H, m), 2.69-2.88 (1H, m), 2.93 (3H, s), 3.09 (1H, d, J=13.6Hz), 3.17-3.35 (4H, m), 3.64-3.89 (4H, m), 4.49 (1H, d, J=7.8Hz), 7.17-7.31 (1H, m), 7.34-7.51 (m, 1H), 8.09-8. 19 (1H, m), 8.20-8.30 (1H, m), 8. 65 (2H, s) MS m/z : 437 (M+H)⁺ | 99 |
| 18 | 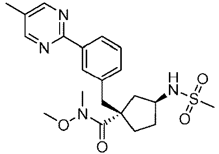 | ¹H-NMR (CDCl₃) δ ppm : 1.18-1.44 (1H, m), 1.61-1.79 (1H, m), 1.83-1.98 (1H, m), 1.98-2. 20 (2H, m), 2.34 (3H, s), 2. 73-2. 86 (1H, m), 2.92 (3H, s), 3.07 (1H, d, J=13.6 Hz), 3. 19-3. 33 (4H, m), 3.64-3.86 (4H, m), 4.61 (1H, d, J=8.0Hz), 7.15-7.24 (1H, m), 7. 33-7.46 (1H, m), 8.12-8.21 (1H, m), 8.21-8.30 (1H, m), 8. 58-8. 72 (2H, m) MS m/z : 433 (M+H)⁺ | 87 |

**[Table 18]**

| Ex. No. | Str. | Phys. data | Act. (%) |
|---|---|---|---|
| 19 | 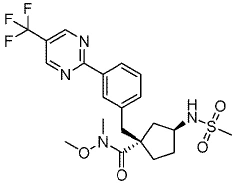 | MS m/z : 487 (M+H)⁺ | 45 |
| 20 | 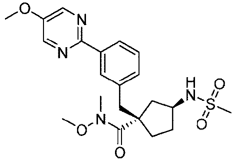 | MS m/z : 449 (M+H)⁺ | 75 |
| 21 | 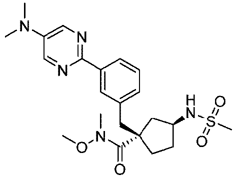 | MS m/z : 462 (M+H)⁺ | 48 |
| 22 | 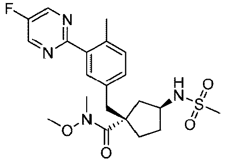 | MS m/z : 451 (M+H)⁺ | 87 |
| 23 | 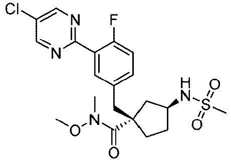 | MS m/z : 471 (M+H)⁺ | 83 |
| 24 | 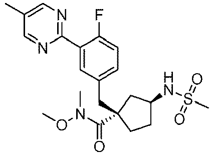 | MS m/z : 451 (M+H)⁺ | 83 |

**[Table 19]**

| Ex. No. | Str. | Phys. data | Act. (%) |
|---|---|---|---|
| 25 | 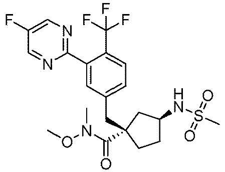 | MS m/z : 505 (M+H)⁺ | 63 |
| 26 | 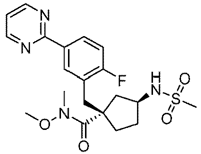 | MS m/z : 437 (M+H)⁺ | 99 |
| 27 | 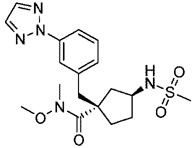 | MS m/z : 408 (M+H)⁺ | 87 |
| 28 | 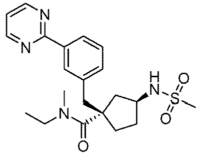 | MS m/z : 417 (M+H)⁺ | 92 |
| 29 | 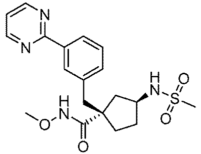 | MS m/z : 405 (M+H)⁺ | 47 |
| 30 | 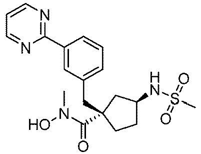 | MS m/z : 405 (M+H)⁺ | 48 |

**[Table 20]**

| Ex. No. | Str. | Phys. data | Act. (%) |
|---|---|---|---|
| 31 | 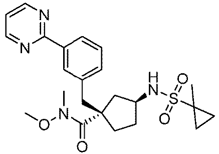 | MS m/z : 459 (M+H)⁺ | 107 |
| 32 | 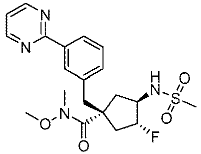 | ¹H-NMR (CDCl₃) δ ppm :1.76-1. 87 (1H, m), 2. 16-2. 29 (1H, m), 2. 40-2. 55 (1H, m), 2. 89 (3H, s), 2. 97 (1H, d, J=13.4Hz), 3.00-3.10 (1H, m), 3.23 (1H, d, J=13.4Hz), 3.27 (3H, s), 3.76-3.95 (4H, m), 4.12-4.32 (1H, m), 5.10 (1H, d, J=8. 3Hz), 7. 19-7. 31 (2H, m), 7.42-7.51 (1H, m), 8. 18-8. 24 (1H, m), 8.25-8.33 (1H, m), 8.81-8.89 (2H, m) | 89 |
| | | MS m/z : 437 (M+H)⁺ | |
| 33 | 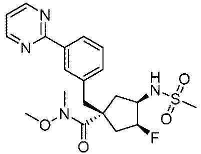 | ¹H-NMR (CDCl₃) δ ppm :1.73-1. 89 (1H, m), 2. 14-2. 34 (1H, m), 2. 39-2. 63 (1H, m), 2. 75-2. 90 (1H, m), 2. 97 (3H, s), 3. 19 (1H, d, J=13.7Hz), 3.28 (3H, s), 3. 42 (1H, d, J=13.7Hz), 3. 60-3. 88 (4H, m), 4.65 (1H, d, J=9. 6Hz), 4. 88-5. 15 (1H, m), 7.13-7.23 (2H, m), 7. 33-7. 44 (1H, m), 8. 15-8. 24 (1H, m), 8. 26-8. 35 (1H, m), 8. 73-8. 85 (2H, m) | 83 |
| | | MS m/z : 437 (M+H)⁺ | |
| 34 | 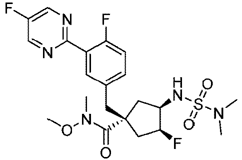 | ¹H-NMR (CDCl₃) δ ppm :1.67-1. 83 (1H, m), 2. 11-2. 31 (1H, m), 2. 32-2. 57 (1H, m), 2.62-2.89 (7H, m), 3.14 (1H, d, J=13.9Hz), 3.25 (3H, s), 3.35 (1H, d, J=13.9Hz), 3.51-3.76 (1H, m), 3.81 (3H, s), 4.49 (1H, d, J=9.8Hz), 4.92-5.12 (1H, m), 7.04-7.21 (2H, m), 7.73-7.83 (1H, m), 8. 72 (2H, s) | 90 |
| | | MS m/z : 502 (M+H)⁺ | |
| 35 | 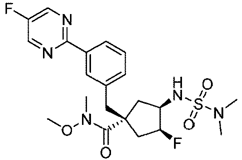 | MS m/z : 484 (M+H)⁺ | 92 |
| 36 | 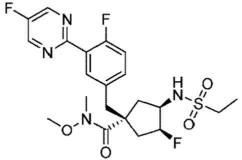 | MS m/z : 487 (M+H)⁺ | 84 |

**[Table 21]**

| Ex. No. | Str. | Phys. data | Act. (%) |
|---|---|---|---|
| 37 | 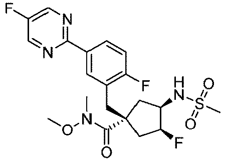 | MS m/z : 473 (M+H)⁺ | 90 |
| 38 | 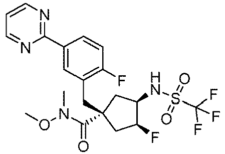 | MS m/z : 509 (M+H)⁺ | 93 |
| 39 | 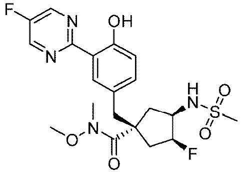 | ¹H-NMR (CDCl₃) δ ppm : 1.70-1. 87 (1H, m), 2. 11-2. 32 (1H, m), 2.33-2.60 (1H, m), 2.73-2.90 (1H, m), 2.98 (3H, s), 3.08 (1H, d, J=13.8Hz), 3.27 (3H, s), 3. 30 (1H, d, J=13.8Hz), 3.53-3.94 (4H, m), 4.67 (1H, d, J=9. 1Hz), 4.86-5.17 (1H, m), 6.93 (1H, d, J-8. 4Hz), 7.10 (1H, dd, J=2.3, 8. 4Hz), 8.14 (1H, d, J=2.3Hz), 8.66 (2H, s), 12.25 (1H, s) | 91 |
| | | MS m/z : 471 (M+H)⁺ | |
| 40 | 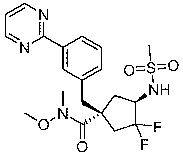 | ¹H-NMR (CDCl₃) δ ppm : 1.66-1. 81 (1H, m), 2. 25-2.47 (1H, m), 2. 72-2.98 (2H, m), 3.01 (3H, s), 3.13-3.35 (5H, m), 3. 83 (3H, s), 3.89-4.09 (1H, m), 4.61 (1H, d, J=9. 4Hz), 7. 10-7. 23 (2H, m), 7. 35-7.45 (1H, m), 8.14-8.22 (1H, m), 8.28-8.38 (1H, m), 8.80 (2H, d, J=4.8Hz) | 88 |
| | | MS m/z : 455 (M+H)⁺ | |
| 41 | 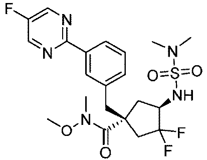 | MS m/z : 502 (M+H)⁺ | 93 |
| 42 | 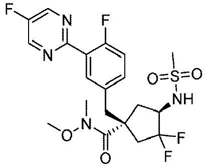 | MS m/z : 491 (M+H)⁺ | 87 |

**[Table 22]**

| Ex. No. | Str. | Phys. data | Act. (%) |
|---|---|---|---|
| 43 | 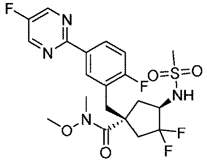 | MS m/z : 491 (M+H)⁺ | 93 |
| 44 | 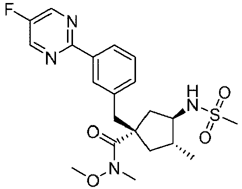 | ¹H-NMR (CDCl₃) δ ppm : 1.04 (3H, d, J=6. 4Hz), 1. 39-1. 80 (3H, m), 2.07-2.19 (1H, m), 2. 86-2. 98 (4H, m), 3.03 (1H, d, J=13.5Hz), 3.12-3.33 (5H, m), 3. 80 (3H, s), 4.48 (1H, d, J=9. 1Hz), 7.17-7.24 (1H, m), 7.35-7.43 (1H, m), 8. 10-8. 16 (1H, m), 8. 19-8. 26 (1H, m), 8. 66 (2H, s) | 96 |
| | | MS m/z : 451 (M+H)⁺ | |
| 45 | 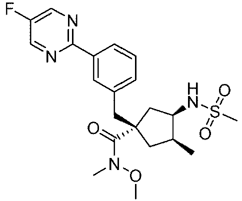 | ¹H-NMR (CDCl₃) δ ppm : 0.98 (3H, d, J=7.0Hz), 1.39-1.50 (1H, m), 1.75-1.86 (1H, m), 2.10-2.26 (1H, m), 2.49-2.60 (1H, m), 2. 61-2. 72 (1H, m), 2.91 (3H, s), 3. 14 (1H, d, J=13.5Hz), 3. 20-3. 32 (4H, m), 3.67-3.78 (1H, m), 3.81 (3H, s), 4.19 (1H, d, J=8.8Hz), 7.16-7.25 (1H, m), 7. 35-7. 44 (1H, m), 8. 10-8. 17 (1H, m), 8. 21-8. 29 (1H, m), 8.65 (2H, s) | 93 |
| | | MS m/z : 451 (M+H)⁺ | |
| 46 | 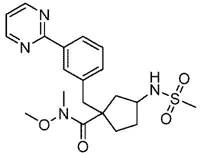 | ¹H-NMR (CDCl₃) δ ppm : 1. 20-1. 42 (1H, m), 1. 62-1. 73 (1H, m), 1. 84-2. 00 (1H, m), 2.00-2. 22 (2H, m), 2. 73-2. 88 (1H, m), 2.92 (3H, s), 3.09 (1H, d, J=13.6Hz), 3. 22-3. 34 (4H, m), 3.69-3.85 (4H, m), 4.48 (1H, d, J=8.0Hz), 7. 17-7.25 (2H, m), 7.38-7.45 (1H, m), 8.18-8.24 (1H, m), 8. 26-8. 33 (1H, m), 8.78-8.86 (2H, m) | 93 |
| | | MS m/z : 419 (M+H)⁺ | |
| 47 | 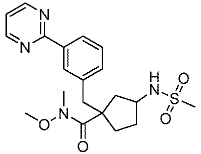 | ¹H-NMR (CDCl₃) δ ppm : 1.23-1.42 (1H, m), 1. 62-1. 74 (1H, m), 1. 88-2. 00 (1H, m), 2.00-2.21 (2H, m), 2.75-2.87 (1H, m), 2.92 (3H, s), 3.09 (1H, d, J=13.6Hz), 3. 22-3. 34 (4H, m), 3.69-3.85 (4H, m), 4.48 (1H, d, J=8.0Hz), 7.17-7. 25 (2H, m), 7.38-7.45 (1H, m), 8.18-8.24 (1H, m), 8. 26-8. 33 (1H, m), 8. 78-8.86 (2H, m) | 52 |
| | | MS m/z : 419 (M+H)⁺ | |

**[Table 23]**

| Ex. No. | Str. | Phys. data | Act. (%) |
|---|---|---|---|
| 48 | 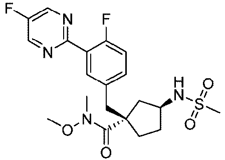 | ¹H-NMR (CDCl₃) δ ppm : 1. 16-1. 37 (1H, m), 1. 53-1. 72 (1H, m), 1. 78-1. 96 (1H, m), 1.98-2.24 (2H, m), 2. 72-2. 87 (1H, m), 2.94 (3H, s), 3.04 (1H, d, J=13.6Hz), 3. 16-3. 30 (4H, m), 3. 66-3. 88 (4H, m), 4.63 (1H, d, J=8. 1Hz), 7.03-7. 24 (2H, m), 7.72-7.88 (1H, m), 8. 75 (2H, s) | 101 |
| | | MS m/z : 455 (M+H)⁺ | |
| 49 | 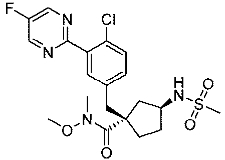 | ¹H-NMR (CDCl₃) δ ppm : 0.98-1.20 (1H, m), 1.44-1.74 (1H, m), 1.77-1.92 (1H, m), 1.93-2.06 (1H, m), 2.07-2.21 (1H, m), 2.76-2.89 (1H, m), 2.93 (3H, s), 3.01 (1H, d, J=13.5Hz), 3.14-3.28 (4H, m), 3.67-3.86 (4H, m), 4.85 (1H, d, J=8.3Hz), 7.15 (1H, dd, J=2.2, 8.2Hz), 7. 43 (1H, d, J=8. 2Hz), 7. 55 (1H, d, J=2.2Hz), 8.77 (2H, s) | 88 |
| | | MS m/z : 471 (M+H)⁺ | |
| 50 | 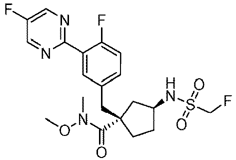 | ¹H-NMR (CDCl₃) δ ppm : 1.03-1.21 (1H, m), 1. 69-1. 81 (1H, m), 1. 82-1. 92 (1H, m), 1. 93-2. 20 (2H, m), 2. 76-2. 93 (1H, m), 3.03 (1H, d, J=13.5Hz), 3. 19 (1H, d, J=13.5Hz), 3. 23 (3H, s), 3.69-3.92 (4H, m), 5.05 (2H, d, J=47.0Hz), 5. 28 (1H, d, J=8. 6Hz), 7.09-7.24 (2H, m), 7.76-7.85 (1H, m), 8.76 (2H, s) | 110 |
| | | MS m/z : 473 (M+H)⁺ | |
| 51 | 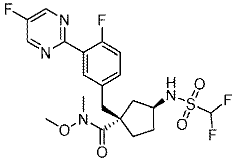 | ¹H-NMR (CDCl₃) δ ppm : 0.76-0.96 (1H, m), 1. 76-2. 00 (3H, m), 2. 03-2. 17 (1H, m), 2.82-2.96 (1H, m), 3.01 (1H, d, J=13.5Hz), 3.17 (1H, d, J=13.5Hz), 3.23 (3H, s), 3. 77 (3H, s), 3.86-4.03 (1H, m), 5.82 (1H, d, J=9. 3Hz), 6. 15 (1H, t, J=54. 2Hz), 7.08-7.33 (2H, m), 7.75-7.90 (1H, m), 8. 77 (2H, s) | 98 |
| | | MS m/z : 491 (M+H)⁺ | |
| 52 | 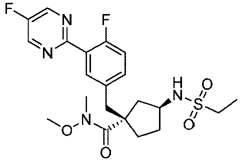 | ¹H-NMR (CDCl₃) δ ppm : 1. 19-1.38 (4H, m), 1.63-1.70 (1H, m), 1.81-1.93 (1H, m), 1.98-2.19 (2H, m), 2.73-2.87 (1H, m), 2.90-3.10 (3H, m), 3.13-3.29 (4H, m), 3.64-3.84 (4H, m), 4.58 (1H, d, J=8.4Hz), 7.05-7.25 (2H, m), 7.72-7.87 (1H, m), 8.75 (2H, s) | 99 |
| | | MS m/z : 469 (M+H)⁺ | |
| 53 | 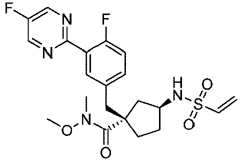 | ¹H-NMR (CDCl₃) δ ppm : 1. 18-1. 37 (1H, m), 1.58-1.73 (1H, m), 1. 80-1. 92 (1H, m), 1.93-2. 19 (2H, m), 2. 69-2. 84 (1H, m), 3.04 (1H, d, J=13.5Hz), 3.09-3. 32 (4H, m), 3.51-3.73 (1H, m), 3.77 (3H, s), 4.71 (1H, d, J=7.6Hz), 5.90 (1H, d, J=9. 9Hz), 6.23 (1H, d, J=16.5Hz), 6.50 (1H, dd, J=9.9, 16.5Hz), 7.05-7.23 (2H, m), 7.73-7.86 (1H, m), 8. 75 (2H, s) | 106 |
| | | MS m/z : 467 (M+H)⁺ | |

**[Table 24]**

| Ex. No. | Str. | Phys. data | Act. (%) |
|---|---|---|---|
| 54 | 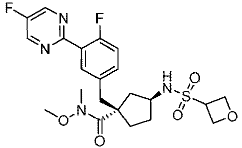 | MS m/z : 497 (M+H)⁺ | 90 |
| 55 | 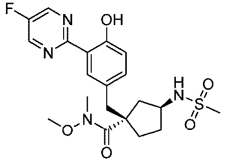 | MS m/z : 453 (M+H)⁺ | 106 |
| 56 | 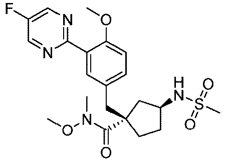 | MS m/z : 467 (M+H)⁺ | 51 |
| 57 | 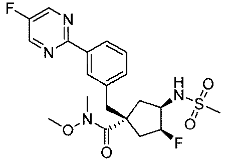 | ¹H-NMR (CDCl₃) δ ppm : 1.73-1.87 (1H, m), 2. 16-2. 33 (1H, m), 2.39-2.62 (1H, m), 2. 75-2. 90 (1H, m), 2.97 (3H, s), 3. 18 (1H, d, J=13.6Hz), 3.27 (3H, s), 3.41 (1H, d, J=13.6Hz), 3.62-3.90 (4H, m), 4. 66 (1H, d, J=9. 8Hz), 4.90-5. 15 (1H, m), 7. 14-7. 24 (1H, m), 7.34-7.44 (1H, m), 8.06-8.16 (1H, m), 8.20-8.30 (1H, m), 8. 64 (2H, s) | 92 |
| | | MS m/z : 455 (M+H)⁺ | |
| 58 | 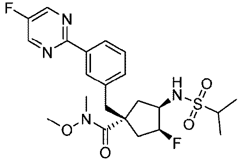 | MS m/z : 483 (M+H)⁺ | 95 |
| 59 | 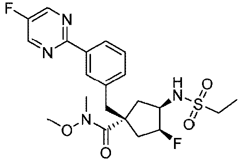 | ¹H-NMR (CDCl₃) δ ppm : 1. 36 (3H, t, J=7.4Hz), 1. 75-1. 87 (1H, m), 2.15-2.32 (1H, m), 2.38-2.60 (1H, m), 2.74-2.87 (1H, m), 3.03 (2H, q, J=7.4Hz), 3. 18 (1H, d, J=13.6Hz), 3.27 (3H, s), 3.41 (1H, d, J=13.6Hz), 3.59-3.79 (1H, m), 3.83 (3H, s), 4.54 (1H, d, J=9. 6Hz), 4.87-5.11 (1H, m), 7.13-7.23 (1H, m), 7.33-7.43 (1H, m), 8.08-8.16 (1H, m), 8. 19-8. 29 (1H, m), 8.64 (2H, s) | 98 |
| | | MS m/z : 469 (M+H)⁺ | |

**[Table 25]**

| Ex. No. | Str. | Phys. data | Act. (%) |
|---|---|---|---|
| 60 | 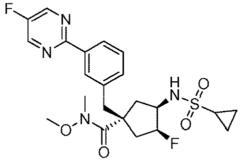 | MS m/z : 481 (M+H)⁺ | 94 |
| 61 | 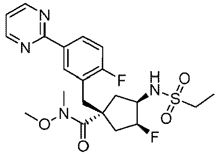 | ¹H-NMR (CDCl₃) δ ppm : 1.35 (3H, t, J=7.4Hz), 1.79-1.92 (1H, m), 2.19-2.36 (1H, m), 2. 39-2. 59 (1H, m), 2. 77-2. 89 (1H, m), 3.03 (2H, q, J=7. 4Hz), 3. 22 (1H, d, J=13.7Hz), 3.28 (3H, s), 3.44 (1H, d, J=13.7Hz), 3.58-3.77 (1H, m), 3.83 (3H, s), 4.57 (1H, d, J=9.8Hz), 4.88-5.09 (1H, m), 7.07-7.15 (1H, m), 7.18 (1H, t, J=4. 9Hz), 8.18-8.26 (1H, m), 8.27-8.36 (1H, m), 8.77 (2H, d, J=4.9Hz) | 105 |
| | | MS m/z : 469 (M+H)⁺ | |
| 62 | 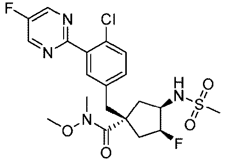 | ¹H-NMR (CDCl₃) δ ppm : 1.67-1.82 (1H, m), 2. 10-2. 29 (1H, m), 2.37-2.60 (1H, m), 2. 76-2. 88 (1H, m), 2.98 (3H, s), 3.12 (1H, d, J=13.8Hz), 3. 23 (3H, s), 3.34 (1H, d, J=13.8Hz), 3.61-3.86 (4H, m), 4. 70 (1H, d, J=9. 6Hz), 4.90-5.11 (1H, m), 7.09 (1H, dd, J=2. 2, 8.3Hz), 7.39 (1H, d, J=8.3Hz), 7.47 (1H, d, J=2. 2Hz), 8. 73 (2H, s) | 96 |
| | | MS m/z : 489 (M+H)⁺ | |
| 63 | 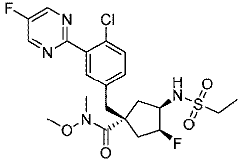 | ¹H-NMR (CDCl₃) δ ppm : 1.36 (3H, t, J=7.4Hz), 1.64-1.81 (1H, m), 2.11-2.27 (1H, m), 2.37-2.58 (1H, m), 2.75-2.87 (1H, m), 3.04 (2H, q, J=7. 4Hz), 3.12 (1H, d, J=13.8Hz), 3.22 (3H, s), 3.34 (1H, d, J=13.8Hz), 3.58-3.83 (4H, m), 4.61 (1H, d, J=9. 6Hz), 4.86-5.09 (1H, m), 7.09 (1H, dd, J=2.2, 8. 3Hz), 7.39 (1H, d, J=8.3Hz), 7.47 (1H, d, J=2.2Hz), 8.73 (2H, s) | 96 |
| | | MS m/z : 503 (M+H)⁺ | |
| 64 | 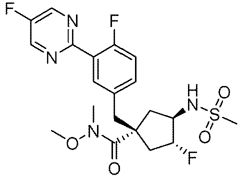 | ¹H-NMR (CDCl₃) δ ppm : 1.70-1.84 (1H, m), 2. 11-2.27 (1H, m), 2. 36-2. 56 (1H, m), 2.85-2. 97 (4H, m), 2.98-3.10 (1H, m), 3.19 (1H, d, J=13.5Hz), 3.25 (3H, s), 3. 72-3. 96 (4H, m), 4.02-4. 29 (1H, m), 5.43 (1H, d, J=8. 5Hz), 7.12-7.26 (2H, m), 7.75-7.84 (1H, m), 8.79 (2H, s) | 101 |
| | | MS m/z : 473 (M+H)⁺ | |

**[Table 26]**

| Ex. No. | Str. | Phys. data | Act. (%) |
|---|---|---|---|
| 65 | 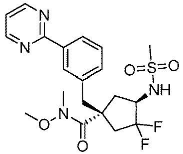 | ¹H-NMR (CDCl₃) δ ppm :1.55-1. 81 (1H, m), 2.29-2.46 (1H, m), 2.74-2.98 (2H, m), 3.01 (3H, s), 3.18 (1H, d, J=13.6Hz), 3. 26 (1H, d, J=13.6Hz), 3.29 (3H, s), 3.83 (3H, s), 3.89-4.09 (1H, m), 4.59-4.86 (1H, m), 7.13-7.24 (2H, m), 7.35-7.45 (1H, m), 8. 14-8.20 (1H, m), 8.29-8.37 (1H, m), 8.80 (2H, d, J=4.9Hz) | 86 |
| | | MS m/z : 455 (M+H)⁺ | |
| 66 | 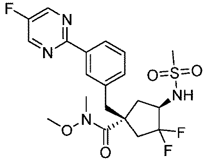 | ¹H-NMR (CDCl₃) δ ppm : 1.65-1.80 (1H, m), 2.27-2.45 (1H, m), 2.74-2.98 (2H, m), 3.02 (3H, s), 3.13-3.32 (5H, m), 3.84 (3H, s), 3.89-4.10 (1H, m), 4.61 (1H, d, J=9.3Hz), 7. 12-7.20 (1H, m), 7.35-7.44 (1H, m), 8.07-8.14 (1H, m), 8.22-8.31 (1H, m), 8.65 (2H, s) | 95 |
| | | MS m/z : 473 (M+H)⁺ | |
| 67 | 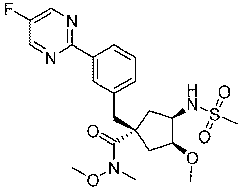 | MS m/z : 467 (M+H)⁺ | 88 |
| 68 | 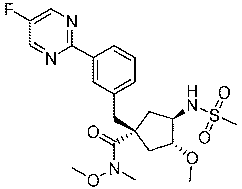 | MS m/z : 467 (M+H)⁺ | 91 |

**[Table 27]**

| Ex. No. | Str. | Phys. data | Act. (%) |
|---|---|---|---|
| 69 | 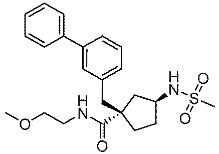 | MS m/z : 431 (M+H)⁺ | 44 |
| 70 | 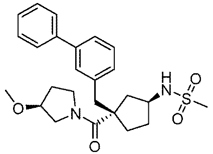 | MS m/z : 457 (M+H)⁺ | 27 |
| 71 | 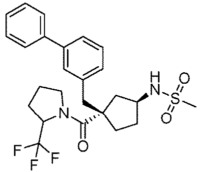 | MS m/z : 495 (M+H)⁺ | 41 |
| 72 | 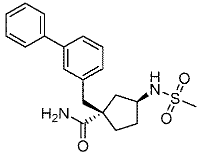 | MS m/z : 371 (M-H)⁻ | 23 |
| 73 | 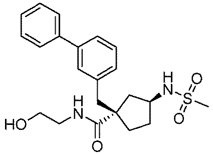 | MS m/z : 417 (M+H)⁺ | 36 |
| 74 | 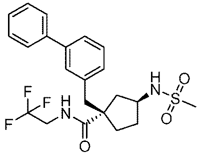 | MS m/z : 453 (M-H)⁻ | 33 |

**[Table 28]**

| Ex. No. | Str. | Phys. data | Act. (%) |
|---|---|---|---|
| 75 | 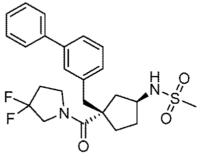 | MS m/z : 461 (M-H)⁻ | 23 |

The stereochemical notations of Ex. Nos. 1-45 and Ex. Nos. 69-75 in the tables represent relative configuration.

### Test Example 1

### 1) Obtaining of human OX2R-expressing cells

A human OX2R sequence (Accession No. NM_001526.4) was inserted into the multiple cloning site of pcDNA3.4 (Life Technologies Japan Ltd.) to clone a plasmid vector for human OX2R expression. The plasmid was introduced into CHO-K1 cells using Lipofectamine 2000 (Life Technologies Japan Ltd.). Two days later, the transgenic CHO-K1 cells were passaged into 175T flasks and cultured selectively for 6 days. As a selection medium, Ham's F-12 Nutrient Mix (Life Technologies Japan Ltd.), containing Geneticin (registered trademark) (Life Technologies Japan Ltd.) at a final concentration of 1 mg/mL and inactivated fetal bovine serum (Biowest) at a final concentration of 10%, was used. After the selective culture, the cells were reseeded into 96-well plates, and a human OX2R-expressing cell line was obtained by limiting dilution.

### 2) Measurement of OX2R agonist activity

The agonist effects of test substances on human OX2R were evaluated using the intracellular calcium concentration-change upon stimulation as an indicator. A day before measurement of the intracellular calcium concentrations, human OX2R-expressing cells suspended in a medium were seeded into 384-well plates at 5 x 10³ cells/50 µL/well and cultured overnight in an incubator set at 37°C and 5% CO₂. After incubation, the medium was removed and replaced with 20 µL of a loading buffer. As the loading buffer, an assay buffer containing Fluo4-AM (DOJINDO Laboratories) at a final concentration of 1.14 µM, cremophor (registered trademark) EL (NACALAI TESQUE, INC.) at a final concentration of 0.02%, probenecid (Life Technologies Japan Ltd.) at a final concentration of 1.5 mM, and amaranth (Sigma-Aldrich Japan LLC.) at a final concentration of 0.5 mg/mL was used. In addition, as the assay buffer, Hanks' balanced salt solution adjusted to pH 7.4 containing 20 mM HEPES, 1.5 mM CaCl₂, 0.5 mM MgCl₂ and 0.4 mM MgSO₄ was used. After incubation of the cells at 37°C for 45 minutes, the fluorescence intensities were measured sequentially using FDSS (registered trademark) 7000 (Hamamatsu Photonics) (excitation wavelength: 480 nm, fluorescence wavelength: 540 nm). Twenty seconds after the start of the measurement, a medium containing a test substance (final concentration; 10 µM) or the medium alone was added, and measurement was made for 2 minutes. As the medium, a solution prepared by diluting dimethyl sulfoxide to a final concentration of 0.1% in the assay buffer containing bovine serum albumin (FUJIFILM Wako Pure Chemical Corporation) at a final concentration of 0.1% was used. The agonist activities of the test substances on human OX2R were calculated by setting the fluorescence intensity obtained when human orexin A peptide (PEPTIDE INSTITUTE, INC.) was added at a final concentration of 1 µM as 100% and the fluorescence intensity obtained when the medium alone was added as 0%. The activation rates of the test substances at a compound concentration of 10 µM are shown in the table above. It was demonstrated from the results that the compounds of the present invention have an agonist activity on human OX2R.

### [Industrial Applicability]

The compounds of the present invention or a pharmaceutically acceptable salt thereof have an OX2R agonist activity and thus are useful as agents for the treatment of sleep disorders involving OX2R.

## Claims

1. A compound represented by the formula (I): 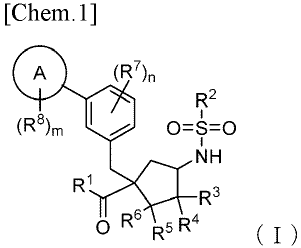 [in the formula,
ring A is C₆₋₁₀ aryl, 5- or 6-membered heteroaryl, or 9- or 10-membered heteroaryl; R¹ is a group selected from the group consisting of the following (a) to (c):
(a) -NR^{a}R^{a}', 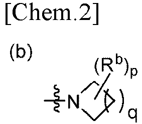
and 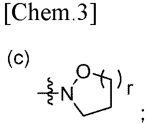
R^{a} and R^{a}' are each independently a hydrogen atom, a hydroxy group, C₁₋₆ alkyl, C₁₋₆ alkoxy, hydroxy C₁₋₆ alkyl, C₁₋₆ alkoxy C₁₋₆ alkyl, or halo C₁₋₆ alkyl;
R^{b} is a hydrogen atom, a halogen atom, C₁₋₆ alkyl, C₁₋₆ alkoxy, or halo C₁₋₆ alkyl;
p is an integer number of 1 to 3;
when p is 2 or 3, these R^{b} are the same or different from each other;
q is an integer number of 1 to 3;
r is 1 or 2;
R² is C₁₋₆ alkyl, C₂₋₆ alkenyl, C₃₋₈ cycloalkyl which may be substituted by 1 to 3 C₁₋₆ alkyl, 3- to 8-membered heterocycloalkyl which may be substituted by 1 to 3 C₁₋₆ alkyl, halo C₁₋₆ alkyl, or C₁₋₆ alkylamino;
R³ and R⁴ are each independently a hydrogen atom, a halogen atom, C₁₋₆ alkyl, or C₁₋₆ alkoxy;
R⁵ and R⁶ are each independently a hydrogen atom or a halogen atom;
R⁷ is a hydrogen atom, a halogen atom, a hydroxy group, an amino group, C₁₋₆ alkyl, halo C₁₋₆ alkyl, C₁₋₆ alkoxy, or C₁₋₆ alkylamino;
R⁸ is a hydrogen atom, a halogen atom, a hydroxy group, a cyano group, C₁₋₆ alkyl, C₁₋₆ alkoxy, halo C₁₋₆ alkyl, or C₁₋₆ alkylamino;
n is 1 or 2;
when n is 2, these R⁷ are the same or different from each other; and
m is 1 or 2;
when m is 2, these R⁸ are the same or different from each other];
or a pharmaceutically acceptable salt thereof.

2. The compound according to claim 1;
wherein ring A is C₆₋₁₀ aryl or 5- or 6-membered heteroaryl;
R⁵ and R⁶ are hydrogen atoms;
R⁷ is a hydrogen atom, a halogen atom, a hydroxy group, C₁₋₆ alkyl, halo C₁₋₆ alkyl, or C₁₋₆ alkoxy; and
n is 1;
or a pharmaceutically acceptable salt thereof.

3. The compound according to claim 2;
wherein R^{a} and R^{a}' are each independently a hydrogen atom, a hydroxy group, C₁₋₆ alkyl, or C₁₋₆ alkoxy;
R^{b} is C₁₋₆ alkyl;
p is 1;
q is 2;
r is 1; and
R³ and R⁴ are each independently a hydrogen atom, a halogen atom, or C₁₋₆ alkyl;
or a pharmaceutically acceptable salt thereof.

4. The compound according to claim 3:
wherein R¹ is -NR^{a}R^{a}'; and
R^{a} and R^{a}' are each independently C₁₋₆ alkyl or C₁₋₆ alkoxy;
or a pharmaceutically acceptable salt thereof.

5. The compound according to claim 4;
wherein ring A is 5- or 6-membered heteroaryl;
or a pharmaceutically acceptable salt thereof.

6. The compound according to claim 5;
wherein R² is C₁₋₆ alkyl, C₃₋₈ cycloalkyl which may be substituted by 1 to 3 C₁₋₆ alkyl, 3-to 8-membered heterocycloalkyl which may be substituted by 1 to 3 C₁₋₆ alkyl, or halo C₁₋₆ alkyl;
or a pharmaceutically acceptable salt thereof.

7. The compound according to claim 6 which is represented by the formula (II): 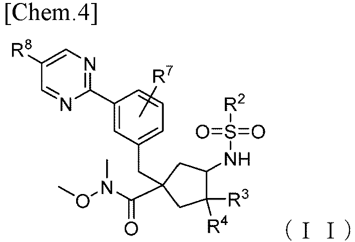 [in the formula,
R² is C₁₋₆ alkyl, C₃₋₈ cycloalkyl which may be substituted by 1 to 3 C₁₋₆ alkyl, 3- to 8-membered heterocycloalkyl which may be substituted by 1 to 3 C₁₋₆ alkyl, or halo C₁₋₆ alkyl;
R³ and R⁴ are each independently a hydrogen atom, a halogen atom, or C₁₋₆ alkyl;
R⁷ is a hydrogen atom, a halogen atom, a hydroxy group, or C₁₋₆ alkyl; and
R⁸ is a hydrogen atom, a halogen atom, or C₁₋₆ alkyl];
or a pharmaceutically acceptable salt thereof.

8. A compound selected from the group consisting of the following compounds: 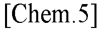 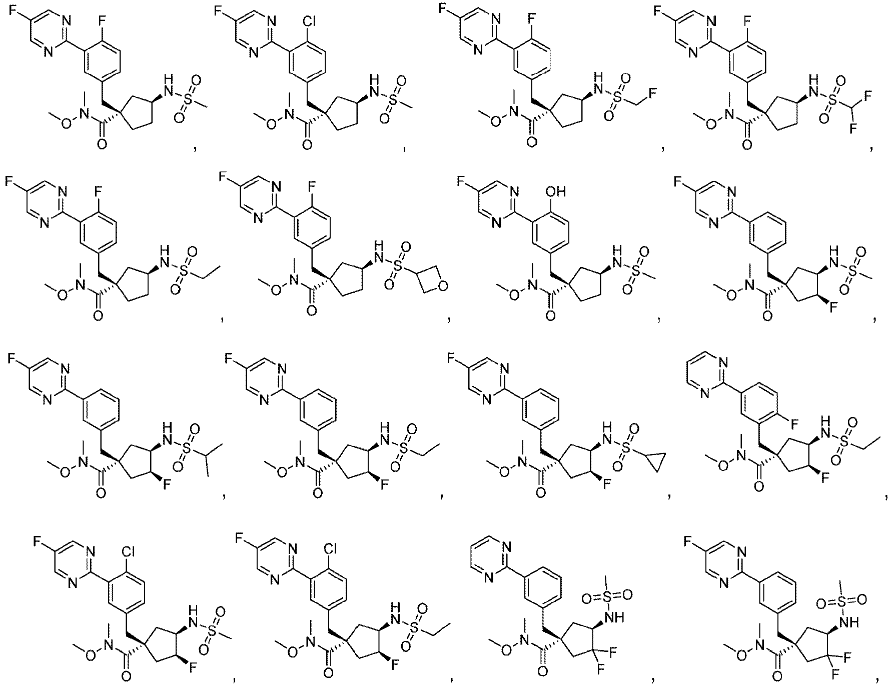 and 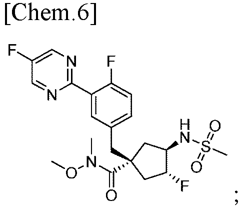 or a pharmaceutically acceptable salt thereof.

9. A compound selected from the group consisting of the following compounds: 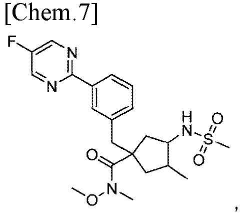 and 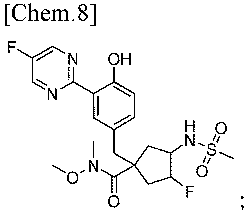 or a pharmaceutically acceptable salt thereof.

10. A pharmaceutical composition comprising the compound according to any one of claims 1 to 9 or a pharmaceutically acceptable salt thereof, and a pharmaceutical additive.

11. The pharmaceutical composition according to claim 10 which is a pharmaceutical composition for use in the treatment of a sleep disorder involving OX2R.

12. The pharmaceutical composition according to claim 11 wherein the sleep disorder involving OX2R is narcolepsy.
